(19) Europäisches Patentamt
European Patent Office
Office européen des brevets

(11) EP 2 368 891 B1

(12) # FASCICULE DE BREVET EUROPEEN

(45) Date de publication et mention de la délivrance du brevet:
**19.09.2012 Bulletin 2012/38**

(51) Int Cl.:
***C07D 471/04*** *(2006.01)*

(21) Numéro de dépôt: **11003015.2**

(22) Date de dépôt: **13.05.2005**

(54) **Dérivés de pyrimidines en tant qu' antagonistes du récepteur de la vitronectine**

Pyrimidin Derivate als Antagonisten von Vitronectin Rezeptoren

Pyrimidines derivatives as antagonists of the vitronectine receptors

(84) Etats contractants désignés:
**AT BE BG CH CY CZ DE DK EE ES FI FR GB GR HU IE IS IT LI LT LU MC NL PL PT RO SE SI SK TR**

(30) Priorité: **18.05.2004 FR 0405407**

(43) Date de publication de la demande:
**28.09.2011 Bulletin 2011/39**

(62) Numéro(s) de document de la (des) demande(s) initiale(s) en application de l'article 76 CBE:
**05771271.3 / 1 747 222**

(73) Titulaire: **Galapagos NV**
**2800 Mechelen (BE)**

(72) Inventeurs:
- **Lefrancois, Jean-Michel**
  **93340 Le Raincy (FR)**
- **Heckmann, Bertrand**
  **91440 Bures sur Yvette (FR)**

(74) Mandataire: **Lord, Hilton David et al**
**Marks & Clerk LLP**
**90 Long Acre**
**London**
**WC2E 9RA (GB)**

(56) Documents cités:
**WO-A-96/00730**
**WO-A-99/32457**
**WO-A-99/37621**
**WO-A-2004/048375**
**DE-A- 10 042 655**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 368 891 B1

## Description

Cette demande est une demande divisionnaire de la demande EP05771271.3.

La présente invention a pour objet un nouveau dérivé antagoniste du récepteur de la vitronectine, son procédé de préparation, son application comme médicament et les compositions pharmaceutiques le renfermant.

L'invention a pour objet le dérivé de pyrimidine de formule (I') :

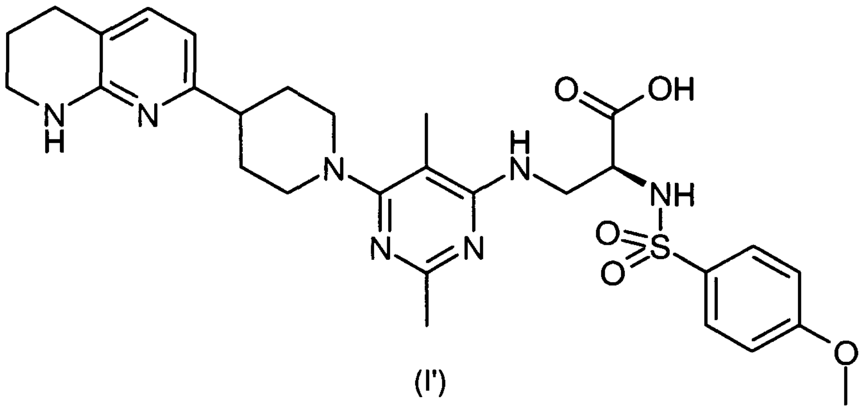

(I')

ainsi que les sels d'addition physiologiquement acceptables et les solvates de ce composé. L'invention de plus a pour objet l'application du dérivé de formule (I') à titre de médicament et les compositions pharmaceutiques le renferment.

Également décrits ci-après sont les dérivés de pyrimidine de formule qénérale (I) :

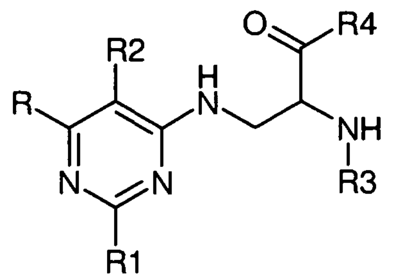

(I)

dans laquelle R, $R^1$, $R^2$, $R^3$ et $R^4$ ont les significations indiquées ci-après, ainsi que leurs formes isomères et leurs mélanges et leurs sels physiologiquement acceptables. Le dérivé de formule (I') selon l'invention est un exemple spécifique d'un dérivé de pyrimidine de formule générale (I). Les composés de formule (I) sont des composés ayant une activité pharmacologique et sont donc utilisables à titre de médicaments. Ce sont des antagonistes du récepteur de la vitronectine et des inhibiteurs de l'adhésion cellulaire et ils inhibent la résorption osseuse médiée par les ostéoclastes. Ils sont donc utiles pour le traitement thérapeutique ou prophylactique de maladies qui sont causées au moins en partie par une augmentation non désirée de la résorption osseuse, par exemple l'ostéoporose. Également décrits ci-après sont les procédés de préparation des composés de formule (I), leur application, en particulier à titre de médicament et les compositions pharmaceutiques les renfermant.

L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. La majorité des désordres au niveau de l'os sont basés sur un équilibre perturbé entre la formation osseuse et la résorption osseuse. L'ostéoporose est caractérisée par une perte sèche de cette matrice osseuse. Un ostéoclaste mature activé résorbe l'os après adhésion à la matrice osseuse via la sécrétion d'enzyme protéolytique, et de protons à l'intérieur de la zone d'adhésion, aboutissant à des dépressions ou des creusements de la surface de l'os qui apparaissent au moment où l'ostéoclaste se détache de l'os.

Des études ont montré que la fixation de l'ostéoclaste sur l'os est médiée par des récepteurs : les intégrines. Les intégrines sont une superfamille de récepteurs médiant les processus d'adhésion cellule/cellule et plus particulièrement cellule/matrice, incluant notamment $\alpha_{IIb}\beta_3$ comme récepteur des plaquettes sanguines (fibrinogène) et $\alpha_v\beta_3$ comme récepteur de la vitronectine. Les peptides contenant le motif RGD ainsi que les anticorps anti $\alpha_v\beta_3$ sont connus pour leur capacité d'inhibition de la résorption de la dentine et d'empêchement de l'adhésion des ostéoclastes sur les

matrices minéralisées (Horton et al. Exp. Cell. Res. (1991), 195, 368). Le peptide Echistatine, isolé du venin de serpent contenant également un motif RGD et décrit comme inhibiteur de l'adhésion des ostéoclastes à l'os est un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1411).

Le récepteur $\alpha_v\beta_3$ est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés de formule (I).

En effet, les récepteurs $\alpha_v\beta_3$, exprimés au niveau de la membrane des ostéoclastes sont à la base du processus d'adhésion/résorption, contribuent à l'organisation du cytosquelette cellulaire, et sont impliqués dans l'ostéoporose. Les récepteurs $\alpha_v\beta_3$ exprimés au niveau des cellules du muscle lisse de l'aorte, stimulent leur migration vers la néointima, ce qui entraîne la formation de l'artériosclérose et la survenue de resténose post-angioplastique (Brown et al., cardiovascular Res. (1994), 28, 1815). Les cellules endothéliales secrètent des facteurs de croissance qui sont mitogènes pour l'endothélium et peuvent contribuer à la formation de nouveaux vaisseaux sanguins (Angiogenèse).

Les antagonistes de l'intégrine $\alpha_v\beta_3$ peuvent ainsi entraîner une régression des tumeurs cancéreuses en induisant l'apoptose des vaisseaux sanguins angiogéniques. (Brook et al. Cell (1994) 79, 1157).

Cheresh et al (Science 1995, 270, 1500) ont décrit des anticorps anti-$\alpha_v\beta_3$ ou des antagonistes du récepteur $\alpha_v\beta_3$ qui inhibent le processus d'angiogénèse induit par bFGF dans l'oeil de rat, une propriété qui peut être utilisée pour le traitement des rétinopathies, notamment du diabètique.

La demande de brevet WO-A-94/12181 décrit des systèmes aromatiques ou non aromatiques substitués et WO-A-94/08577 décrit des hétérocycles substitués comme antagonistes du récepteur de fibrinogène et inhibiteurs de l'agrégation plaquettaire. EP-A-528586 et EP-A-528587 décrivent des dérivés de la phénylalanine substitués par un aminoalcoyle ou un hétérocycle et WO-A-95/32710 décrivent des dérivés aryliques comme inhibiteurs de la résorption osseuse par les ostéoclastes. WO-A-96/00574 décrit des benzodiazépines et WO-A-96/00730 décrit des composés inhibiteurs du récepteur fibrinogène, en particulier benzodiazépines qui sont liés à un cycle à 5 chaînons azoté comme antagonistes du récepteur de la vitronectine. WO 9800395, WO 99/32457 et WO 99/37621 décrivent des antagonistes du récepteur de la vitronectine dérivés de la tyrosine. EP 0 820991 revendique des dérivés de cycloalcoyle comme antagonistes du récepteur de la vitronectine.

WO 2004/048375 décrit des dérivés de la pyrimidine ayant une activité comme antagoniste du récepteur de la vitronectine et de la résorption osseuse médiée via les ostéoclastes, néanmoins, le composé de la présente invention n'y est pas décrit.

D'autres investigations ont permis de montrer que les dérivés de formule (I) présentent une forte activité comme antagonistes du récepteur de la vitronectine et de la résorption osseuse médiée via les ostéoclastes.Également décrits ci-après sont les composés de formule (I) :

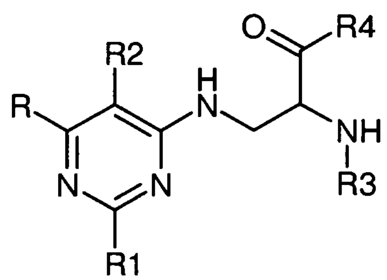

(I)

sous leurs formes isomères, à l'état pur ou sous forme de mélange de ces formes, ainsi que leurs sels d'addition physiologiquement acceptables, dans laquelle :

**I)**

- **R** représente

■ un radical -X-alk-Het pour lequel X est un atome d'oxygène ou de soufre ou un radical -NR'- pour lequel R' est un atome d'hydrogène ou un radical alcoyle droit ou ramifié contenant 1 à 4 atomes de carbone, alk représente un radical alcoyle droit ou ramifié contenant 1 à 4 atomes de carbone et Het représente un système monocyclique ou polycyclique, chaque cycle étant constitué de 4 à 10 chaînons aromatiques ou non aromatiques, et pouvant comprendre 1 à 4 hétéroatomes choisis parmi l'azote, l'oxygène ou le soufre et pouvant être lui même substitué par un ou plusieurs groupements R° définis ci-après, ou bien

■ un radical

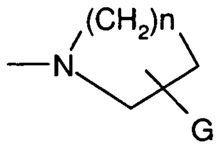

(Ia)

pour lequel G représente un radical hétérocyclyle monocyclique ou polycyclique, aromatique ou non aromatique, comprenant 5 à 10 chaînons et dans lequel au moins l'un des cycles renferme 1 à 4 atome d'azote ledit radical hétérocyclyle pouvant être lui-même éventuellement substitué par un radical ($C_1$-$C_8$)alcoylamino dont la partie alcoyle en chaîne droite ou ramifiée peut être substituée par un radical phényle ou hétérocyclyle à 5 ou 6 chaînons contenant 1 à 4 hétéroatomes choisis parmi l'azote, l'oxygène ou le soufre, et n représente 1 ou 2, ou bien

■ un radical

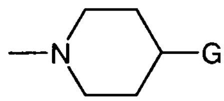

(Ib)

pour lequel G représente un radical hétérocyclyle monocyclique aromatique à 5 ou 6 chaînons contenant 1 à 4 atomes d'azote et substitué par un radical alcoyl($C_1$-$C_6$)amino dont le radical alcoyle peut être lui-même substitué par un radical phényle ou hétérocyclyle monocyclique aromatique à 5 ou 6 chaînons, contenant un hétéroatome choisi parmi l'azote, l'oxygène ou le soufre;

- **$R^1$** représente un atome d'hydrogène; un groupement ($C_5$-$C_{14}$)-aryle; ($C_5$-$C_{14}$)-aryl-($C_1$-$C_4$)-alcoyle-; un radical amino non substitué, monosubstitué ou disubstitué par un radical alcoyle et/ou un radical acyle contenant 1 à 4 atomes de carbone;
- **$R^2$** représente un atome d'hydrogène; un atome d'halogène; un groupement nitro; un radical alcoyle renfermant de 1 à 4 atomes de carbones; un radical amino non substitué ou monosubstitué ou disubstitué par un radical alcoyle et/ou un radical acyle renfermant de 1 à 4 atomes de carbone; un groupement $-(CH_2)_{0-2}-CO_2R^5$; ou un groupement $-(CH_2)_{0-2}-OR^5$;
- **$R^3$** représente

■ un atome d'hydrogène
■ un radical $-CO_2R^5$,
■ un radical $-SO_2R^5$ ou
■ un système monocyclique ou polycyclique, chaque cycle étant constitué de 4 à 10 chaînons aromatiques ou non aromatiques, le cycle ou au moins l'un des cycles renfermant de 1 à 4 hétéroatomes choisis parmi N, O ou S, substitué ou non substitué par un ou plusieurs radicaux R°,

- $R^4$ représente OH; ($C_1$-$C_8$)-alkoxy-; ($C_5$-$C_{14}$)-aryl-($C_1$-$C_4$)-alcoyloxy-; ($C_5$-$C_{14}$)-aryloxy-; ($C_3$-$C_{12}$)-cycloalcoyloxy; ($C_3$-$C_{12}$)-cycloalcoyl-($C_1$-$C_4$)-alcoyloxy-; ($C_1$-$C_8$)-alcoylcarbonyloxy-($C_1$-$C_4$)-alcoyloxy-; ($C_5$-$C_{14}$)-aryl-(C1-C4)-alcoylcarbonyloxy-($C_1$-$C_4$)alcoyloxy-; ($C_1$-$C_8$)dialcoylaminocarbonylméthyloxy-; ($C_5$-$C_{14}$)-aryl-($C_1$-$C_4$)-dialcoylaminocarbonylméthyloxy-; un radical amino non substitué ou monosubstitué ou disubstitué par un radical ($C_1$-$C_4$)-alcoyle et/ou ($C_5$-$C_{14}$)-aryle et/ou ($C_5$-$C_{14}$)-aryl-($C_1$-$C_4$)-alcoyle- et/ou un radical ($C_1$-$C_5$)-acyle; ou bien le reste d'un aminoacide D ou L;
- **$R^5$** représente ($C_1$-$C_8$)-alcoyle; ($C_5$-$C_{14}$)-aryle; ($C_5$-$C_{14}$)-aryl-($C_1$-$C_4$)-alcoyle-; ($C_3$-$C_{12}$)-cycloalcoyle ou ($C_3$-$C_{12}$)-cycloalcoyl-($C_1$-$C_4$)-alcoyle-, bicycloalcoyle-($C_1$-$C_4$)-alcoyle-, tricycloalcoyle-($C_1$-$C_4$)-alcoyle-, les dits radicaux aryles, alcoyles, cycloalcoyles, bicycloalcoyles et tricycloalcoyles étant non substitués ou substitués par un ou plusieurs groupements R°;
- **R°** représente halogène; amino; nitro; hydroxyle, ($C_1$-$C_4$)-alcoyloxy-; ($C_1$-$C_4$)-alcoylthio-; ($C_1$-$C_4$)-alcoylsulfonyle-; carboxy; ($C_1$-$C_4$)-alcoyloxycarbonyle-; ($C_1$-$C_8$)-alcoyle non substitué ou substitué par un ou plusieurs atomes d'halogène, ($C_5$-$C_{14}$)-aryle, ($C_5$-$C_{14}$)-aryl-($C_1$-$C_4$)-alcoyle-; ($C_5$-$C_{14}$)-aryl-($C_1$-$C_4$)-alcoyloxy-; ($C_5$-$C_{14}$)-hétérocyclyle

ou bien

**II)**

- **R** représente un radical

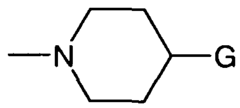

(Ib)

pour lequel G est choisi parmi les radicaux :

Het-NH-CO-;
Het-NH-$CH_2$-,
Het-,
Het représente un système monocyclique ou polycyclique, chaque cycle étant constitué de 4 à 10 chaînons aromatiques ou non aromatiques, le cycle ou au moins l'un des cycles renfermant de 1 à 4 atome d'azote, et pouvant être lui même substitué ou non substitué par un ou plusieurs groupements R°;

- **$R^1$** représente un radical alcoyle contenant 1 à 4 atomes de carbone en chaine droite ou ramifiée; un radical cycloalcoyle contenant 3 à 6 atomes de carbone; ou un radical alcoyloxy ou alcoylthio dont la partie alcoyle contient 1 à 4 atomes de carbone en chaîne droite ou ramifiée;
- **$R^2$, $R^3$, $R^4$** et **$R^5$** sont définis comme précédemment en I) ;
- **R°** est défini comme précédemment en I);

ou bien
III)

- R est défini comme précédemment en II);
- **$R^1$, $R^3$, $R^4$** et **$R^5$** sont définis comme précédemment en I);
- **$R^2$** représente un radical hydroxyméthyle, un radical formyle ou un radical amino disubstitué dont les substituants forment ensemble avec l'atome d'azote auquel ils sont attachés, un hétérocycle saturé ou insaturé contenant 4 à 6 chaînons; et
- **R°** est défini comme précédemment en I);

ou bien
**IV)**

- **R** est défini comme précédemment en II);
- **$R^1$, $R^2$, $R^4$** et sont définis comme précédemment en I);
- **$R^3$** représente

■ un radical alcoyle ($C_1$-$C_4$) ou alcényle ($C_2$-$C_4$) droit ou ramifié, éventuellement substitué par un radical aryle ou hétérocyclyle mono ou polycycliques à 4 à 10 chaînons, pouvant être eux-mêmes substitués par un ou plusieurs radicaux choisis parmi hydroxy, ($C_1$-$C_4$)alcoyloxy, amino, ($C_1$-$C_4$)alcoylamino, ($C_1$-$C_4$)-dialcoylamino, phényle, cyanophényle ou hétérocyclyle monocyclique contenant 1 à 4 hétéroatomes choisis parmi l'azote, l'oxygène ou le soufre;
■ un radical -$CO_2R^5$ pour lequel

- **$R^5$** représente ($C_1$-$C_8$)-alcoyle; ($C_5$-$C_{14}$)-aryle; ($C_5$-$C_{14}$)-aryl-($C_1$-$C_4$)-alcoyle-; ($C_3$-$C_{12}$) -cycloalcoyle ou ($C_3$-$C_{12}$)-cycloalcoyl-($C_1$-$C_4$)-alcoyle-, bicycloalcoyle-($C_1$-$C_4$)-alcoyle-, tricycloalcoyle-($C_1$-$C_4$)-alcoyle-, les dits radicaux aryles, alcoyles, cycloalcoyles, bicycloalcoyles et tricycloalcoyles étant non substitués ou substitués par un ou plusieurs groupements R° choisis parmi ($C_1$-$C_4$)-alcoylsulfonyle-; ($C_5$-$C_{14}$)-aryl-($C_1$-$C_4$)-alcoyloxy- ou ($C_5$-$C_{14}$)-hétérocyclyle,
ou bien

■ un radical -COR'$^5$,
■ un radical -$SO_2$R"$^5$,

- **R'$^5$** représente ($C_1$-$C_8$)-alcoyle substitué par un radical tel que défini pour $R^5$ ou par un radical ($C_5$-$C_{14}$)aryloxy, les radicaux aryle ou cycloalcoyle pouvant être eux-mêmes substitués par un ou plusieurs radicaux R°; ou **R'$^5$** représente un radical cycloalcoyle, aryle ou hétérocyclyle mono ou polycycliques éventuellement substitués par des radicaux trifluorométhylalcoyloxy ou ($C_1$-$C_{10}$)-aryle; ou

**R'⁵** représente ($C_1$-$C_4$) alcoylamino; ($C_3$-$C_8$) cycloalcoylamino; arylamino ou hétérocyclylamino dont la partie aryle ou hétérocyclyle sont mono ou polycycliques, ces radicaux $R'^5$ pouvant être eux-mêmes substitués parun atome d'halogène, un radical nitro, amino, ($C_1$-$C_4$)alcoyloxy, ($C_1$-$C_4$)alcoyloxycarbonyle, aryle ou arylalcoyle dont la partie alcoyle contient 1 à 4 atomes de carbone en chaîne droite ou ramifiée; et

- **$R''^5$** représente un radical ($C_1$-$C_4$)alcoylamino ou di($C_1$-$C_4$) alcoylamino dont les parties alcoyle peuvent former ensemble un hétérocycle à 5 à 7 chaînons, avec l'atome d'azote auquel elles sont liées, un radical arylamino, aralcoyl($C_1$-$C_4$)amino ou hétéroaralcoyl($C_1$-$C_4$) amino dont le radical aryle ou hétéroaryle est mono ou polycyclique et comprend 5 à 10 chaînons, le radical hétéroaryle pouvant comprendre 1 à 4 hétéroatomes choisis parmi l'azote l'oxygène ou le soufre; et
- **$R^°$** est défini comme précédemment en I);

ou bien

**V)**

- **R, $R^2$, $R^3$** et **$R^4$** sont définis comme précédemment en I);
- **$R^1$** est défini comme précédemment en II);

ou bien

**VI)**

- **R, $R^1$, $R^3$** et **$R^4$** sont définis comme précédemment en II);
- **$R^2$** est défini comme précédemment en III);

ou bien

**VII)**

- **R, $R^1$** et **$R^2$** sont définis comme précédemment en I);
- **$R^4$** est défini comme précédemment en I); et
- **$R^3$** est défini comme précédemment en IV);

ou bien

**VIII)**

- **R, $R^1$** et **$R^2$** sont définis comme précédemment en II);
- **$R^4$** est défini comme précédemment en I); et
- **$R^3$** est défini comme précédemment en IV);

ou bien

**IX)**

- **R** et **$R^3$** sont définis comme précédemment en I);
- **$R^4$** est défini comme précédemment en I);
- **$R^1$** est défini comme précédemment en II); et
- **$R^2$** est défini comme précédemment en III);

ou bien

X)

- **R** et **$R^2$** sont définis comme précédemment en I);
- **$R^4$** est défini comme précédemment en I);
- **$R^1$** est défini comme précédemment en II); et
- **$R^3$** est défini comme précédemment en IV);

ou bien

**XI)**

- **R** et **$R^1$** sont définis comme précédemment en II);
- **$R^4$** est défini comme précédemment en I);

- **$R^2$** est défini comme précédemment en III); et
- **$R^3$** est défini comme précédemment en IV);

ou bien

**XII)**

- **R** et **$R^4$** sont définis comme précédemment en I);
- **$R^1$** est défini comme précédemment en II);
- **$R^2$** est défini comme précédemment en III);
- **$R^3$** est défini comme précédemment en IV);
sous réserve que les radicaux ci-dessus ne puissent pas avoir simultanément la signification
- R représente un radical (Ib) dans lequel G est 1,2,3,4-tétrahydro-1,8-naphtyridin-7-yle,
- $R^1$ représente méthyle,
- $R^2$ représente méthyle,
- $R^3$ représente benzyloxycarbonyle et
- $R^4$ représente OH ou t.butoxy.

Tous les radicaux qui peuvent se retrouver plusieurs fois dans les composés de formule (I), comme par exemple le radical R°, sont indépendants les uns des autres et peuvent être identiques ou différents.Il est entendu que les radicaux ou portions alcoyles peuvent être droits ou ramifiés.

Les radicaux cycloalcoyle peuvent être monocycliques, bicycliques ou tricycliques. A titre d'exemple les radicaux monocycliques peuvent être choisis parmi cyclopropyle, cyclobutyle, cyclopentyle, cyclohexyle, cycloheptyle, cyclooctyle, cyclononyle, cyclodécyle, cycloundécyle, cyclododécyle, cyclotétradécyle ou cyclooctadécyle qui le cas échéant peuvent être substitués par exemple par un alcoyle renfermant de 1 à 4 atomes de carbone en chaîne droite ou ramifiée. Comme radicaux cycloalcoyles monocycliques substitués, on peut citer notamment le 4-méthylcyclohexyle et le 2,3-diméthylcyclohexyle.

Les radicaux bicycloalcoyles et tricycloalcoyles peuvent être non substitués ou substitués en toute position, par exemple par un ou plusieurs radicaux oxo et/ou 1 ou plusieurs radicaux alcoyles identiques ou différents comme par exemple méthyle, éthyle ou isopropyle, de préférence méthyle. La liaison de jonction du radical bi ou tricyclique peut se situer en toutes positions de la molécule. La liaison peut se situer au niveau d'un atome de carbone ponté ou d'un des autres atomes de carbone. Cette liaison peut présenter également toute position du point de vue de la stéréochimie, par exemple exo ou endo. Comme exemple de radicaux bicycloalcoyles ou tricycloalcoyles, on peut citer le camphanyle, le bornyle, l'adamantyle tel que le 1-adamantyle ou le 2-adamantyle, le caranyle, l'épiisobornyle, l'épibornyle, le norbornyle ou le norpinanyle.

Par halogène on entend fluor, chlore, brome ou iode.

Par le terme ($C_5$-$C_{14}$)-aryle on entend :

- soit les radicaux ($C_5$-$C_{14}$)-aryle hétérocycliques (ou ($C_5$-$C_{14}$)-hétéroaryle), dans lesquels un ou plusieurs atomes de carbone du cycle sont remplacés par un hétéroatome tel que azote, oxygène ou soufre,
- soit les radicaux ($C_6$-$C_{14}$)-aryle carbocyclique.

Parmi les radicaux($C_6$-$C_{14}$)-aryle carbocycliques, on peut citer le phényle, le naphtyle, l'anthryle ou le fluorényle et tout particulièrement le 1-naphtyle, le 2-naphtyle et le phényle.

Sauf indication contraire, les radicaux aryles, en particulier phényle, peuvent être non substitués ou substitués par un ou plusieurs radicaux identiques ou différents choisis parmi ($C_1$-$C_8$)-alcoyle, en particulier ($C_1$-$C_4$)-alcoyle, hydroxyle, ($C_1$-$C_8$)-alcoyloxy, ($C_1$-$C_8$)-alcoylthio, halogène choisi parmi fluor, chlore et brome, nitro, amino, ($C_1$-$C_4$)-alcoylamino, di-($C_1$-$C_4$)-alcoylamino, trifluorométhyle, méthylènedioxy, cyano, carbamoyle, ($C_1$-$C_4$)-alcoylcarbamoyle, di-($C_1$-$C_4$)-alcoylcarbamoyle, carboxy, ($C_1$-$C_4$)-alkoxycarbonyle, phényle, phénoxy, benzyle et benzyloxy.

Dans le cas d'un radical phényle monosubstitué, la position des substituants est indifférente, de préférence il est substitué en position 3 ou 4. Dans le cas où le phényle est disubstitué, la position des substituants est indifférente. De préférence les 2 substituants sont en position 3,4. Lorsque le phényle est tri-substitué la position des substituants est indifférente. De la même manière, les radicaux naphtyles ou d'autres radicaux aryles peuvent être substitués en toute position.

Lorsque le groupement ($C_5$-$C_{14}$)-aryle représente un système aromatique monocyclique ou polycyclique dans lequel 1 à 4 atomes de carbone du cycle sont remplacés par des hétéroatomes, identiques ou différents choisis parmi l'azote, l'oxygène et le soufre, ou lorsque l'on utilise la signification hétéroaryle, on peut citer à titre d'exemple les groupements pyridyle, pyrrolyle, furyle, thiényle, imidazolyle, pyrazolyle, oxazolyle, isoxazolyle, thiazolyle, isothiazolyle, tétrazolyle, pyridyle, pyrazinyle, pyrimidinyle, indolyle, isoindolyle, indazolyle, phtalazinyle, quinolyle, isoquinolyle,

naphtyridinyle, quinoxalinyle, quinazolinyle, cinnolinyle, □-carbolinyle, ou encore des dérivés benzo-condensés, cyclopenta-, cyclohexa-, ou cyclohepta- condensés de ces radicaux comme par exemple tétrahydroquinokyle ou tétrahydronaphtyridinyle. Le système hétérocyclique peut être substitué par les substituants cités précédemment pour le système aryle-carbocyclique.

Les radicaux hétérocyclyle sont, sauf mention spéciale, mono, ou polycycliques aromatiques ou non, et peuvent être notamment choisis, à titre non limitatif, parmi pyrrolyle, pyrazolyle, imidazolyle, triazinyle, tétrazolyle, thiényle, furyle, thiazolyle, isothiazolyle, oxazolyle, isoxazolyle, pyridyle, pyrazinyle, pyrimidinyle, pyridazinyle, pyrannyle, thiopyrannyle, indolyle, isoindolyle, benzofurannyle, isobenzofurannyle, benzothiényle, indazolyle, indolizinyle, benzimidazolyle, imidazopyridyle, benzopyrimidinyle, benzoxazolyle, benzothiazolyle, oxazolopyridyle, quinolyle, isoquinolyle, quinalozinyle, quinoxalinyle, naphtyridinyle, naphtooxazolyle, naphtothiazolyle, pyrrolidinyle, pyrrolinyle, imidazolidinyle pyrazolidinyle, pyrazolinyle, pipéridinyle, pipérazinyle, dihydropyrannyle, tétrahydropyrannyle, dihydrothiopyrannyle, tétrahydrothiopyrannyle, morpholinyle, thiomorpholinyle, indolinyle, chromannyle, tétrahydroquinolyle, tétrahydrobenzimidazolyle, tétrahydrobenzopyrimidinyle, tétrahydronaphtyridinyle, dihydropyrrolopyridine, quinuclidinyle.

Les atomes de carbone optiquement actifs contenus dans les composés de formule (I) peuvent indépendamment les uns des autres présenter la configuration R ou la configuration S.

Les composés de formule (I) peuvent être sous la forme d'énantiomères purs ou de diastéréoisomères purs ou sous la forme d'un mélange d'énantiomères, par exemple sous forme de racémates ou de mélanges de diastéréoisomères.

Décrits sont donc les énantiomères purs, les mélanges de ces énantiomères, les diastéréoisomères purs et les mélanges de ces diastéréoisomères.

Également décrits sont les mélanges de deux ou plus de deux stéréoisomères de formule (I) et tous les rapports de ces stéréoisomères au sein des dits mélanges.

Les composés de formule (I) peuvent le cas échéant être présent sous forme d'isomères E ou d'isomères Z. Décrits sont donc les isomères E purs, les isomères Z purs et les mélanges E/Z selon un rapport quelconque.

Les stéréoisomères ainsi que les isomères E et Z peuvent être séparés en forme pures selon les méthodes connues, par exemple par chromatographie, chromatographie en phase chirale ou par cristallisation.

Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que l'acide carboxylique, les sels peuvent être par exemple des sels de métaux alcalins ou alcalino terreux tels que les sels de sodium, de potassium, de magnésium, de calcium, des sels d'addition avec des amines physiologiquement acceptables comme par exemple la triéthylamine, l'éthanolamine ou la tris-(2-hydroxyéthyle)amine ou avec l'ammoniac et également les sels d'ammonium quaternaires physiologiquement acceptables.

Lorsque les composés de formule (I) contiennent un groupe basique, ils peuvent former des sels d'addition avec les acides par exemple avec les acides minéraux tels que l'acide chlorhydrique, sulfurique, phosphorique ou avec les acides carboxyliques tels que l'acide acétique, trifluoracétique, citrique, benzoïque, maléique, fumarique, tartrique, méthanesulfonique ou p.toluènesulfonique.

Les composés de formule (I) qui comportent un groupe basique et un groupe acide, peuvent être présents sous forme de zwiterions (bétaïnes).

Le cas échéant un anion $Q^-$ physiologiquement acceptable peut être contenu dans les composés de formule (I) renfermant un groupement ammonium chargé. Il s'agit de préférence d'un anion monovalent ou d'un équivalent d'anion polyvalent d'un acide organique ou minéral non toxique, physiologiquement acceptable et en particulier pharmaceutiquement acceptable, par exemple l'anion ou un équivalent d'anion d'un des acides cités plus haut utile pour la formation des sels d'addition.

$Q^-$ peut être par exemple un des anions (ou équivalent d'anion) d'un groupe choisi parmi chlore, sulfate, phosphate, acétate, trifluoracétate, citrate, benzoate, maléate, fumarate, tartrate, méthanesulfonate et p.toluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant approprié ou un dispersant ou à partir d'un autre sel par échange de cation ou d'anion.

Également décrits ci-après sont tous les sels des composés de formule (I) qui, à cause de leur faible acceptabilité physiologique, ne sont pas directement utilisables comme médicament, mais sont utilisables comme produits intermédiaires pour la purification, ou pour mettre en oeuvre des modifications chimiques ultérieures au niveau des composés de formule (I) ou comme produits de départ pour la préparation de sels physiologiquement acceptables.

Également décrits ci-après sont tous les solvates des composés de formule (I) par exemple les hydrates, les solvates formés avec les alcools, et tous les dérivés des composés de formule (I), par exemple les esters, prodrugs et autres dérivés physiologiquement acceptables, ainsi que les métabolites des composés de formule (I).

Également décrits ci-après sont les prodrugs des composés de formule (I), à savoir les dérivés chimiquement

modifiés des composés de formule (I) afin d'obtenir des propriétés améliorées de manière désirée, qui sont connus de l'homme du métier.

Pour avoir plus d'information sur le type de prodrug envisagé, on peut citer les ouvrages suivants : Fleicher et al., Advanced Drug Delivery Review 19 (1996) 115-130; Design of prodrugs, H. Bundgaard, Ed., Elsevier, 1985; H. Bungaard, Drugs of the Future 16 (1991) 443; Saulnier et al. Bioorg. Med. Chem. Lett. 4 (1994) 1985; Safadi et al. Pharmaceutical Res. 10 (1993) 1350. Parmi les prodrugs appropriées des composés de formule (I) on peut citer de préférence :

- les prodrugs sous forme d'esters des groupes carboxyliques,
- les prodrugs sous forme d'acyle et de carbamate pour les groupes contenant un azote acylable tel que les groupes amino.

Dans les prodrugs acylés ou sous forme de carbamate, une ou plusieurs fois, par exemple deux fois, un atome d'hydrogène situé sur l'atome d'azote est remplacé par un groupe acyle ou carbamate. Parmi les groupes acyles ou carbamates préférés, on peut citer les groupes $R^6CO$-, $R^7OCO$-, dans lesquels $R^6$ est un hydrogène ou un radical ($C_1$-$C_{18}$)-alkyle, ($C_3$-$C_{14}$)-cycloalkyle, ($C_3$-$C_{14}$)-cycloalkyle-($C_1$-$C_8$)-alkyle, ($C_5$-$C_{14}$)-aryle, dans lequel 1 à 5 atomes de carbone peuvent être remplacés par des hétéroatomes tels que N, O, S ou ($C_5$-$C_{14}$)-aryle-($C_1$-$C_8$)alkyle, dans lequel 1 à 5 atomes de carbone dans la partie aryle peuvent être remplacés par des hétéroatomes tels que N, O, S et $R^7$ à les mêmes valeurs que $R^6$ à l'exception de représenter l'hydrogène.

Également décrits ci-après sont les composés de formule (I) dans laquelle G représente un groupement Het, Het-NHCO-, ou Het-NH-$CH_2$- dans lequel Het représente :

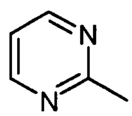
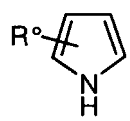
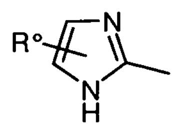

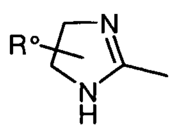
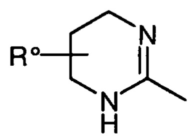
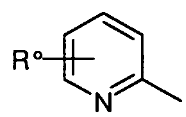

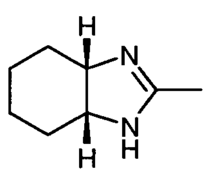
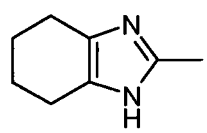
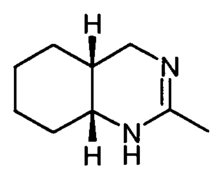

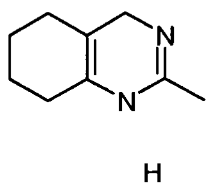
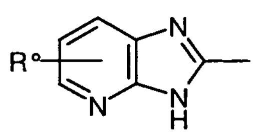
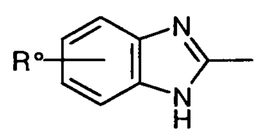

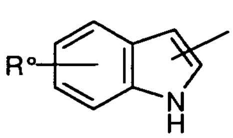
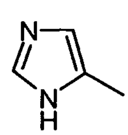
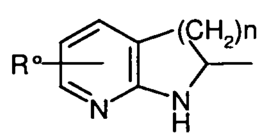

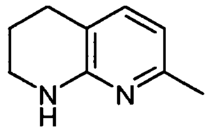 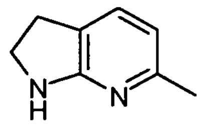 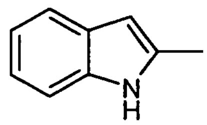 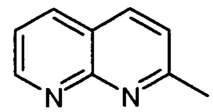

Plus particulièrement décrits sont les composés de formule (I) tels que définis plus haut dans laquelle $R^3$ est un groupement benzyloxycarbonyle, ou dans laquelle $R^3NH$- forme une fonction amide ou urée ainsi que leurs sels d'addition pharmaceutiquement acceptables.

Plus particulièrement décrits sont les composés de formule (I) tels que définis ci-dessus dans laquelle $R^2$ est un hydrogène, un radical alcoyle renfermant de 1 à 4 atomes de carbone, particulièrement méthyle et éthyle, un radical hydroxyméthyle ou un atome de fluor ainsi que leurs sels d'addition pharmaceutiquement acceptables.

Plus particulièrement décrits sont les composés de formule (I) tels que définis ci-dessus dans laquelle $R^1$ est un radical alcoyle contenant 1 à 4 atomes de carbone en chaîne droite ou ramifiée, ou cycloalcoyle contenant 3 à 6 atomes de carbone.

Plus particulièrement décrits sont les composés de formule (I) tels que définis plus haut dans laquelle :

. G représente :

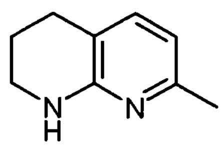 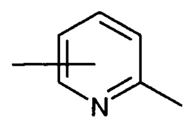

ainsi que leurs sels d'addition pharmaceutiquement acceptables.

Également décrit est un procédé de préparation des composés de formule (I). Les composés peuvent généralement être préparés, par exemple par synthèse convergente par couplage de deux ou plusieurs fragments qui peuvent être dérivés par rétrosynthèse des composés de formule (I). Afin d'éviter que les groupes fonctionnels puissent mener à des réactions indésirables ou secondaires au cours de chaque étape de synthèse, il peut être avantageux ou nécessaire préalablement à certaines phases de synthèse des composés de formule (I), d'introduire les groupes fonctionnels sous forme de précurseurs qui sont par la suite convertis en groupes fonctionnels désirés ou bien de protéger temporairement ces groupes fonctionnels selon les méthodes connues pour la mise en place et l'élimination de radicaux protecteurs, qui n'altèrent pas le reste de la molécule; notamment selon Greene, Wuts protective Group in Organic Synthesis, Wiley (1991).

Les produits de formule générale (I) peuvent être préparés selon le schéma suivant :

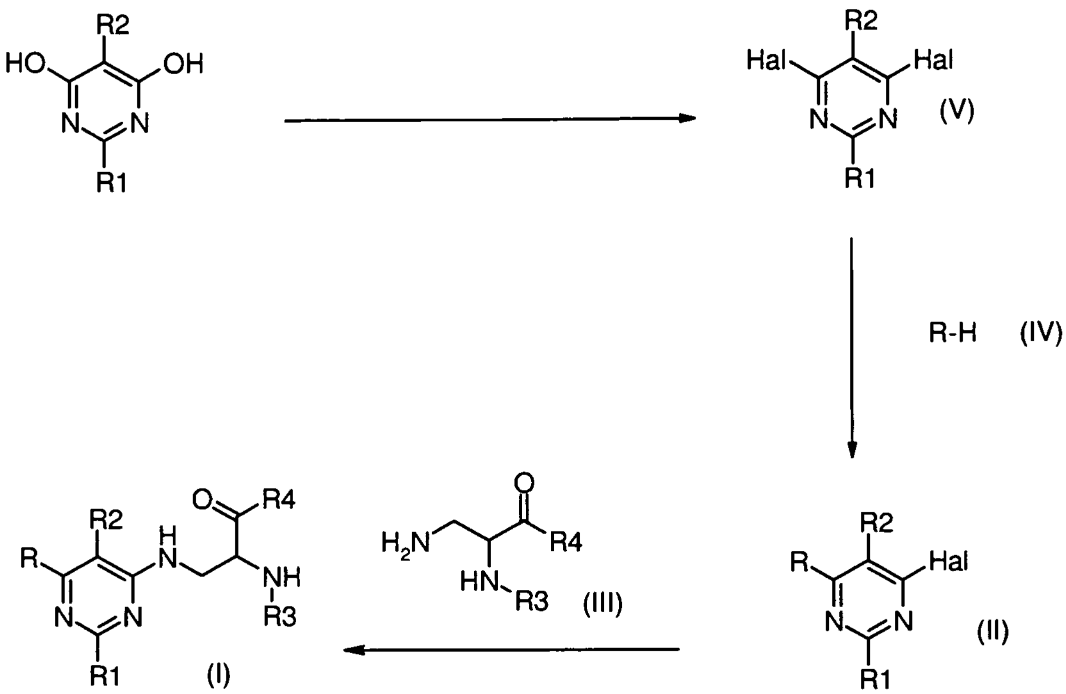


dans lequel $R^1$, $R^2$, $R^3$, $R^4$ et R sont définis tels que précédemment en I) à XII) et Hal représente un atome d'halogène, de préférence le chlore ou le brome.

Le procédé de préparation des produits de formule (I) consiste dans

a) l'action d'un dérivé de la pyrimidine de formule (II)

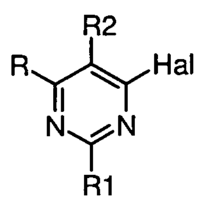

(II)

dans laquelle $R^1$, $R^2$, R et Hal sont tels que définis précédemment, sur une amine de formule (III)

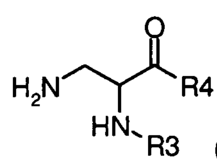

(III)

dans laquelle $R^3$ et $R^4$ sont définis comme précédemment, soit en présence d'une base forte, soit par catalyse au palladium,

b) puis lorsque l'on souhaite obtenir un produit pour lequel le radical R est saturé ou partiellement saturé, le produit de formule générale (I) est le cas échéant soumis à une étape d'hydrogénation,

c) puis, le cas échéant, lorsque l'on souhaite obtenir un dérivé de la pyrimidine de formule générale (I) pour lequel $R^2$ est hydroxyméthyle, réduction du dérivé correspondant pour lequel $R^2$ représente un radical formyle,

d) et/ou le cas échéant, lorsque G représente un radical hétérocyclyle portant un radical amino substitué, substitution du produit correspondant portant une fonction amine primaire sur le radical hétérocyclyle,

e) puis le cas échéant clivage de la fonction $R^3$-NH- du produit de formule générale (I) pour régénérer l'amine libre, et condenser un radical $R^3$ de structure $-CO_2$-$R^5$, -CO-R'$^5$, $-SO_2$-$R^5$ ou - $SO_2$-R"$^5$ ou alcoyle éventuellement substitué,

f) et/ou éventuellement hydrolyse et/ou estérification ou amidification et/ou salification du dérivé de pyrimidine obtenu.

La réaction de la pyrimidine de formule (II) avec l'amine de formule (III) s'effectue généralementen présence d'une base forte encombrée telle que la diisopropyléthylamine dans des conditions réactionnelles connues de l'homme du métier dans la mise en oeuvre de substitution nucléophile. De préférence on opère en présence d'un amide (diméthylacétamide, diméthylformamide par exemple), à une température comprise entre 90°C et la température de reflux du mélange réactionnel. Par ailleurs le groupement $COR^4$ est de préférence choisi parmi les groupes ester encombrés comme par exemple tertbutoxycarbonyle. Il est également possible d'opérer par catalyse au palladium (par exemple tris(dibenzylidèneacétone) dipalladium) en présence de fluorure de césium, à la température de reflux du mélange réactionnel. Il est entendu que les fonctions pouvant interférer avec la réaction sont protégées. La protection et la libération de ces fonctions s'effectue selon les méthodes habituelles qui n'altèrent pas le reste de la molécule.

Lorsque l'on souhaite obtenir un produit pour lequel le radical R est saturé ou partiellement saturé, la réaction d'hydrogénation s'effectue en présence d'oxyde de platine, dans un solvant tel qu'un alcool (éthanol, méthanol par exemple), sous pression atmosphérique.

Lorsque l'on souhaite obtenir un dérivé de la pyrimidine de formule générale (I) pour lequel $R^2$ est hydroxyméthyle, la réduction du dérivé correspondant pour lequel $R^2$ représente un radical formyle, s'effectue avantageusement au moyen d'un borohydrure alcalin (borohydrure de sodium), dans un solvant tel qu'un alcool (éthanol, méthanol par exemple), à une température comprise entre 10 et 40°C, de préférence à température ambiante.

Lorsque l'on souhaite obtenir un dérivé de la pyrimidine de formule générale (I) pour lequel G (dans R) représente un radical hétérocyclyle portant un radical amino substitué, la substitution du produit correspondant portant une fonction amine primaire sur le radical hétérocyclyle s'effectue par action de l'aldéhyde correspondant, en milieu réducteur, notamment en présence d'un borohydrure comme par exemple un triacétoxyborohydrure de métal alcalin. La réaction s'effectue dans un solvant organique comme un solvant chloré (dichlorométhane, dichloréthane par exemple), à une température comprise entre 10 et 40°C, de préférence à température ambiante.

La condensation de radicaux $R^3$ de structure $-CO_2-R^5$, $-CO-R'^5$, $-SO_2-R^5$ ou $-SO_2-R''^5$ sur l'amine libre, s'effectue par action d'un dérivé réactif ou du chlorure de l'acide carboxylique ou de l'acide sulfonique, en présence d'une base azotée (comme par exemple la pyridine), dans un solvant organique chloré (dichlorométhane, dichloréthane, chloroforme par exemple), à une température comprise entre 10 et 40°C, de préférence à température ambiante. Lorsque $R'^5$ est un radical amino substitué, la condensation s'effectue au moyen de l'isocyanate correspondant dans un solvant organique comme un éther (tétrahydrofurane par exemple), à une température comprise entre 10 et 40°C, de préférence à température ambiante.

La condensation de radicaux $R^3$ de structure alcoyle éventuellement substitué s'effectue par action de l'aldéhyde correspondant, en milieu réducteur, notamment en présence d'un borohydrure comme par exemple un triacétoxyborohydrure de métal alcalin. La réaction s'effectue dans un solvant organique comme un éther (tétrahydrofurane par exemple), à une température comprise entre 10 et 40°C, de préférence à température ambiante.

La réaction d'hydrolyse afin d'obtenir un dérivé d'acide ($COR^4=CO_2H$), d'estérification afin d'obtenir un ester ou une prodrogue (particulièrement $COR^4$=alcoyloxycarbonyle ou aryloxycarbonyle à partir de l'acide correspondant) ou d'amidification ($COR^4$=aminocarbonyle mono ou disubstitué à partir de l'acide correspondant) s'effectuent selon les méthodes usuelles connues de l'homme du métier.

Notamment l'hydrolyse s'effectue en milieu acide, en présence d'acide trifluoracétique par exemple, dans un solvant organique halogéné comme le dichlorométhane par exemple.

Si nécessaire, la conversion en des sels physiologiquement acceptables s'effectue par des procédés connus de l'homme du métier.

Les dérivés de pyrimidine de formule (II) peuvent être préparés par action d'un produit de formule (IV) dans laquelle R est défini comme précédemment, sur un dérivé dihalogéné de la pyrimidine de formule générale :

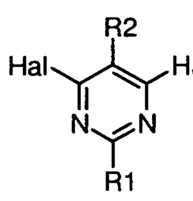

(V)

dans laquelle $R^1$, $R^2$ et Hal sont définis comme précédemment.

La réaction s'effectue avantageusement en présence d'une base forte encombrée, à la température de reflux du mélange réactionnel. On opère dans les conditions décrites ci-après dans les exemples et notamment en présence d'une amine encombrée comme la diisopropyléthylamine, dans un amide comme le diméthylacétamide par exemple. Il est entendu que les fonctions pouvant interférer avec la réaction sont préalablement protégées. La protection et la libération de ces fonctions s'effectue selon les méthodes habituelles qui n'altèrent pas le reste de la molécule.

La préparation des pyrimidines dihalogénées de formule générale (V) peut être effectuée selon ou par analogie

avec les méthodes décrites ci-après dans les exemples.

Le composé de formule (I') est un composé ayant une activité pharmacologique et peut ainsi être utilisé à titre de médicaments notamment dans le traitement ou la prévention des maladies de l'os, des maladies tumorales ainsi que des désordres cardiovasculaires.

La présente invention a donc pour objet le composé de formule (I') et/ou ses sels physiologiquement acceptables à titre de médicament.

Le composé de formule (I') ainsi que ses sels physiologiquement acceptables peuvent être administrés aux animaux, de préférence aux mammifères et en particulier aux êtres humains comme médicaments à titre thérapeutique ou prophylactique.

Il peut être administré tels quels ou en mélange avec un ou plusieurs autres composés de formule (I) ou encore sous la forme d'une préparation pharmaceutique (composition pharmaceutique) qui permet une administration entérale ou parentérale et qui renferme à titre de composé actif une dose efficace d'un composé de formule (I') et/ou ses sels physiologiquement acceptables ainsi que des supports et/ou additifs courants et pharmaceutiquement inertes.

Les compositions pharmaceutiques selon l'invention permettent une administration entérale ou parentérale; elles renferment à titre de composé actif une dose efficace d'un composé de formule (I') et/ou ses sels physiologiquement acceptables ainsi qu'un ou plusieurs supports pharmaceutiquement inertes, et/ou un ou plusieurs additifs usuels.

L'invention a donc pour objet les compositions pharmaceutiques renfermant un composé de formule (I') à l'état pur ou en présence d'un ou plusieurs excipients.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire, par voie parentérale, par exemple sous forme de solutions injectables ou d'infusions, de microcapsules ou d'implants, par voie percutanée, par exemple sous la forme de pommade, de solutions, de pigments ou de colorants, par voie transdermique sous forme de patchs ou par d'autres voies telles que sous la forme d'aérosol ou de spray nasal.

Les compositions pharmaceutiques selon l'invention sont préparées selon des méthodes connues en soi, des supports organiques ou inorganiques, pharmaceutiquement inertes pouvant être additionnés aux composés de formule (I') et/ou à leurs sels physiologiquement acceptables.

Pour la production de pilule, de comprimés, de comprimés enrobés et de capsule en gélatine dure, il est possible d'utiliser par exemple, du lactose, de l'amidon de maïs ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les micro capsules ou les implants sont par exemple les copolymères d'acide glyoxylique et d'acide lactique. Les préparations pharmaceutiques contiennent normalement de 0,5% à 90% en poids de composés de formule (I') et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants.

En outre, en plus d'un composé de formule (I') et/ou ses sels physiologiquement acceptables, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs.

Les composés de formule (I) sont tout particulièrement des antagonistes des récepteurs de la Vitronectine et sont capables ainsi par exemple d'inhiber l'adhésion des ostéoclastes sur la surface de l'os et ainsi la résorption osseuse par les ostéoclastes.

L'action des composés de formule (I) peut être démontrée par exemple dans un test dans lequel l'inhibition de la liaison de la vitronectine aux cellules qui contiennent le récepteur de la vitronectine est déterminée. Des précisions sur ce test sont données plus bas. Comme antagonistes du récepteur de la vitronectine, les composés de formule (I) et leurs sels physiologiquement acceptables conviennent en général pour le traitement ou la prévention de maladies liées aux interactions entre les récepteurs de la vitronectine et leurs ligands, dans les processus d'interaction cellule-cellule ou cellule-matrice ou qui peuvent être influencés par l'inhibition des interactions de ce type, pour soulager ou guérir lorsqu'une inhibition des interactions de ce type est désirée. Comme on l'a expliqué au début, une telle interaction joue un rôle important dans la résorption osseuse, dans l'angiogénèse ou dans la prolifération des cellules du muscle

vasculaire lisse.

Les maladies de l'os dont le traitement ou la prévention nécessitent l'emploi des composés de formule (I), sont notamment l'ostéoporose, l'hypercalcémie, l'ostéopénie, par exemple causée par les métastases osseuses, les désordres dentaires par exemple les parodontites, l'hyperparathyroïdisme, les érosions péri articulaires dans l'arthrite rhumatoïde, et la maladie de Paget. En outre les composés de formule (I) peuvent être utilisés pour soulager, empêcher ou traiter les désordres de l'os qui sont causés par les traitements, par les glucocorticoïdes, les thérapies liées à la prise de stéroïdes ou de corticostéroïdes ou par les déficiences d'hormones sexuelles mâles ou femelles. Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes. A côté de cette utilisation comme inhibiteur de la résorption osseuse médiée via les ostéoclastes, les composés de formule (I) et leurs sels physiologiquement acceptables sont utilisés comme inhibiteurs de la croissance tumorale ou des métastases cancéreuses, dans le traitement des désordres inflammatoires, pour le traitement ou la prévention des désordres cardiovasculaires, telles que l'artériosclérose ou la resténose, ou le traitement ou la prévention de néphropathie ou rétinopathie tel que par exemple la rétinopathie diabétique.

Les composés selon l'invention peuvent posséder également une activité vis-à-vis d'autres intégrines qui interagissent avec leur ligand via la séquence tripeptidique RGD ($\alpha_v\square_1$, $\alpha_v\square_1$, $\alpha_{IIb}\square_3$), leur conférant des propriétés pharmacologiques utilisables pour traiter les pathologies associées à ces récepteurs.

Cette activité vis-à-vis des intégrines rend ainsi les composés de formule (I) utilisables dans la prévention ou le traitement de nombreuses maladies telles que celles mentionnées plus haut ou dans la revue de Dermot Cox DN§P 8(4) mai 1995, 197-205 dont le contenu est intégré dans la présente demande à titre de référence.

La présente invention a donc plus particulièrement pour objet le composé de formule (I') et/ou ses sels physiologiquement acceptables tel que défini plus haut à titre de médicament ayant une activité antagoniste du récepteur de la vitronectine.

La présente invention a donc plus particulièrement pour objet le composé de formule (I') et/ou ses sels physiologiquement acceptables tel que défini plus haut à titre de médicament ayant une activité inhibitrice de la résorption osseuse ou pour le traitement ou la prévention de l'ostéoporose.

La présente invention a donc plus particulièrement pour objet le composé de formule (I') et/ou ses sels physiologiquement acceptables tel que défini plus haut à titre de médicament ayant une activité inhibitrice de la croissance tumorale ou des métastases cancéreuses.

La présente invention a donc plus particulièrement pour objet le composé de formule (I') et/ou ses sels physiologiquement acceptables tel que défini plus haut à titre de médicament ayant une activité anti-inflammatoire ou pour le traitement ou la prévention des désordres cardiovasculaires, la resténose, l'artériosclérose, les néphropathies ou rétinopathies.

La présente invention a également pour objet l'utilisation du composé de formule (I') et/ou ses sels physiologiquement acceptables tels que définis plus haut pour la préparation de médicaments destinés à la prévention ou au traitement de l'ostéoporose.

La présente invention a également pour objet l'utilisation du composé de formule (I') et/ou ses sels physiologiquement acceptables tels que définis plus haut pour la préparation de médicaments destinés à inhiber la croissance tumorale ou les métastases cancéreuses.

La présente invention a également pour objet l'utilisation des composés de formule (I') et/ou ses sels physiologiquement acceptables tels que définis plus haut pour la préparation de médicaments destinés à la prévention ou au traitement des désordres cardiovasculaires, de la resténose, de l'artériosclérose, des néphropathies ou des rétinopathies.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,1 à 100 mg/kg et de préférence de 0,1 à 50 mg/kg.

Dans le cas d'une administration par voie intraveineuse, la dose quotidienne varie approximativement de 0,01 à 100 mg/kg et de préférence de 0,1 à 10 mg/kg.

La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante. Mis à part l'utilisation des composés de formule (I) comme médicaments, on peut également envisager leur utilisation comme véhicule ou support de composés actifs afin de transporter ces composés actifs de manière spécifique vers un site d'action (Drug targeting, voir Targeted Drug Delivery, R. C. Juliano, Handbook of Expérimental Pharmacology, Vol 100, Ed. Born, G. V. R. et al, Springer Verlag). Les composés actifs qui peuvent être transportés sont en particulier ceux utilisés pour le traitement ou la prévention des maladies citées plus haut.

Les composés de formule (I) et leurs sels peuvent également être employés comme agent de diagnostique, par exemple pour des méthodes in vitro ou comme auxiliaire dans des études biochimiques dans lesquelles on désire bloquer le récepteur de la vitronectine ou influencer les interactions cellules-cellules ou cellules-matrices. Ils peuvent en outre être utilisés comme intermédiaire pour la préparation d'autres composés, en particulier d'autres agents actifs, qui sont accessibles à partir des composés de formule (I), par exemple par modification ou introduction de radicaux ou de groupes fonctionnels.

## Exemples

Les produits ont été identifiés par spectre de masse (MS), infrarouge (IR) et/ou spectre RMN. Les composés, qui ont été purifiés par chromatographie en utilisant un éluant qui contient par exemple de l'acide acétique ou trifluoroacétique, et qui ensuite sont séchés ou dans lesquels lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

**Abréviations/noms chimiques éventuellement employés :**

AcOEt : acétate d'éthyle; EDCI : chlorhydrate de 1-(3-diméthylaminopropyl)-3-éthyl-carbodiimide; DMF : diméthylformamide; DIPEA : Diisopropyléthylamine; MeOH : méthanol; TEA : triéthylamine; TFA : acide trifluoroacétique; THF : tétrahydrofurane; MCPBA : acide méta-chloroperoxybenzoique; DBU : 1,8-diazabicyclo[5.4.0] undec-7-ene; APTS : acide paratoluènesulfonique; DPPA : diphénylphosphorylazide; DMSO : diméthylsulfoxyde; Pd/C palladium sur charbon; Boc : terbutoxycarbonyle; CBz : benzyloxycarbonyle; DCC 1,3-dicyclohexylcarbodiimide; TMSBr : bromotriméthylsilane; TMSI : iodure de triméthylsilane.

IR : Infrarouge; RMN : Résonnance Magnétique Nucléaire; SM : Spectre de Masse; ESP : Electrospray mode positif; ep. : Épaulement; F : fort; s : singulet; d : doublet; t : triplet; q : quadruplet; quint : quintuplet; 1 : large; m : multiplet ou massif; J : constante de couplage; Rf : facteur de rétention (chromatographie).

Exemple comparatif 1

Synthèse de la (6-bromo-5-éthyl-pyrimidin-4-yl)-[3-(5,6,7,8-tétrahydro-[1,8]-naphthyridin-2-yl)-propyl]-amine

Un mélange de 0,20 g (1 mmole) de 3-(5,6,7,8-tétrahydro-[1,8]-naphthyridin-2-ylpropylamine [préparé selon J. Org. Chem. 2004, 69, (1959-1966)] et de 0,30 g (1,1 mmoles) de 4,6-dibromo-5-éthyl-pyrimidine dans 20 ml de diméthylacétamide et 1 ml de diisopropyléthylamine est porte à 120°C durant 6 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane 100% jusqu'à acétate d'éthyle 100%. On obtient 0,21 g de produit attendu sous forme d'une huile jaune.
**CCM** : Rf=0,25 (silicagel, éluant : acétate d'éthyle)
**1H-RMN (CDCl3)** : δ 1, 13 (t, 3H, $CH_2$-$\underline{CH_3}$) ; 1,93 et 2,03 (2m, 2X2H, $CH_2$-$\underline{CH_2}$-$CH_2$); 2,57 (q, 2H, $\underline{CH_2}$-$CH_3$); 2,71 (m, 4H, $\underline{CH_2}$-$CH_2$-$CH_2$-NH) ; 3,44 et 3,55 (2m, 2X2H, $CH_2$-$CH_2$-$\underline{CH_2}$-NH); 6,39 et 7,11 (2d, 2H, H naphthyridine); 8,17 ppm (s,N=$\underline{CH}$-N); 5,01 et 5,67 (2m, 2X1H, Hmobiles).
**SM** : 378,379 (MH+).

Synthèse du 2-benzyloxycarbonylamino-3-[5-éthyl-6-[3-(5,6,7,8-tétrahydro-[1,8]-naphthyridin-2-yl)propylamino]-pyrimidin-4-ylamino]-propionate de tert-butyle.

On chauffe au reflux un mélange de 105 mg (0,28 mmole) de (6-bromo-5-éthyl-pyrimidin-4-yl)-[3-(5,6,7,8-tétrahydro-[1,8]-naphthyridin-2-yl)-propyl]-amine, de 150 mg (0,5 mmole) de 3-amino-2-benzyloxycarbonylamino propionate de tert-butyle) (préparé selon J. Med. Chem.(2001), 44(8), 1158-1176), de 140 mg (0,92 mmole) de fluorure de césium, de 40 mg (0,044 mmole) de tris(dibenzylidèneacétone)dipalladium(O), et de 60 mg (0,097 mmole) de 2,2'-bis (diphényl-phosphino)-1,1'-binaphtyl dans 20 ml de dioxane pendant 4 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient d'heptane-acétate d'éthyle 100-0 à 0-100. On obtient 70 mg de produit attendu sous forme d'huile jaune.

**CCM :** Rf=0,66 (silicagel, éluant : dichlorométhane-méthanol-eau-acide acétique 85-15-2-2)
**1H-RMN ($CDCl_3$)** : δ 1,11 (t, 3H, $CH_2$-$\underline{CH_3}$); 1,47 (s, 9H, tBu); 1,96 et 2,16 (2m, 2X2H, $CH_2$-$\underline{CH_2}$-$CH_2$); 2,41 (q, 2H, $\underline{CH_2}$-$CH_3$) ; 2,77 et 2,84 (2t, 2X2H, $\underline{CH_2}$-$CH_2$-$CH_2$-NH); 3,44 et 3,52 (2m, 2X2H, $CH_2$-$CH_2$-$\underline{CH_2}$-NH); 3,86 (m, 2H, NH-$\underline{CH_2}$-CH-NH); 4,39 (m, 1H, NH-$CH_2$-$\underline{CH}$-NH); 5,16 (s, 2H, $\underline{CH_2}$-Ph); 6,43 et 7,35 (masqué) (2d, 2H, H naphthyridine); 7,35 (m, 5H, Ph) 8,15 ppm (s,N=$\underline{CH}$-N)
**SM :** 590 (MH+); 534 (MH-tBu+); 400 (MH-COOCH2Ph+).

Syntèse de l'acide 2-benzyloxycarbonylamino-3-[5-éthyl-6-[3-(5,6,7,8-tétrahydro-[1,8]-naphthyridin-2-yl)propylamino]-pyrimidin-4-ylamino]-propionique bis(trifluoroacetate).

On agite 65 mg (0,11 mmole) de 2-benzyloxycarbonylamino-3-[5-éthyl-6-[3-(5,6,7,8-tétrahydro-[1,8]-naphthyridin-2-yl)propylamino]-pyrimidin-4-ylamino]-propionate de tert-butyle dans 5 ml de dichlorométhane avec 0,5 ml d'acide trifluoroacétique à température ambiante pendant 16heures. On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 45 mg de produit attendu sous forme d'un solide beige.
**CCM :** Rf=0,55 (silicagel, éluant : dichlorométhane-méthanol-eau-acide acétique 85-15-2-2)
**HPLC/SM :** 534 (MH+); 400 (MH-COOCH2Ph+); 532- (M-H-); 424- (532-OCH2Ph-); 1065- (2M-H-)

Exemple comparatif 2

Synthèse de la (6-chloro-5-méthyl-pyrimidin-4-yl)-[3-(5,6,7,8-tétrahydro-[1,8]-naphthyridin-2-yl)-propyl]-amine

Un mélange de 774,3 mg (4,05 mmoles) de 3-(5,6,7,8-tétrahydro-[1,8]-naphthyridin-2-ylpropylamine (préparé selon J. Org. Chem. 2004, 69, 1959-1966) et de 600 mg (3,68 mmoles) de 4,6-dichloro-5-méthyl-pyrimidine dans 6 ml de diméthylacétamide et 1,2 ml de diisopropyléthylamine est porte à 120°C durant 9 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur Alumine puis sur silice en éluant avec un gradient d'heptane 100% jusqu'à acétate d'éthyle 100%. On obtient 680 mg (Rdt=58%) de produit attendu.
**CCM :** Rf=0,096 (silicagel, éluant : acétate d'éthyle 100%)
**1H-RMN ($CDCl_3$-d6)** : δ 1,96 et 2,1 (2m, 2*2H, $CH_2$-$\underline{CH_2}$-$CH_2$); 2,12 (s, 3H, $CH_3$); 2,75 (m, 2*2H, $\underline{CH_2}$-$CH_2$-$CH_2$-NH); 3,46 et 3,6 (2m, 2*2H, $CH_2$-$\underline{CH_2}$-$CH_2$-NH) ; 4,92 et 5,45 (2m, 2*1H, NH); 6,42 et 7,13 (2d, 2*1H, naphthyridine); 8,3 (s, 1H, N=$\underline{CH}$-N).
**SM :** 318 (MH+).

Synthèse du 2-benzyloxycarbonylamino-3-[5-méthyl-6-[3-(5,6,7,8-tétrahydro-[1,8]-naphthyridin-2-yl)propylamino]- pyrimidin-4-ylamino]-propionate de tert-butyle.

On chauffe au reflux un mélange de 680 mg (2,14 mmole) de (6-Chloro-5-méthyl-pyrimidin-4-yl)-[3-(5,6,7,8-tétrahydro-[1,8]-naphthyridin-2-yl)-propyl]-amine, de 755 mg (2,57 mmole) de 3-amino-2-benzyloxycarbonylamino propionate de tert-butyle) (préparé selon J. Med. Chem.(2001), 44(8), 1158-1176), de 455 mg (3 mmole) de fluorure de césium, de 133 mg (0,214 mmole) de tris(dibenzylidèneacétone)dipalladium(0), et de 98 mg (0,107 mmole) de 2,2'-bis (diphényl-phosphino)-1,1'-binaphtyl dans 20 ml de diméthoxyétane pendant 24 heures.
Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié par 2 fois sur alumine en éluant avec un gradient d'acétate d'éthyle-éther isopropylique (50/50) avec 10% d'un mélange dichlorométhane-méthanol (9/1). On obtient 220 mg de produit attendu.
**CCM :** Rf=0,30 (silicagel, éluant : dichlorométhane-méthanol-eau-acide acétique 90-10-1-1)
**1H-RMN ($CDCl_3$)** : δ 1,45 (s, 9H, tBu); 1,76 à 2, 1 (2m, 7H, $CH_2$-$\underline{CH_2}$-$CH_2$, $CH_2$-$\underline{CH_2}$-$CH_2$, $CH_3$); 2,63 à 2,8 (m, 2*2H, $\underline{CH_2}$-$CH_2$-$CH_2$-NH); 3,4 à 3,6 (m, 2*2H, $CH_2$-$CH_2$-$\underline{CH_2}$-NH); 3,7 à 4,05 (2m,2H, NH-$\underline{CH2}$-CH-NH); 4,47 (m, 1H, NH-$CH_2$-$\underline{CH}$-NH); 4,73 et 4,8 (2m,2H, NH); 5,03 (s, 2H, $\underline{CH2}$-O-Ph); 6,42 et 7,35 (2d, 2H, naphthyridine); 6,55, 6,8 et 6,95 (3m, 3*1H, NH); 7,02 à 7,35 (m, 5H, Ph); 8,18 (s, 1H, N=$\underline{CH}$-N).
**SM :** 576 (MH+); 520(MH - tbu+); 386(MH -tbu-COOCH2Ph+).

Synthèse de l'acide 2-benzyloxycarbonylamino-3-[5-méthyl-6-[3-(5,6,7,8-tétrahydro-[1,8]-naphthyridin-2-yl)propylamino]-pyrimidin-4-ylamino]-propionique, bis(trifluoroacetate).

On agite 40 mg (0,069 mmole) de 2-benzyloxycarbonylamino-3-[5-méthyl-6-[3-(5,6,7,8-tétrahydro-[1,8]-naphthyridin-2-yl)propylamino]-pyrimidin-4-ylamino]-propionate de tert-butyle dans 1 ml de dichlorométhane avec 0,2 ml d'acide trifluoroacétique à température ambiante pendant 16 heures. On rajoute du toluène et évapore le mélange réactionnel à sec sous pression réduite (2 kPa). Le résidu est solubilisé dans le minimum de dichlorométhane puis versé sur de l'éther diisopropylique. Le précipité est filtré. On obtient 43 mg de produit attendu sous forme d'un solide blanc cassé.

**CCM :** Rf=0,13 (silicagel, éluant : dichlorométhane-méthanol-eau-acide acétique 90-10-1-1)

**RMN ($CDCl_3$)** : δ 1,92 et 2,0 (2m, 2*2H, $CH_2$-$\underline{CH_2}$-$CH_2$); 2,05 (s, 3H, $CH_3$); 2,7 et 2,84 (m, 2*2H, $\underline{CH_2}$-$CH_2$-$CH_2$-NH); 3,42 et 3,52 (2m, 2*2H, $CH_2$-$CH_2$-$\underline{CH_2}$-NH); 3,85 (m, 2H, NH-$\underline{CH_2}$-CH-NH); 4,4 (m, 1H, NH-$CH_2$-$\underline{CH}$-NH); 4,73 et 4,8 (2m, 2H, NH); 5,12 (s, 2H, $\underline{CH_2}$-O-Ph) ; 5,47 (m, 1H, NH); 6,37 et 7,12 (2d, 2H, naphthyridine); 7,35 (m, 5H, Ph); 8,17 (s, N=$\underline{CH}$-N).

**HPLC/SM :** 520 (MH+); 386 (MH-COOCH2Ph+).

Exemple comparatif 3

Préparation du 3-[1,8]naphthyridin-2-yl-propan-1-ol :

Dans un ballon contenant 1 g (8,19 mmoles) de 2-amino-pyridine-3-carbaldéhyde mis en solution dans 5 ml d'éthanol, on ajoute à température ambiante 914 μl (9 mmoles) de 5-Hydroxy-pentan-2-one, 750 μl (9 mmoles) de pyrrolidine et 11,5 μl (0,2 mmole) d'acide sulfurique concentré. Le mélange réactionnel est alors porté au reflux (78°C) pendant 3 heures et 30 minutes.

Après refroidissement, la solution est concentrée à sec sous pression réduite (2 kPa) et le résidu obtenu est repris par un mélange d'eau, d'acétate d'éthyle et d'une solution de bicarbonate de sodium saturée. La phase organique est séparée et la phase acqueuse extraite à l'acétate d'éthyle puis au n-butanol. Les phases organiques regroupées sont séchées sur du sulfate de magnésium. Le filtrat est concentré à sec puis chromatographié sur silicagel en éluant avec un gradient d'acétate d'éthyle (100%) d'acétate d'éthyle-méthanol (98-2) jusqu'à Acétate d'éthyle- méthanol (90-10).

On obtient 990 mg de produit attendu.

**CCM :** Rf=0,1 [silicagel, éluant : acétate d'éthyle-méthanol(98-2)

**1H-RMN ($CDCl_3$-d1)** : δ 2,2 (quintuplet, 2H, O-CH2-$\underline{CH2}$-CH2); 2,97 (m, 1H, $\underline{HO}$-CH2-CH2-CH2); 3,22 (t, 2H, O-CH2-CH2-$\underline{CH2}$); 3,8 (t, 2H, O-$\underline{CH2}$-CH2-CH2); [(7,33; 7,47; 8,12; 8,18; 9,08), 5 multiplets, 5H, Naphthyridine].

**SM :** 189(MH+)

Synthèse du 2-[3-(6-chloro-5-méthyl-pyrimidin-4-yloxy)-propyl]-[1,8]naphthyridine :

Dans un monocol contenant 102 mg (2,12 mmoles) d'hydrure de sodium et 7 ml de dioxane, on ajoute goutte à goutte, à température ambiante et sous azote 800 mg (4,24 mmoles) de 3-[1,8]naphthyridin-2-yl-propan-1-ol mis en solution dans 5 ml de dioxane.

Ce mélange est maintenu sous agitation et sous atmosphère inerte à température ambiante pendant 2 heures.

On ajoute ensuite 346,4 mg (2,12 mmoles) de 4,6-dichloro-5-méthyl-pyrimidine mis en solution dans 5 ml de dioxane.

Le mélange est ensuite chauffé au reflux pendant 5 heures et 30 minutes puis concentré à sec sous vide. Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur du sulfate de magnésium. Le filtrat est concentré à sec puis chromatographié sur silicagel en éluant avec un gradient d'acétate d'éthyle (100%) puis d'acétate d'éthyle- méthanol (98-2).

On obtient 300 mg de produit attendu.

**CCM :** Rf=0,32 (silicagel, éluant : Acétate d'éthyle-Méthanol(95-5).

**1H-RMN ($CDCl_3$)** : δ 2,1 (singulet, 3H, -$\underline{CH3}$); 2,5 (quintuplet, 2H, O-CH2-$\underline{CH2}$-CH2); 3,25 (triplet, 2H, O-CH2-CH2-$\underline{CH2}$); 4,55 (t, 2H, O-$\underline{CH2}$-CH2-CH2); [(7,43; 7,5; 8,15; 8,2; 9,13), 5 multiplets, 5H, Naphthyridine]; 8,32 (singulet, 1H, N=$\underline{CH}$-N).

Synthèse de la 7-[3-(6-chloro-5-méthyl-pyrimidin-4-yloxy)-propyl]-1,2,3,4-tétrahydro-[1,8]naphthyridine :

A 300 mg (0,953 mmoles) de 2-[3-(6-chloro-5-méthyl-pyrimidin-4-yloxy)-propyl]-[1,8]naphthyridine, on ajoute une quantité suffisante d'éthanol pour solubiliser le produit, et 15 mg d'oxyde de platine. Le mélange réactionnel est alors purgé sous vide et surmonté d'un ballon de baudruche contenant de l'hydrogène. On laisse sous agitation et à

température ambiante pendant 6 heures puis la nuit. Le mélange réactionnel est ensuite filtré sur clarcel et concentré à sec sous pression réduite (2 kPa). On obtient alors 300 mg de produit attendu.
**CCM :** Rf=0,6 [Silicagel, éluant : Acétate d'éthyle-méthanol (95-5)].
**1H-RMN ($CDCl_3$)** : □ 1,93 (quintuplet, 2H, CH2-CH2-CH2-NH), 2,22 (multiplet, 5H, -CH3, O-CH2-CH2-CH2); 2,73 (m, 4H, O-CH2-CH2-CH2, CH2-CH2-CH2-NH); 3,43 (m, 2H, CH2-CH2-CH2-NH); 4,45 (triplet, 2H, O-CH2-CH2-CH2); 5,23 (m, 1H, CH2-CH2-CH2-NH); 6,37 et 7,1 (2d, 2H, Naphthyridine]; 8,4 (singulet, 1H, N=CH-N).
**SM :** 319 (MH+)

Synthèse du 2-benzyloxycarbonylamino-3-{5-méthyl-6-[3-(5,6,7,8-tétrahydro-[1,8]naphthyridin-2-yl)-propoxy]-pyrimidin-4-ylamino}-propionate de tert-butyle :

Un mélange de 2 g (6,27 mmoles) de 7-[3-(6-chloro-5-méthyl-pyrimidin-4-yloxy)-propyl]-1,2,3,4-tétrahydro-[1,8] naphthyridine et de 2,2 g (7,52 mmoles) de 3-amino-2-benzyloxycarbonylamino propionate de tert-butyle, en présence de 1,3 g (8,78 mmoles) de fluorure de cesium, de 390 mg (62,7 μmoles) de (2,2'-bis(diphénylphosphino)-1,1'-binaphthyl et de 290 mg (31,3 μmoles) de tris-dibenzylidèneacétone dipalladium(o) dans 35 ml de 1,2-diméthoxyéthane est chauffé au reflux pendant 24 heures.
Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur silice avec un gradient d'acétate d'éthyle-dichlorométhane (50-50) jusqu'à acétate d'éthyle-dichlorométhane (80-20). On obtient 772 mg de produit attendu.
**CCM :** Rf=0,17 (silicagel, éluant : acétate d'éthyle-dichlorométhane 80-20).
**1H-RMN ($CDCl_3$) :** δ 1,45 (s, 9H, tBu); 2,88 (s, 3H, -CH3); 1,93 (m, 2H, NH-CH2-CH2-CH2); 2,17 (m, 2H, O-CH2-CH2-CH2); 2,68 et 2,8 (m, 4H, NH-CH2-CH2-CH2, O-CH2-CH2-CH2); 3,45 (m, 2H, NH-CH2-CH2-CH2); 3,9 (m, 2H, NH-CH2-CH-NH); 4,35 (t, 2H, O-CH2-CH2-CH2); 4,45 (m, 1H, NH-CH2-CH-NH); 5,12 (m, 2H, O-CH2-Ph); 4,95 et 6,10 (2m, 2H, NH); 6,37 et 7,13 (2d, 2H, CH=CH naphthyridine); 7,35 (m, 5H, Ph); 8,2 (s, 1H, N=CH-N).
**SM :** 577 (MH+)

Exemple comparatif 4

Formation de l'acide correspondant à l'ester de l'exemple 3 :

Synthèse de l'acide 2-Benzyloxycarbonylamino-3-{5-méthyl-6-[3-(5,6,7,8-tétrahydro-[1,8]naphthyridin-2-yl)-propoxy]-pyrimidin-4-ylamino}-propionique, bis(trifluoroacetate).

On agite à température ambiante un mélange de 65 mg (0,11 mmoles) de 2-benzyloxycarbonylamino-3-{5-méthyl-6-[3-(5,6,7,8-tétrahydro-[1,8]naphthyridin-2-yl)-propoxy]-pyrimidin-4-ylamino}- propionionate de tert-butyle mis en solution dans 1 ml de dichlorométhane et de 50 μl d'acide trifluoroacétique pendant 25 heures. On additionne alors du toluène et évapore à sec le mélange. Le résidu est solubilisé dans un minimum de dichlorométhane puis versé sur un mélange de pentane et d'éther diisopropylique. Le précipité est filtré. On obtient une poudre jaune impure que l'on doit purifier sur silice avec un gradient d'acétate d'éthyle 100% jusqu'à acétate d'éthyle -méthanol (80-20).
On obtient 21 mg de produit attendu.
**CCM :** Rf=0,33 [ Silicagel, éluant: Dichlorométhane-méthanol-Acide Acétique-eau (90-10-1-1).
**1H-RMN ($CDCl_3$) :** δ 1,72 et 1,95 (m, 5H, -CH3, NH-CH2-CH2-CH2); 2,15 (m, 2H, O-CH2-CH2-CH2); 2,63 et 2,85 (m, 4H, NH-CH2-CH2-CH2, O-CH2-CH2-CH2); 3,45 (m, 2H, NH-CH2-CH2-CH2); 3,7 et 4,0 (m, 2H, NH-CH2-CH-NH); 4,3 (m, 3H, O-CH2-CH2-CH2, NH-CH2-CH-NH); 5,1 (m, 2H, O-CH2-Ph); 5,78 et 6,10 (2m, 2H, NH); 6,33 et 7,25 (2d, 2H, CH=CH naphthyridine); 7,28 et 7,45 (m, 5H, Ph); 8,13 (s, 1H, N=CH-N).
**SM :** 521 (MH+)

Exemple comparatif 5

1°) Synthèse de la N-t.butoxycarbonyl-3-(méthoxy-méthylcarbamoyl)-azétidine.

On dissout 2 g (10 mmol) de N-t.butoxycarbonyl-azétidine-3-carboxylic acid) dans 20 ml de diméthylformamide sous argon.
On refroidit cette solution à 0°C à l'aide d'un bain de glace et on ajoute 5,46 g (12,3 mmol) de benzotriazol-1-yloxytris(diméthylamino)phosphonium hexafluorophosphate, 1,4 g (14,3 mmol) de chlorure de N,O-diméthylhydroxylamine et 6 ml de diisopropyléthylamine. On laisse revenir le mélange réactionnel à température ambiante et on agite 18

heures à température ambiante.

On évapore le diméthylformamide et la diisopropyléthylamine sous pression réduite (2 kPa).

On purifie par chromatographie sur Silicagel en éluant avec un mélange heptane/ acétate d'éthyle 50:50

On récupère 2,1 g d'huile incolore.

**CCM :** Rf=0,25 (silicagel, éluant : Heptane/Acétate d'éthyle 50:50

**1H-RMN ($CDCl_3$)** : δ 1,44 (s, 9H, tBu); 3,22 (s, 3H, -N-C**H3**); 3,64 (m, 1H, H3); 3,67(s, 3H, -O-C**H3**); 4.05 et 4,15(m, 4H, H2 et H2')

2°) Synthèse du N-t.butoxycarbonyl-3-acétyl-azétidine

110 mg (0,45 mmol) de N-t.butoxycarbonyl-3-(méthoxy-méthylcarbamoyl)-azétidine sont solubilisés dans 2 ml d'éther sous argon.

On refroidit cette solution à 0°C à l'aide d'un bain de glace et on additionne goutte à goutte 1ml d'une solution de méthyllythium 1,6M dans l'éther.

On agite 2 heures à cette température puis traite par une solution aqueuse d'acide chlorhydrique 1M.

On décante et sépare les phases aqueuse et éther. On renouvelle 3 fois cette opération puis rassemble les phases éthérées que l'on sèche sur $MgSO_4$, puis après filtration on évapore l'éther sous pression réduite.

On récupère 40 mg d'une huile incolore.

**CCM :** Rf=0,35 (silicagel, éluant : Heptane/Acétate d'éthyle 50:50

**1H-RMN ($CDCl_3$) :** δ 1,44(s, 9H, **tBu**) ; 2,2(s, 3H, -CO-C**H3**) ; 3,45(m, 1H, **H3**); 3,67(s, 3H, -O-C**H3**); 4.07 (m, 4H, **H2** et **H2'**)

N-t.butoxycarbonyl-3-[1,8]naphthyridin-2-yl-azétidine :

30 mg de N-t.butoxycarbonyl-3-acétyl-azétidine (0,150 mmol) sont solubilisés dans 2 ml d'éthanol auxquels on additionne 18 mg (0,150 mmol) de 2-aminopyridine-3-carboxyaldéhyde et 25 mg de carbonate de potassium (0,18 mmol).

Ce mélange est porté au reflux pendant 24 heures puis on laisse revenir à température ambiante et évapore l'éthanol sous pression réduite (2 kPa).

Le résidu obtenu est purifié par chromatographie sur silica gel en éluant avec un mélange dichlorométhane/ MeOH 95:5.

On récupère 30 mg de produit attendu sous la forme d'un solide beige.

**CCM :** Rf=0,25 (silicagel, éluant : CH2C12/MeOH 95:5)

**SM :** 256 (MH+); 230 (MH-tBu+)

**1H-RMN ($CDCl_3$) :** δ 1,47 (s, 9H, **tBu**) ; 4,18 (m, 1H, **H3**); 4,40 (m, 4H, **H2** et **H2'**); 7,52(m, 2H, **H7** et **H4**); 8,22(m, 2H, **H5** et **H6**); 9,14 (m, 1H, **H8**)

3-[1,8]Naphthyridin-2-yl-azétidine

50 mg (0,175 mmol) de N-t.butoxycarbonyl-3-[1,8]Naphthyridin-2-yl-azétidine sont solubilisés dans 2 ml de $CH_2Cl_2$ auxquels on additionne 0,2 ml d'acide trifluoroacétique et laisse agiter 18heures à température ambiante.

On évapore l'acide trifluoroacétique et le dichlorométhane sous pression réduite (2 kPa).

On isole 40 mg produit brut sous forme d'huile jaune.

**CCM :** Rf=0,25 (silicagel, éluant : $CH_2Cl_2$/MeOH 90:10)

**SM :** 186 (MH+)

Synthèse du 7-azétidin-3-yl-1,2,3,4-tétrahydro-[1,8]naphthyridine :

40 mg de 3-[1,8]naphthyridin-2-yl-azétidine sont solubilisés dans 2 ml d'éthanol auxquels on ajoute 10 mg d'oxyde de platine. On agite sous atmosphère d'hydrogène pendant une heure.

On filtre le catalyseur et on évapore l'éthanol sous pression réduite.

Le résidu brut est purifié par filtration sur silica gel en éluant avec un mélange dichlorométhane/MeOH 95:5 et 85:15

On récupère 25 mg (61%)d'une huile incolore

CCM : Rf=0,1 (silicagel, éluant : CH2Cl2/ MeOH 90:10)

**1H-RMN (MeOD) :** δ 1,90 (m, 2H, **H7**) ; 2,75(m, 2H, **H6**) ; 3,41(m, 2H, **H8**) ; 3,96 (m, **1H**, **H3**) ; 4,30(m, 4H, **H2** et **H2'**); 6,40(d, 1H, **H4**) et 7,15 (d, 1H, **H5**)

**SM** : 190 (MH+)

Synthèse de la 2,5-diméthyl-4,6-dihydroxy-pyrimidine :

Un monocol contenant 40 ml de méthanol, placé sous atmosphère d'azote, est refroidit à 0˚C par un bain de glace, on additionne 9,72 g de méthylate de sodium (soit une solution de concentration c=3 $mol.l^{-1}$) au mélange réactionnel puis on ajoute à 0˚C et par petites quantités 5 g (53 mmoles)de chlorhydrate de d'acétamidine. On laisse agiter à température ambiante pendant une vingtaine de minutes, puis on ajoute goutte à goutte 8,3 ml de méthylmalonate de diéthyle. On maintient l'agitation pendant 3 heures. Puis le méthanol est condensée sous pression réduite (2 kPa). Le brut obtenu est repris avec un minimum d'eau, refroidit à 0˚C puis acidifié avec de l'acide acétique pur jusqu'à un pH entre 4 et 5. Le précipité blanc formé est filtré, rincé à l'eau, éther éthylique et pentane. Puis le produit blanc est séché sur $P_2O_5$ sous pression réduite (0,2 kPa). On obtient 3,3 g de produit attendu.**CCM** : Rf=0,2 (Silicagel, éluant : dichlorométhane-méthanol-eau-acide acétique 85-15-2-2).**1H-RMN** (DMSO d6) : δ 1,68 (s, 3H, OH-CH=C-CH3); 2,18 (s, 3H, N=C-CH3).

Synthèse de la 2,5-diméthyl-4,6-dichloro pyrimidine :

Un mélange de 3,3 g (23,5 mmoles) de 2,5-diméthyl-4,6-dihydroxy-pyrimidine et 15 ml d'oxychlorure de phosphore, est porté au reflux pendant 8 heures. Après retour à température ambiante,le mélange réactionnel est versé lentement sur un mélange de glace et d'eau. Cette phase aqueuse est extraite à l'acétate d'éthyle. La phase organique est lavée avec une solution saturée de bicarbonate de sodium puis séchée sur du sulfate de magnésium et évaporée à sec sous pression réduite (2 kPa). On obtient 3,39 g de produit attendu.
**CCM** : Rf=0,9 (Silicagel, éluant : acétate d'éthyle 100%)
**1H-RMN** ($CDCl_3$) : δ 2,46 (s, 3H, C1-CH=C-$CH_3$); 2,68 (s, 3H, N=C-$CH_3$)
**SM :** 177/179 (MH+).

Synthèse du 7-[1-(6-Chloro-2,5-diméthyl-pyrimidin-4-yl)-azétidin-3-yl]-1,2,3,4-tétrahydro-[1,8]naphthyridine:

On dissout 90 mg (0,47 mmol) de 7-azétidin-3-yl-1,2,3,4-tétrahydro-[1,8]naphthyridine dans 2 ml de diméthylacétamide auxquels on additionne 90 mg (0,51 mmol) 2,5-diméthyl-4,6-dichloro pyrimidine.
Ce mélange est chauffé à 100˚C pendant 18 heures.
On évapore le solvant sous pression réduite(2 kPa).
Le résidu brut est purifié par chromatographie sur silica gel en éluant avec un mélange dichlorométhane/MeOH 95:5
On récupère 50 mg(32%) de produit attendu.
**CCM :** Rf=0,25 (Silicagel, éluant : CH2C12/MeOH 95:5)
**SM :** 330 (MH+).

2-benzyloxycarbonylamino-3-{2,5-diméthyl-6-[3-(5,6,7,8-tétrahydro-[1,8]naphthyridin-2-yl)-azétidin-1-yl]-pyrimidin-4-ylamino}-propionate de tert-butyle :

On solubilise dans 2 ml de dioxane 50 mg(0,150 mmol) de 7-[1-(6-Chloro-2,5-diméthyl-pyrimidin-4-yl)-azétidin-3-yl]-1,2,3,4-tétrahydro-[1,8] naphthyridine, 53 mg de 3-amino-2-benzyloxycarbonylamino propionate de tert-butyle et 70 mg (0,460 mmol) de fluorure de césium.
Ce mélange est chauffé 5 minutes à 90˚C puis on y additionne 6 mg (0,01 mmol) de rac-2,2'-Bis(diphenylphosphino)-1,1'-Binaphtyl et 9 mg (0,01 mmol) de tris (dibenzylideneacétone) dipalladium. Ce mélange est chauffé à 110˚C pendant 2 heures sous agitation magnétique. On ajoute de nouveau les mêmes quantités de catalyseur et de fluorure de césium et chauffe pendant 2 heures à 140˚C. On évapore le dioxane sous pression réduite (2 kPa).
Le résidu obtenu est purifié par chromatographie sur silicagel en éluant avec un mélange Heptane/Acétate d'éthyle 1:1.
On récupère 25 mg de produit attendu sous la forme d'un solide jaune
**CCM :** Rf=0,25 (Silicagel, éluant : CH2Cl2/MeOH 95:5)
**SM :** 588 (MH+); 398(MH-(tBu et -CO-O-CH2-Ph)+.

Acide 2-benzyloxycarbonylamino-3-{2,5-diméthyl-6-[3-(5,6,7,8-tétrahydro-[1,8]naphthyridin-2-yl)-azétidin-1-yl]-pyrimidin-4-ylamino}-propionique bis(trifluoroacetate) :

On solubilise dans 1 ml de $CH_2Cl_2$ 20 mg de 2-benzyloxycarbonylamino-3-{2,5-diméthyl-6-[3-(5,6,7,8-tétrahydro-[1,8]naphthyridin-2-yl)-azétidin-1-yl]-pyrimidin-4-ylamino}-propionate de tert-butyle_et on y ajoute 0,1 ml de d'acide trifluoroacétique.

On agite cette solution 24 heures à température ambiante et dilue avec du toluène.

On évapore les solvants et l'acide trifluoroacétique sous pression réduite (2 kPa). On reprend le résidu obtenu avec un minimum de $CH_2Cl_2$ puis on coule cette solution sur de l'éther isopropylique.

On isole ainsi par filtration l'insoluble et on récupère 15 mg de produit attendu sous forme de poudre blanche.
**CCM :** Rf=0,1 (Silicagel, éluant : $CH_2Cl_2$/MeOH 90:10)
**1H-RMN (MeOD) :** δ de 1,95 à 2.00 (m, 5H, **H1** et **H11**) ; 2,50(s, 3H, **H3**) ; 3,35(dd, 2H, **H10) ;** 3,52(dd, 2H, **H12) ;** 3,30 (m, 1H, **H7) ;** 3,97 et 4,15 (2m, 2H, **H4) ;** 4,53 (m, 3H, **H5** + **H2** ou **H2'**) ; 4,80 (dd partiellement masqué par H2O, 2H, H2 ou H2'); 5,10(m, 2H, -O-C**H2**-C6H5); 6,82(d, 1H, **H8) ;** de 7,30 à 7,40(m, 5H, -O-CH2-C6**H5**) ; 7,62(d, 1H, **H9**)
**SM** : 532(MH+); 398(MH-(CO-O-CH2-C6H5)+)

Exemple comparitif 6

Synthèse du 2-benzyloxycarbonylamino-3-[6-(6-amino-3',4',5',6'-tétrahydro-2'H-[2,4']bipyridinyl-1'-yl)-5-méthyl-pyrimidin-4-ylamino]-propionate de tert-butyle

1°) Synthèse de la N-t.butoxycarbonyl-4-hydroxy-pipéridine :

On dissout 1 g (5 mmol) de N-t.butoxycarbonyl-4-oxo-1-pipéridine (commercialisée par Aldrich) dans 5 ml d'éthanol. On refroidit cette solution à 0°C à l'aide d'un bain de glace et on additionne par portion 200 mg (7,56 mmol) de tétraborohydrure de sodium et on agite 4 heures à température ambiante. On additionne sur le mélange réactionnel une solution aqueuse saturée en chlorure d'ammonium. On évapore l'éthanol sous pression réduite (2 kPa) puis on reprend le mélange réactionnel avec de l'acétate d'éthyle. On sépare la phase organique de la phase aqueuse. On répète une fois cette extraction et on rassemble ensuite les phases organiques que l'on sèche sur sulfate de magnésium. On concentre sous pression réduite (2 kPa) et l'on récupère ainsi 1,05 g d'une huile incolore.
**CCM** : Rf=0,5 (silicagel, éluant : CH2Cl2/ MeOH 90:10
**1H-RMN ($CDCl_3$) .** δ 1,47 (s, 9H, tBu) et (m, 2H; -CH**H**-CH2-N-CH2-C**H**H-); 1,87 (m, 2H, -CH**H**-CH2-N-CH2-CH**H**-); 3,04 (m, 2H, -CH**H**-N-C**H**H-) ; 3,85 (m, 2H, -C**H**H-N-CH**H**-) et (m, 1H, -C**H**-OH)

2°) Synthèse du N-t.butoxycarbonyl-4-iodo-1-pipéridine :

On dissout 2,15 g de triphénylphosphine(8,2 mmole) et 2,08 g d'iode (8,2 mmole) dans 30 ml d'acétonitrile.

On laisse agite pendant 10 min à température ambiante puis on ajoute 918 mg d'imidazole (13,5 mmol) et on laisse agiter encore 10 min à température ambiante. On ajoute ensuite 1 g (5 mmol) de N-t.butoxycarbonyl-4-hydroxy-1-pipéridine et on laisse agiter 24 heures à température ambiante. On traite la réaction en ajoutant une solution aqueuse de thiosulfate de sodium et on évapore l'acétonitrile sous pression réduite (2 kPa). On reprend à l'acétate d'éthyle, on extrait et on lave avec une solution aqueuse de thiosulfate de sodium. On sèche les phases organiques sur $MgSO_4$, on filtre et évapore l'acétate d'éthyle sous pression réduite (2 kPa). On chromatographie sur Silicagel en éluant au dichlorométhane puis dichlorométhane/méthanol 90:10.

On récupère 1,1 g (rdt=70%) d'huile incolore.
**CCM :** Rf=0,8 (silicagel, éluant : CH2C12/ MeOH 90:10
**1H-RMN ($CDCl_3$) :** δ 1,47 (s, 9H, tBu); 2.03 (m, 4H, -C**H2**-CHI-C**H2**-); 3,30 et 3,60 (2m, 4H, -C**H2**-N-C**H2**-) ; 4,46 (m, 1H -C**H**I-)

3°) Synthèse de la 2-bromo-6(2,5-diméthyl-pyrol-1-yl)-pyridine (3) :

Dans un ballon de 100 ml surmonté d'un montage de Dean Stark on place 1 g (5,78 mmol) de 2-amino-6-bromopyridine dans 30 ml de toluène. Ajoute 0,3 ml d'acide acétique et 0,8 ml (6,78 mmol) d'acétonylacétone. On chauffe au reflux du toluène pendant 5 heures. On laisse revenir à température ambiante et on évapore le toluène sous pression réduite (2 kPa). On ajoute de l'eau et on extrait à l'acétate d'éthyle. On rassemble les phases organiques que l'on sèche sur sulfate de magnésium. On évapore l'acétate d'éthyle sous pression réduite(2 kPa) et le résidu brut est purifié par chromatographie sur silica gel en éluant au dichlorométhane.

On récupère 1 g de produit attendu sous forme de poudre jaune.
**CCM :** Rf=0,7 (silicagel, éluant : $CH_2Cl_2$)
**1H-RMN ($CDCl_3$) :** δ 2,20 (s, 6H, -C**H3**C=CH-CH=CC**H3**-) ; 5,90 (s, 2H, -CH3C=C**H**-C**H**=CCH3-) ; 7,08 (d, 1H, H3 ou H5) ; 7,16 (d, 1H, H3 ou H5) ; 7,29(t, 1H, H4).

4°) Synthèse du 6-(2,5-diméthyl-pyrrol-1-yl)-3',4',5',6'-tétrahydro-2'H-[2,4']bipyridinyl-1'-carboxylic acid tert-butyl ester :

Sous atmosphère d'argon, on met en suspension 284 mg (4,34 mmol)de zinc électrolytique auxquels on ajoute 0,033 ml de 1,2-dibromoéthane et 1 ml de tétrahydrofurane.

On agite 3 min à 60°C et on laisse revenir à température ambiante. On ajoute 0,047 ml de chlorure de triméthylsilyl et on agite 30 min à température ambiante. On additionne 1 g (3,2 mmol) de N-t.butoxycarbonyl-4-iodo-1-pipéridine solubilisé au préalable dans 2 ml de tétrahydrofurane. Ongite ce mélange réactionnel 45 min à température ambiante et on l'additionne à une solution contenant 30 mg (0,032 mmol) de tris(dibenzylideneacétone) dipalladium commercialisé par Aldrich et 30 mg (0,13 mmol) de tris(2-furyl)phosphine. On ajoute ensuite 1 g (4 mmol) de 2-bromo-6(2,5-diméthyl-pyrol-1-yl)-pyridine solubilisé au préalable dans 10 ml de tétrahydrofurane. Le mélange réactionnel est laissé sous agitation magnétique à 60°C pendant 2 heures. On laisse revenir à température ambiante, on filtre sur clarcel et on extrait entre acétate d'éthyle et une solution aqueuse saturée en bicarbonate de sodium. On extrait 2 fois la phase aqueuse à l'acétate d'éthyle, puis on rassemble les phases organiques que l'on sèche sur sulfate de magnésium. On évapore l'acétate d'éthyle sous pression réduite (2 kPa) et le résidu brut est purifié par chromatographie sur silicagel en éluant avec un mélange heptane/ acétate d'éthyle 4:1. On récupère 350 mg de produit attendu sous forme d'huile jaune.
**CCM :** Rf=0,2 (silicagel, éluant : Heptane/Acétate d'éthyle 90:10).
**1H-RMN ($CDCl_3$) :** δ 1,50 (s, 9H, tBu) ; 1,78 et 1,97 (m, 4H, -C**H2-**CH2-N-CH2-C**H2**-) ; 2,18 (s, 6H, -C**H3**C=CH-CH=CC**H3**-) ; 2,85 et 2,95 (m, 3H, C**H**-CH2-C**H**H-N-C**H**H-CH2-) ; 4,28 (m, 2H, -CH2-CH**H**-N-CH**H-**CH2-); 5,92 (s, 2H, -CH3C=C**H**-C**H**=CCH3-) ; 7,08 (d, 1H, H3 ou H5); 7,16 (d, 1H, H3 ou H5); 7,29 (t, 1H, H4).

5°) Synthèse du 6-(2,5-diméthyl-pyrrol-1-yl)-l',2',3',4',5',6'-hexahydro-[2,4']bipyridinyle

On met en solution 330 mg (0,928 mmol) de 6-(2,5-diméthylpyrrol-1-yl)-3',4',5',6'-tétrahydro-2'H-[2,4']bipyridinyl-1'-carboxylic acid tert-butyl ester dans 3ml de dichlorométhane auxquels on ajoute 0,3 ml d'acide trifluoroacétique. On agite pendant 2 heures à température ambiante. On évapore le dichlorométhane sous pression réduite (2 kPa). On reprend le résidu obtenu à l'eau, on basifie jusqu'à pH = 10 avec de l'ammoniaque concentré et on extrait le produit au dichlorométhane. La phase organique est séchée sur sulfate de magnésium et on évapore le dichlorométhane sous pression réduite (2 kPa). On récupère 220 mg (Rdt=92%) d'huile jaune.
**CCM :** Rf=0,3 (silicagel, éluant : CH2Cl2/ MeOH 90:10)
**1H-RMN ($CDCl_3$) :** δ 1,90 et 2.08 (m, 4H, -C**H2**-CH2-N-CH2-C**H2**-); 2,18 (s, 6H, -C**H3**C=CH-CH=CC**H3**-) ; 2,88 et 3,34 (m, 4H, -CH2-C**H2-**N-C**H2**-CH2-) ; 2,95,(m, 1H, C**H**-CH2-CH2-N-CH2-) ; 4,10 (m, 1H, NH) ; 5,92 (s, 2H, -CH3C=C**H**-C**H**=CCH3-) ; 7.08 (d, 1H, H3 ou H5); 7,16 (d, 1H, H3 ou H5) ; 7,29(t, 1H, H4)
**SM :** 256 (MH+).

6°) Synthèse du 1'-(6-chloro-5-méthyl-pyrimidin-4-yl)-6-(2,5-diméthyl-pyrrol-1-yl)-1',2',3',4',5',6'-hexahydro-[2,4'] bipyridinyle :

On dissout 220 mg (0,860 mmol) de 6-(2,5-diméthyl-pyrrol-1-yl)-1',2',3',4',5',6'-hexahydro-[2,4']bipyridinyle dans 2 ml de diméthylacétamide auxquels on ajoute 140 mg (0,860 mmol) de 4,6-dichloro-5-méthyl-pyrimidine commercialisée par et 0,2 ml de diisopropyléthylamine. Le mélange est chauffé à 110°C sous agitation magnétique pendant une heure. On laisse revenir le mélange à température ambiante et on évapore la diméthylacétamide sous pression réduite (0,2 kPa). On reprend le résidu brut à l'acétate d'éthyle et lave à l'eau. On extrait la phase aqueuse 2 fois à l'acétate d'éthyle, les phases organiques sont rassemblées et séchées sur sulfate de magnésium. On évapore l'acétate d'éthyle sous pression réduite (0,2 kPa) et on récupère 330 mg de résine marron qui sont mis en jeu pour le stade suivant sans purification.
**CCM :** Rf=0,4 (silicagel, éluant : $CH_2Cl_2$)
**1H-RMN ($CDCl_3$) :** δ 2,02 et 2,10 (m, 4H, -C**H2**-CH2-N-CH2-C**H2**-) ; 2,18 (s, 6H, -C**H3**C=CH-CH=CC**H3**-) ; 2,30(s, 3H, CH3); 3.08 et 4,01 (m, 4H, -CH2-C**H2**-N-C**H2**-CH2-) ; 3,02,(m, 1H, C**H**-CH2-CH2-N-CH2-) ; 5,92 (s, 2H, -CH3C=C**H**-C**H**=CCH3-) ; 7,09 (d, 1H, H3 ou H5) ; 7,20 (d, 1H, H3 ou H5) ; 7,29(t, 1H, H4) ; 8,41 (s, 1H,=N-CH=N)

Synthèse du 2-benzyloxycarbonylamino-3-{6-[6-(2,5-diméthylpyrrol-1-yl)-3',4',5',6'-tétrahydro-2'H-[2,4']bipyridinyl-1'-yl]-5-méthyl-pyrimidin-4-ylamino}-propionate de tert-butyle :

On dissout 330 mg (0,866 mmol) de 1'-(6-chloro-5-méthylpyrimidin-4-yl)-6-(2,5-diméthyl-pyrrol-1-yl)-1',2',3',4',5',6'-hexahydro-[2,4']bipyridinyle dans 5 ml de diméthoxyéthane. On ajoute successivement 286 mg (1 mmol) 3-amino-2-benzyloxycarbonylamino propionate de tert-butyle 184 mg (1,21 mmol) de fluorure de Césium, 54 mg (10% mol) de 2,2'-bis(diphenylphosphino)-1,1'-Binaphtyl, et 40 mg (5% mol) de tris(dibenzylideneacétone)dipalladium. Ce

mélange est chauffé à 100˚C pendant 18 heures sous agitation magnétique. On laisse revenir à température ambiante et on évapore le diméthoxyéthane sous pression réduite (2 kPa).On reprend le résidu obtenu à l'acétate d'éthyle et on lave avec une solution aqueuse saturée en bicarbonate de sodium. On extrait la phase aqueuse à l'acétate d'éthyle, rassemble les phases organiques que l'on sèche sur sulfate de magnésium. On évapore l'acétate d'éthyle sous pression réduite (2 kPa). Le résidu obtenu est purifié par chromatographie sur silicagel en éluant avec un mélange heptane/acétate d'éthyle 1:1. On récupère 200 mg de solide jaune.

**CCM :** Rf=0,2 (silicagel, éluant : Heptane/ acétate d'éthyle 1:1) **1H-RMN ($CDCl_3$) :** δ 1,50 (s,9H, tBu); 1,97 (s, 3H, CH3) ; 2,02 à 2,10(m, 4H, -C**H2**-CH2-N-CH2-C**H2**-); 2,18(s, 6H, -C**H3**C=CH-CH=CC**H3-**); 3,20 et 3,78(m, 4H, -CH2-C**H2**-N-C**H2**-CH2-) ; 3,00(m, 1H, C**H**-CH2-CH2-N-CH2-); de 3,85 à 4,00 (m,2H, NH-C**H2**-CHCOOtBuNH) ; 4,47(m,1 H,NH-CH2C**H**-COOtBuNH) ; 5,12(2H,-O-**CH2**-Phényl); 5,92 (s, 2H, -CH3C=C**H**-C**H**=CCH3-) ; 6,12(m,1H mobile); 7,09(d,1H, H3 ou H5) ; 7,21(d,1H,H3 ou H5) ; 7,78(t, 1H, H4) ; 7,45(m,5H aromatiques); 8,32(s, 1H,=N-CH=N)

**SM :** 641 (MH+),584(MH-tBu+)

Synthèse du 3-[6-(6-amino-3',4',5',6'-tétrahydro-2'H-[2,4']bipyridinyl-1'-yl)-5-méthyl-pyrimidin-4-ylamino]-2-benzyloxycarbonylamino-propionate de tert-butyle :

On dissout 100 mg (0,15 mmol) de 2-benzyloxycarbonylamino-3-{6-[6-(2,5-diméthyl-pyrrol-1-yl)-3',4',5',6'-tétrahydro-2'H-[2,4']bipyridinyl-1'-yl]-5-méthyl-pyrimidin-4-ylamino}-propionate de tert-butyle dans 3 ml d'éthanol et 0,3 ml d'eau. On ajoute 50 mg (0,75 mmol) de chlorhydrate d'hydroxylamine et on chauffe 18 heures à 90˚C. On évapore les solvants sous pression réduite (2 kPa) et on purifie le résidu brut par chromatographie sur silicagel en éluant avec un mélange $CH_2Cl_2$/MeOH 90:10. On obtient 30 mg de produit attendu sous forme de résine.

**CCM :** Rf=0,5 (silicagel, éluant : CH2Cl2/ MeOH 90:10)

**1H-RMN ($CDCl_3$) :** δ 1,50 (s,9H, tBu); 1,97(s,3H, CH3) ; 2.02 à 2,10(m, 4H, -C**H2**-CH2-N-CH2-C**H2**-) ; 2,18(s, 6H, -C**H3**C=CH-CH=CC**H3-**) ; 3,20 et 3,78(m, 4H, -CH2-C**H2**-N-C**H2**-CH2-) ; 3,00(m, 1H, C**H**-CH2-CH2-N-CH2-); de 3,85 à 4,00(m,2H, NH-**CH2-**CHCOOtBuNH) ;4,47(m,1H,NH-CH2C**H**-COOtBuNH); 5,12(2H,-O-**CH2-**Phényl); 5,92 (s, 2H, -CH3C=C**H**-C**H**=CCH3-) ; 6,18, 6,57,6,62(3d,H3 et H5 +1H mobile); 7,45(m,5H aromatiques); 7,60(t, 1H, H4); 8,32(s, 1H,=N-CH=N).

**SM :** 562 (MH+),372(MH-tBu et -CO-O-benzyl+).

Synthèse du 2-benzyloxycarbonylamino-3-[6-(6-éthylamino-3',4',5',6'-tétrahydro-2'H-[2,4']bipyridinyl-1'-yl)-5-méthyl-pyrimidin-4-ylamino]-propionate de tert-butyle :

50 mg (0,09 mmol) de 2-benzyloxycarbonylamino-3-[6-(6-amino-3',4',5',6'-tétrahydro-2'H-[2,4']bipyridinyl-1'-yl)-5-méthylpyrimidin-4-ylamino]-propionate de tert-butyle sont solubilisés dans 2 ml de dichlorométhane. L'ensemble est placé sous atmosphère d'argon et refroidi à 0˚C à l'aide d'un bain de glace.

On additionne 0,01 ml (0,18 mol) d'acétaldéhyde et 30 mg (0,14 mmol) de triacétoxyborohydrure de sodium.

On agite ce mélange pendant 1 heure à température ambiante et ajoute à nouveau les mêmes quantités d'acétaldéhyde et de triacétoxyborohydrure de sodium.

L'agitation à température ambiante est maintenue pendant 2 heures puis on ajoute enfin 30 mg supplémentaires de triacétoxyborohydrure de sodium.

On laisse agiter à température ambiante pendant 15 minutes puis évapore le dichlorométhane sous pression réduite(2 kPa).

Le résidu brut est purifié par chromatographie sur Silicagel en éluant au dichlorométhane/méthanol 95:5 puis 90:10.

On récupère 40 mg de produit attendu.

**CCM :** Rf=0,7 (silicagel, éluant : CH2Cl2/ MeOH 90:10

**1H-RMN (CDCl3) :** δ 1,30(t, 3H, **H1**) ; 1,45(s, 9H, **tBu**) ; 1,95(s, 3H, **H9**) ; de 1,85 à 2.05(m, 4H, **H7** et **H7'**) ; 2,72(m, 1H, **H6**) ; 2,95(m, 2H, **H8** ou **H8'**) ; 3,30(m, 2H, **H2**) ; 3,68(m, 2H, **H8** ou **H8'**) ; 3,90(m, 2H, **H11**) ; 4, 45 (m, 1H, **H12**) ; 4, 95 (m, 1H, H mobile) ; 5,12(s, 2H, - O-C**H2**-C6H5) ; 6,19(m, 1H, H mobile) ; 6,30(d, 1H, **H3**) ; 6,50(d, 1H, **H5**) ; 7,35 (m, 5H, O-CH2-C6**H5**) ; 7,46(dd, 1H, **H4**) ; 8,30(s, 1H, **H10**)

**SM :** 590=(MH+), 534=(MH-tBu+), 400=MH-(tBu et - CO-O-$CH_2$-$C_6H_5$)+

Synthèse de l'acide 2-benzyloxycarbonylamino-3-[6-(6-éthylamino-3',4',5',6'-tétrahydro-2'H-[2,4']bipyridinyl-1'-yl)-5-méthylpyrimidin-4-ylamino]-propionique bis(trifluoroacetate) :

300 mg (0,51 mmol) de 2-benzyloxycarbonylamino-3-[6-(6-éthylamino-3',4',5',6'-tétrahydro-2'H-[2,4']bipyridinyl-1'-yl)-5-méthyl-pyrimidin-4-ylamino]-propionate de tert-butyle sont solubilisés dans 5 ml de dichlorométhane auxquels on additionne 0,5 ml d'acide trifluoroacétique.

Ce mélange est agité à température ambiante pendant 4 heures.

On dilue au toluène et on évapore l'acide trifluoroacétique, le dichlorométhane et le toluène sous pression réduite (2 kPa).

On reprend le résidu obtenu avec un minimum de $CH_2Cl_2$ puis on coule cette solution sur de l'éther isopropylique.

On filtre le précipité et l'on récupère 220 mg de produit attendu sous forme d'un solide beige.

**CCM :** Rf=0,7 (silicagel, éluant : CH2Cl2/ MeOH 85 :15

**1H-RMN (MeOD) :** δ 1,32(t, 3H, **H1**) ; 2.00(s, 3H, **H9**); de 1,90 à 2,10(m, 4H, **H7** et **H7') ;** 2,95 à 3,15(m, 3H, **H6** et **H8** ou **H8'**) ; 3,45(m, 2H, **H2**); 3,75(m, 2H, **H8** ou **H8'**); 3,80 et 4.00(2m, 2H, **H11**) ; 4,50(m, 1H, **H12**) ; 5,10(m, 2H, -O-**CH2**-C6H5) ; 6,80(d, 1H, **H3**) ; 6,90(d, 1H, **H5**) ; de 7,35 à 7,40(m, 5H, O-CH2-C6**H5**) ; 7,86(dd, 1H, **H4**) ; 8,20(s, 1H, **H10**)

**SM :** 534=(MH+), 400=MH-(CO-O-CH2-C6H5)+

Exemples comparatifs 7 à 11

En opérant de manière analogue à la méthode décrite dans l'exemple comparatif 6, on prépare les produits suivants :

| Exemple comparatif | Ester formé | Rf | SM (MH+) | Acide formé | FW | SM (MH+) | Rf |
|---|---|---|---|---|---|---|---|
| 7 | 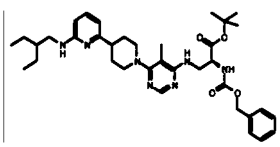 | 0.3 | 646 | 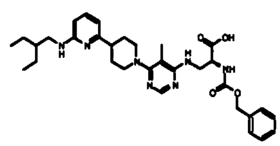 | 589 | 590 | 0.25 |
| 8 | 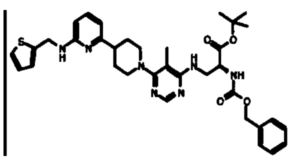 | 0.3 | 659 | 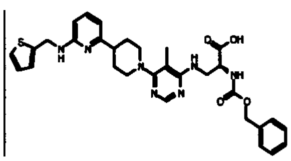 | 601 | 602 | 0.25 |
| 9 | 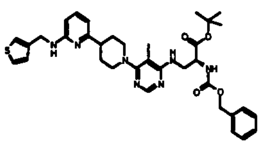 | 0.3 | 659 | 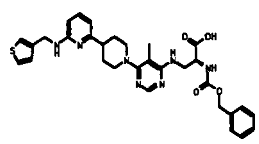 | 601 | 602 | 0.25 |
| 10 | 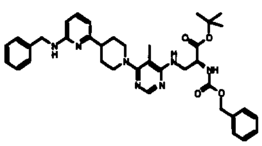 | 0.3 | 653 | 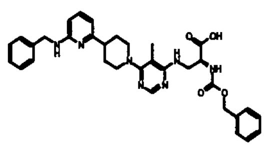 | 595 | 598 | 0.25 |
| 11 | 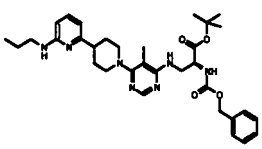 | 0.3 | 604 | 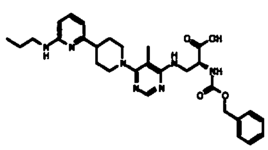 | 547 | 548 | 0.25 |

Exemple comparatif 12

Synthèse de la 2-méthyl-4,6-dihydroxy-5-méthoxy-pyrimidine.

A une solution de 5,5 g (58 mmoles) de chlorhydrate d' acétamidine dans 100 ml d'éthanol refroidie à 0°C on ajoute 10,8 g (200 mmoles) de méthylate de sodium et agite le mélange pendant 15 minutes; on additionne alors une solution de 7,2 ml (52 mmoles) de méthoxy malonate de diméthyle dans 50 ml d'éthanol et agite une nuit à température ambiante. On évapore le mélange réactionnel à sec sous pression réduite (2 kPa). Le résidu est repris par 100 ml d'une solution saturée en chlorure de sodium et extrait par 800 et 200 ml de n-butanol. les phases organiques sont séchées sur sulfate de sodium, filtrées et évaporées à sec sous pression réduite (2 kPa). On obtient 2,4 g de produit attendu sous forme d'une solide blanc.

**1H-RMN ($CD_3OD$) :** δ 2,31 ppm (s, 3H, C-$\underline{CH_3}$) ; 3,74 (s, 3H, C-O$\underline{CH_3}$) .

**SM :** 157 (MH+).

Synthèse de la 4,6-dichloro-2-méthyl-5-méthoxy-pyrimidine.

Un mélange de 1,9 g (12,2 mmoles) de 2-méthyl-4,6-dihydroxy-5-méthoxy-pyrimidine dans 60 ml d'oxychlorure de phosphore est porté au reflux durant 2 heures. Après retour à température ambiante, le mélange réactionnel est versé sur un mélange de glace et d'eau puis on ajoute doucement du bicarbonate de sodium jusqu'à pH basique. On extrait à l'acétate d'éthyle, sèche sur sulfate de magnésium et évapore à sec sous pression réduite (2 kPa). On obtient 0,67 g (Rdt=28%) d'une huile marron.
**CCM :** Rf=0,40 (silicagel, éluant : heptane-acétate d'éthyle 90-10) .
**1H-RMN ($CDCl_3$) :** δ 2,68 ppm(s, 3H, C-$\underline{CH_3}$) ; 3,96(s, 3H, C-O$\underline{CH_3}$).
**SM :** 193,195 (MH+).

Synthèse de la 2-pipéridin-4-yl-[1,8]naphthyridine :

51 g (300 mmoles) de 4-(2-méthyl-[1,3]dioxolan-2-yl)-pipéridine(preparé selon J. Org. Chem.; 29; 1964; 2898-2903) sont solubilisés dans 550 ml d'éthanol.
Ce mélange est refroidi à 0°C à l'aide d'un bain de glace.
On additionne 85 ml d'acide chlorhydrique 6N (510 mmoles).
Le mélange est chauffé au reflux de l'éthanol pendant 24h.
On ajoute ensuite 20,4 g (510 mmoles) de soude en pastille pour neutraliser le pH puis 32,13 g (260 mmoles) de 2-aminopyridine-3-carboxyaldéhyde et 200 ml d'éthanol.
Enfin on additionne 40,8 g (295 mmoles) de carbonate de potassium.
On agite ce mélange à 100°C pendant 8 heures puis 18 heures à température ambiante.
On évapore sous pression réduite l'éthanol, on dilue à l'eau et au butanol. On sépare les 2 phases et on extrait ainsi le produit au butanol.
La phase organique est séchée sur sulfate de sodium puis filtrée et évaporée sous pression réduite.
On récupère un solide marron 65 g dont utilisé sans purification pour la suite.
**CCM :** Rf=0,1 (silicagel, éluant : CH2Cl2/ MeOH/ NH4OH 89:10:1
**1H-RMN (MeOD)** : δ de 1,90 à 2,10(m, 4H, H7 et H7') ; de 2,8 à 2,90(m, 2H, H8 ou H8') ; de 3,12 à 3,28(m, 3H, H6 + H8 ou H8') ; de 7,58 à 7,68(m, 2H, H2 et H5) ; de 8,38 à 8,45(m, 2H, H3 et H4) ; 9.03(m, 1H, H1).
**SM** : 270 (MH+)

Synthèse du 2-[1-(6-Chloro-5-méthoxy-2-méthyl-pyrimidin-4-yl)-pipéridin-4-yl]-[1,8]naphthyridine :

Dans un monocol contenant 1,1 g (5,2 mmoles) de 2-Pipéridin-4-yl-[1,8]naphthyridine on ajoute 30 ml de diméthylacétamide, 0,9 g (4,66 mmoles) 4,6-dichloro-2-méthyl-5-méthoxy-pyrimidine et 3 ml de diisopropyléthylamine. Ce mélange est chauffé à 120°C pendant 4 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant est évaporé sous pression réduite (2 kPa). Le résidu est chromatographié sur silicagel en éluant avec un gradient d'heptane-acétate d'éthyle de 100-0 à 0-100. On obtient 870 mg de produit attendu sous la forme d'un solide jaune.
**CCM :** Rf=0,25 [silicagel, éluant : acétate d'éthyle (100%)]
**1H-RMN ($CDCl_3$) :** δ 2,16ppm (m, 4H, N-CH2-CH2-CH-CH2) ; 2,47 (s, 3H, C-$\underline{CH_3}$) ; 3,11 et 4,84 (m et dl, 4H, CH2-CH2-N-CH2-CH2) ; 3,26 (m, 1H, CH2-CH-CH2,) ; 3,75 (s, 3H, C-O$\underline{CH_3}$) ; 7,45 et 8,16 (2d, 2H, C-CH=CH-C(CH) =N) ; 7,47 (dd, 1H, N-CH=CH-CH=C) ; 8,18 (dd, 1H N-CH=CH-CH=C) ; 9,11 (dd, 1H, N-CH=CH-CH=C).
**SM :** 370,372 (MH+).

Synthèse du 2-benzyloxycarbonylamino-3-[5-méthoxy-2-méthyl-6-(4-[1,8]naphthyridin-2-yl-pipéridin-1-yl)-pyrimidin-4-ylamino]-propionate de tert-butyle

On chauffe au reflux un mélange de 870 mg (2,36 mmoles) de 2-[1-(6-Chloro-5-méthoxy-2-méthyl-pyrimidin-4-yl)-pipéridin-4-yl]-[1,8]naphthyridine, de 766 mg (2,59 mmoles) de 3-amino-2-benzyloxycarbonylamino propionate de tert-butyle, de 540 mg (3,57 mmoles) de fluorure de césium, de 120 mg (0,124 mmole) de tris(dibenzylidèneacétone) dipalladium(0), et 70 mg (0,108 mmole) de 2,2'-bis(diphényl-phosphino)-1,1'-binaphtyl dans 50 ml de dioxane pendant 6 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate

de magnésium et le solvant évaporé sous pression réduite (2 kPa). Le résidu est chromatographié une première fois sur alumine en éluant avec acétate d'éthyle-éther éthylique 50-50 puis une deuxième fois sur silice en éluant avec acétate d'éthyle-heptane 50-50. On obtient 820 mg de produit attendu sous forme de solide amorphe blanc.
**CCM :** Rf=0,30 (silicagel, éluant : acétate d'éthyle).
**1H-RMN ($CDCl_3$) :** δ 1,47ppm (s, 1H, tBu) ; 2,07 à 2,16 (m, 4H, N-CH2-CH2-CH-CH2) ; 2,38 (s, 3H, C-$CH_3$) ; 2,97 et 4,53 (m et dl, 4H, CH2-CH2-N-CH2-CH2) ; 3,19 (m, 1H, CH2-CH-CH2,) ; 3,63 (s, 3H,C-O$CH_3$) ; 3,86 (m, 2H, NH-$CH_2$-CH-NH) ; 4,36 (m, 1H, NH-$CH_2$-CH-NH) ; 5,12 (d, 2H, $CH_2$-Ph) ;; 7,35 (m, 5H, CH2Ph); 7,46 et 8,15 (2d, 2H, C-CH=CH-C(CH)=N) ; 7,48 (dd, 1H, N-CH=CH-CH=C) ; 8,18 (dd, 1H, N-CH=CH-CH=C) ; 9,10 (dd, 1H, N-CH=CH-CH=C).
**SM :** 628 (MH+); 438 (MH- COOCH2Ph+).

Synthèse du 2-benzyloxycarbonylamino-3-{5-méthoxy-2-méthyl-6-[4-(5,6,7,8-tétrahydro-[1,8]naphthyridin-2-yl)-pipéridin-1-yl]-pyrimidin-4-ylamino}-propionate de tert-butyle

On agite un mélange de 820 mg (1,3 mmoles) de 2-benzyloxycarbonylamino-3-[5-méthoxy-2-méthyl-6-(4-[1,8] naphthyridin-2-yl-pipéridin-1-yl)-pyrimidin-4-ylamino]-propionate de tert-butyle et de 30 mg d'oxyde de platine dans 50 ml d'éthanol pendant 2 heures sous pression atmosphérique d'hydrogène. Le mélange réactionnel est filtré sur clarcel puis évaporé à sec sous pression réduite (2 kPa). Le résidu est cristalisé dans de l'éther diisopropylique. On obtient 570 mg de produit attendu sous forme d'un solide blanc amorphe.
**CCM :** Rf=0,28 (silicagel, éluant : acétate d'éthyle).
**1H-RMN ($CDCl_3$) :** δ 1,48 ppm (s, 1H, tBu); 1,67 et 1,97 (m, 6H, $CH_2$-$CH_2$-$CH_2$-NH et $CH_2$-CH-$CH_2$) ; 2,35 (s, 3H, C-$CH_3$) ; 2,65 (tt, 1H, $CH_2$-CH-$CH_2$) ; 2,71 (t, 2H, $CH_2$-$CH_2$-$CH_2$-NH) ; 2,87 et 4,47 (tl et dl, 4H, CH2-CH2-N-CH2-CH2) ; 3,41 (m, 2H, $CH_2$-$CH_2$-$CH_2$-NH) ; 3,57 (s, 3H,C-O$CH_3$) ; 3,84 (m, 2H, NH-$CH_2$-CH-NH) ; 4,36 (m, 1H, NH-$CH_2$-CH-NH); 5,12 (d, 2H, $CH_2$-Ph) ; 6,38 et 7,11 (2d, 2H, C=CH=CH=C) ; 7,36 (m, 5H, CH2Ph) ; 4,75 et 5,27 (m; tl et dl H mobiles).
**SM :** 632 (MH+); 442 (MH- COOCH2Ph+).

Exemple comparatif 13

Synthèse de l'acide 2-benzyloxycarbonylamino-3-{2-méthoxy-5-méthyl-6-[4-(5,6,7,8-tétrahydro-[1,8]naphthyridin-2-yl)-pipéridin-1-yl]-pyrimidin-4-ylamino}-propionique bis(trifluoroacetate).

On agite 280 mg (0,443 mmole) de 2-benzyloxycarbonylamino-3-{5-méthoxy-2-méthyl-6-[4-(5,6,7,8-tétrahydro-[1,8]naphthyridin-2-yl)-pipéridin-1-yl]-pyrimidin-4-ylamino}-propionate de tert-butyle dans 15 ml de dichlorométhane avec 3 ml d'acide trifluoroacétique à température ambiante pendant 18 heures. On rajoute du toluène et évapore le mélange réactionnel à sec sous pression réduite (2 kPa). Le résidu est solubilisé dans le minimum de dichlorométhane puis versé sur de l'éther diisopropylique. On obtient par filtration 210 mg de produit attendu sous forme d'un solide blanc.
**CCM :** Rf=0,45 (silicagel, éluant : chlorure de méthylène-méthanol-eau-acide acétique 90-10-1-1).
**1H-RMN ($CDCl_3$) :** δ 1,75 et 2,10ppm (m, 6H, $CH_2$-$CH_2$-$CH_2$-NH et $CH_2$-CH-$CH_2$) ; 2,50 (s, 3H, C-$CH_3$) ; 2,78 (tl, 2H, $CH_2$-$CH_2$-$CH_2$-NH) ; 3,05 (tl, 1H, $CH_2$-CH-$CH_2$) ; 3,50 et 4,79 (tl et dl, 4H, CH2-CH2-N-CH2-CH2) ; 3,51 (m, 2H, $CH_2$-$CH_2$-$CH_2$-NH) ; 3,57 (s, 3H, C-O$CH_3$) ; 3,95 et 4,01 (2m, 2H, NH-$CH_2$-CH-NH) ; 4,51 (m, 1H, NH-$CH_2$-CH-NH) ; 5,11 (d, 2H, $CH_2$-Ph); 6,39 et 7,39 (2d, 2H, C=CH=CH=C) ; 7,30 (m, 5H, CH2Ph) ; 9,71 (m H mobiles).
**SM :** 576(MH+); 442(MH-COO$CH_2$Ph+) ; 574(MH-); 466(574-O$CH_2$Ph-) ; 1150(2M-).

Exemple comparatif 14 Synthèse de la 2-tert-butyl-5-méthyl-4,6-dihydroxy-pyrimidine :

Un monocol contenant 80 ml d'éthanol est refroidit à 0°C par un bain de glace, on additionne 4 g (55,6 mmoles) d'éthylate de sodium et 3,8 g (27,8 mmoles)de chlorhydrate de tert-butylcarbamidine. On laisse le mélange réactionnel revenir à température ambiante, puis on ajoute goutte à goutte 5 ml (27,8 mmoles) de méthylmalonate de diéthyle ester. On maintient l'agitation à température ambiante pendant une nuit. L'éthanol est ensuite condensé sous pression réduite (2 kPa). Le brut obtenu est solubilisé dans un minimum d'eau (environ 40 ml), puis acidifié à 0°C avec de l'acide acétique pur jusqu'à un pH compris entre 4 et 5. Le précipité blanc formé est filtré, rincé sucessivement à l'eau, à l'éther éthylique et au pentane. Puis la poudre blanche obtenue est séché sur $P_2O_5$ sous pression réduite (0,2 kPa). On obtient 2,4 g de produit attendu.
**CCM :** Rf=0,56 (Silicagel, éluant : dichlorométhane-méthanol 90-10) .
**SM :** (MH+)=183, (MH-)=181
**1H-RMN (DMSOd6)** : δ 1,25 (s,9H,tert-butyl); 1,72 (s,3H, N=C-$CH_3$).

Synthèse de la 2-tert-butyl-4,6-dichloro-5-méthyl-pyrimidine

Un mélange de 2,91 g (15,9 mmoles) de 2-tert-Butyl-5-méthyl-4,6-dihydroxy-pyrimidine et 15 ml d'oxychlorure de phosphore, est porté au reflux pendant 1 heure 30 minutes. Après retour à température ambiante, le mélange réactionnel est versé lentement sur un mélange de glace, d'eau et de carbonate de sodium solide. Cette phase aqueuse est extraite à l'acétate d'éthyle. La phase organique est lavée plusieurs fois avec une solution saturée de bicarbonate de sodium jusqu'à neutralisation totale de l'acide puis séchée sur du sulfate de magnésium et évaporée à sec sous pression réduite (2 kPa). On obtient 3,27 g de produit attendu.
**CCM :** Rf=0,63 (Silicagel, éluant : cyclohexane-acétate d'éthyle 98-2)
**1H-RMN ($CDCl_3$) :** δ 1,38 (s,9H,tert-butyl) ; 2,45 (s,3H, N=C-$CH_3$) . **SM** : (MH+)=210.

Synthèse de la 2-[1-(2-tert-butyl-6-chloro-5-méthyl-pyrimidin-4-yl)-pipéridin-4-yl]-[1,8]naphthyridine :

Dans un monocol contenant 1,5 g (6,84 mmoles) de 2-tert-Butyl-4,6-dichloro-5-méthyl-pyrimidine et 1,46 g (6,84 mmoles) de 2-Pipéridin-4-yl-[1,8]naphthyridine, on ajoute 20 ml de diméthylacétamide et 5 ml (17,8 mmoles) de diisopropyléthylamine. Ce mélange est chauffé à 100°C pendant une nuit. Le lendemain on ajoute 0,2 équivalent de naphthyridine et on chauffe 6 heures de plus. le mélange réactionnel est ramené à température ambiante avant concentration à sec. Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur sulfate de magnésium puis le solvant évaporé sous pression réduite (2 kPa). Le résidu est chromatographié sur silicagel en éluant avec un gradient d'heptane-acétate d'éthyle (70-30) jusqu'à heptane-acétate d'éthyle (50-50). On obtient 1,77 g de produit attendu.
**CCM :** Rf=0,50 [silicagel, éluant heptane-acétate d'éthyle(50-50).
**1H-RMN ($CDCl_3$)** : δ 1,37 (s,9H, tert-butyl); 2,1 (m, 1H, cyclopropyl); 2,15 (m, 4H, N-CH2-<u>CH2</u>-CH-<u>CH2</u>-CH2) ; 2,27 (s, 3H, CH3) ; 3,1 et 4,05 (2m, 4H, CH2-<u>CH2</u>-N-<u>CH2</u>-CH2) ; 3,25 (m, 1H, N-CH2-CH2-CH-CH2-CH2) ; [(7,48; 8,18; 9,12), 3m, 5H, naphthyridine].
**SM** : 396(MH+).

Synthèse du 2-benzyloxycarbonylamino-3-[2-tert-butyl-5-méthyl-6-(4-[1,8]naphthyridin-2-yl-pipéridin-1-yl)-pyrimidin-4-ylamino]-propionate de tert-butyle

Un mélange de 1,7 g (4,2 mmoles) de 2-[1-(2-tert-Butyl-6-chloro-5-méthyl-pyrimidin-4-yl)-pipéridin-4-yl]-[1,8] naphthyridine et de 1,51 g (5,04 mmoles) de 3-amino-2-benzyloxycarbonylamino propionate de tert-butyle), en présence de 1,95 g (12,6 mmoles) de fluorure de cesium, de 266 mg (0,42 mmoles) de (2,2'-bis(diphénylphosphino)-1,1'-binaphthyl dans 20 ml de dioxane est porté au reflux, on ajoute à chaud 230 mg (0,21 mmoles) de tris-dibenzylidèneacétone) dipalladium(o) mis en solution dans 5 ml de dioxane, puis on laisse au reflux pendant 15 heures. Le mélange réactionnel est ensuite ramené à température ambiante, concentré à sec sous pression réduite (2 kPa) puis repris par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous pression réduite (2 kPa). Le résidu est chromatographié sur silice avec un gradient dichlorométhane-d'acétate d'éthyle (95-5) jusqu'à (70-30). On obtient 1,91 g de produit attendu.
**CCM :** Rf=0,17 (silicagel, éluant : dichlorométhane-acétate d'éthyle (70-30)
**1H-RMN ($CDCl_3$) :** δ 1,33 (s, 9H, tert-butyl); 1,48 (s, 9H, tBu); 1,95 (s, 3H, CH3); 2,08 à 2,25 (m,4H, N-CH2-<u>CH2</u>-CH-<u>CH2</u>-CH2) ; 3,0 et 3,8 (2m, 4H, CH2-<u>CH2</u>-N-<u>CH2</u>-CH2) ; 3,18 (m, 1H, N-CH2-CH2-<u>CH</u>-CH2-CH2) ; 3,83 à 4,0 (2m, 2H, NH-<u>CH2</u>-CH-NH) ; 4,35 (m, 1H, NH-CH2-<u>CH</u>-NH) ; 4,65 et 7,3 (2m, 2H, NH); 5,1 (s, 2H, O-<u>CH2</u>-Ph) ; 7,32 (m, 5H, phényl) ; [(7,47 ; 8,17 ; 9,12), (3m, 5H, naphthyridine).
**SM :** 654(MH+), 464(MH-CBZ-tbu)

Synthèse du 2-benzyloxycarbonylamino-3- [2-tert-butyl-5-méthyl-6-[4-(5,6,7,8-tétrahydro-[1,8]naphthyridin-2-yl-pipéridin-1-yl)-pyrimidin-4-ylamino]-propionate de tert-butyle

A 1,8 g (2,75 mmoles)de 2-benzyloxycarbonylamino-3-[2-tert-butyl-5-méthyl-6-(4-[1,8]naphthyridin-2-yl-pipéridin-1-yl)-pyrimidin-4-ylamino]-propionate de tert-butyle mis en solution dans 80 ml d'éthanol, on ajoute 120 mg d'oxyde de platine (IV). Le mélange réactionnel est alors purgé sous vide et surmonté d'un ballon de baudruche contenant de l'hydrogène. On laisse sous agitation et à température ambiante pendant 6 heures. Le mélange réactionnel est ensuite filtré sur clarcel et concentré à sec sous pression réduite (2 kPa). Le résidu est chromatographié sur silice avec un gradient dichlorométhane-méthanol (98-2). On obtient 1,6 g de produit attendu.
**CCM :** Rf=0,76 [silicagel, éluant : dichlorométhane-méthanol 90-10]

**1H-RMN ($CDCl_3$)** : δ 1,3 (s, 9H, tBu) ; 1,48 (s, 9H, tBu) ; 1,7 à 2,0 (m, 9H, CH3, CH2-CH2-CH2-NH, N-CH2-CH2-CH-CH2-CH2) ; 2,63 (m, 1H, N-CH2-CH2-CH-CH2-CH2) ; 2,71 (t, 2H, CH2-CH2-CH2-NH); 2,9 et 3,73 (2m, 4H, CH2-CH2-N-CH2-CH2) ;3,41 (m, 2H, CH2-CH2-CH2-NH) ; 3,8 à 4,3 (2m, 2H, NH-CH2-CH-NH) ; 4,35 (m, 1H, NH-CH2-CH-NH) ; [(4,56; 4,75; 7,03), 3m, 3H, NH); 5,08 (s, 2H, O-CH2-Ph) ; 6,4 et 7,12 (2d, 2H, naphthyridine); 7,32 (m, 5H, phényl).
**SM :** 658(MH+).

Exemple comparatif 15

Acide 2-benzyloxycarbonylamino-3- [2-tert-butyl-5-méthyl-6-[4-(5,6,7,8-tétrahydro-[1,8]naphthyridin-2-yl-pipéridin-1-yl)-pyrimidin-4-ylamino]- propionique (bis trifluoroacetate) :

On agite à température ambiante un mélange de 350 mg (0,53 mmoles) de 2-benzyloxycarbonylamino-3-[2-tert-butyl-5-méthyl-6-[4-(5,6,7,8-tétrahydro-[1,8]naphthyridin-2-yl-pipéridin-1-yl)-pyrimidin-4-ylamino]- propionate de tert-butyle mis en solution dans 6 ml de dichlorométhane et de 2,2 ml d'acide trifluoroacétique pendant 24 heures. On additionne alors du toluène et évapore à sec le mélange. Le résidu est solubilisé dans un minimum de dichlorométhane puis versé sur un mélange de pentane et d'éther diisopropylique. Le précipité est filtré. On obtient une poudre blanche impure que l'on doit purifier sur silice avec un gradient de dichlorométhane 100% jusqu'à dichlorométhane-méthanol (90-10).

On obtient 390 mg de produit attendu.
**CCM :** Rf=0,27 (silicagel, éluant : dichlorométhane-méthanol-eau-acide acétique 90-10-1-1).
**1H-RMN ($CDCl_3$) :** δ 1,37 (s, 9H, tBu) ; 1,75 (m, 2H, CH2-CH2-CH2-NH); 1,90 à 2,05 (m,5H, CH3, N-CH2-CH2-CH-CH2-CH2) ; 2,75 (t, 2H, CH2-CH2-CH2-NH);3,02 (m, 1H, N-CH2-CH2-CH-CH2-CH2) ; 3,15 et 3,77 (2m, 4H, CH2-CH2-N-CH2-CH2) ; 3,5 (m, 2H, CH2-CH2-CH2-NH) ; 4,17 (m, 3H, NH-CH2-CH-NH, NH-CH2-CH-NH) ; 5,08 (s, 2H, O-CH2-Ph) ; 6,4 et 7,35 (2d, 2H, naphthyridine); 6,12 (m, 1H, NH); 7,2 à 7,35 (m, 5H, phényl) ; 10,05 (m,1H, OH acide).
**SM :** 602 (MH+); 600 (MH-)

Exemple comparatif 16 Synthèse de la 2-méthyl-5-cyclopropyl-4,6-dihydroxy-pyrimidine :

Un monocol contenant 80 ml d'éthanol est refroidit à 0˚C par un bain de glace, on additionne 4,5 g (66,4 mmoles) d'éthylate de sodium et 4 g (33,2 mmoles)de chlorhydrate de cyclopropylcarbamidine. On laisse le mélange réactionnel revenir à température ambiante, puis on ajoute goutte à goutte 5,7 ml (33,2 mmoles) de méthylmalonate de diéthyle ester. On maintient l'agitation à température ambiante pendant une nuit. L'éthanol est ensuite condensé sous pression réduite (2 kPa). Le brut obtenu est solubilisé dans un minimum d'eau (environ 40 ml), puis acidifié à 0˚C avec de l'acide acétique pur jusqu'à un pH compris entre 4 et 5. Le précipité blanc formé est filtré, rincé sucessivement à l'eau, à l'éther éthylique et au pentane. Puis la poudre blanche obtenue est séché sur $P_2O_5$ sous pression réduite (0,2 kPa). On obtient 3 g de produit attendu.
**CCM :** Rf=0,36 (Silicagel, éluant : dichlorométhane-méthanol 90-10).
**SM :** (MH+)=167, (MH-)=165
**H-RMN (DMSOd6) :** δ 0,95 (m,4H,cyclopropyl) ; 1, 67 (s, 3H, N=C-$CH_3$) ; 1,83 (m, 1H, cyclopropyl).

Synthèse de la 2-cyclopropyl-4,6-dichloro-5-méthyl-pyrimidine :

Un mélange de 3,68 g (22 mmoles) de 2-cyclopropyl-5-méthyl-4,6-dihydroxy-pyrimidine et 20 ml d'oxychlorure de phosphore, est porté au reflux pendant 1 heure et 30 minutes. Après retour à température ambiante, le mélange réactionnel est versé lentement sur un mélange de glace, d'eau et de carbonate de sodium solide. Cette phase aqueuse est extraite à l'acétate d'éthyle. La phase organique est lavée plusieurs fois avec une solution saturée de bicarbonate de sodium jusqu'à neutralisation totale de l'acide puis séchée sur du sulfate de magnésium et évaporée à sec sous pression réduite (2 kPa). On obtient 4,26 g (Rdt=95%) de produit attendu.
**CCM :** Rf=0,56 (Silicagel, éluant : cyclohexane-acétate d'éthyle 98-2)
**1H-RMN ($CDCl_3$) :** δ 1,07 à 1,2 (m, 4H, cyclopropyl) ; 2,18 (m, 1H, cyclopropyl); 2,42 (s,3H, N=C-$CH_3$) ;
**SM :** (MH+)=204.

Synthèse de la 2-[1-(2-cyclopropyl-6-chloro-5-méthyl-pyrimidin-4-yl)-pipéridin-4-yl]-[1,8]naphthyridine :

Dans un monocol contenant 1,5 g (7,39 mmoles)de 2-cyclopropyl-4,6-dichloro-5-méthyl-pyrimidine et 1,58 g (7,39 mmoles) de 2-Pipéridin-4-yl-[1,8]naphthyridine, on ajoute 20 ml de diméthylacétamide et 3,36 ml (19,2 mmoles)de diisopropyléthylamine. Ce mélange est chauffé à 100˚C pendant une nuit. Le lendemain on ajoute 0,2 équivalent de naphthyridine et on chauffe 6 heures de plus. Puis le mélange réactionnel est ramené à température ambiante avant concentration à sec. Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur sulfate de magnésium puis le solvant évaporé sous pression réduite (2 kPa). Le résidu est chromatographié sur silicagel en éluant avec un gradient d'heptane-acétate d'éthyle (50-50) jusqu'à acétate d'éthyle 100%. On obtient 1,45 g de produit attendu.
**CCM :** Rf=0,28 [silicagel, éluant heptane-acétate d'éthyle(50-50).
**1H-RMN ($CDCl_3$) :** δ 0,98 et 1,09 (2m,4H, cyclopropyl) ; 2,1 (m, 1H, cyclopropyl); 2,15 (m, 4H, N-CH2-CH2-CH-CH2-CH2) ; 2,25 (s, 3H, CH3) ; 3,05 et 4,0 (2m, 4H, CH2-CH2-N-CH2-CH2) ; 3,23 (m, 1H, N-CH2-CH2-CH-CH2-CH2) ; [(7,05; 8,2; 9,13), 3m, 5H,naphthyridine].
**SM :** 380(MH+).

Synthèse du 2-benzyloxycarbonylamino-3- [2-cyclopropyl-5-méthyl-6-(4-[1,8]naphthyridin-2-yl-pipéridin-1-yl)-pyrimidin-4-ylamino]-propionate de tert-butyle

Un mélange de 1,4 g (3,68 mmoles) de 2-[1-(2-tert-Butyl-6-chloro-5-méthyl-pyrimidin-4-yl)-pipéridin-4-yl]-[1,8] naphthyridine et de 1,3 g (4,42 mmoles) de 3-amino-2-benzyloxycarbonylamino propionate de tert-butyle), en présence de 1,68 g (11 mmoles) de fluorure de cesium, de 230 mg (0,37 mmoles) et de (2,2'-bis(diphénylphosphino)-1,1'-binaphthyl dans 20 ml de dioxane est porté au reflux. On ajoute à chaud 200 mg (0,19 mmoles) de tris-dibenzylidèneacétone) dipalladium(o) mis en solution dans 5 ml de dioxane, puis on laisse au reflux pendant 15 heures. Le mélange réactionnel est ensuite ramené à température ambiante, et concentré à sec sous pression réduite (2 kPa) puis repris par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous pression réduite (2 kPa). Le résidu est chromatographié sur Alumine puis sur silice avec un gradient dichlorométhane-d'acétate d'éthyle (95-5) jusqu'à (70-30). On obtient 1,5 g de produit attendu.
**CCM :** Rf=0,17 (silicagel, éluant : dichlorométhane-acétate d'éthyle (70-30)
**1H-RMN ($CDCl_3$) :** δ 0,82 et 1,05 (2m, 4H, cyclopropyl); 1,45 (s, 9H, tBu); 1,93 (s, 3H, CH3); 1,97 (m, 1H, cyclopropyl); 2,07 à 2,25 (m,4H, N-CH2-CH2-CH-CH2-CH2) ; 3,0 et 3,73 (2m, 4H, CH2-CH2-N-CH2-CH2) ; 3,15 (m, 1H, N-CH2-CH2-CH-CH2-CH2) ; 3,73 à 4,0 (2m, 2H, NH-CH2-CH-NH) ; 4,36 (m, 1H, NH-CH2-CH-NH) ; 4,65 et 8,87 (2m, 2H, NH); 5,12 (s, 2H, O-CH2-Ph) ; 7,33 (m, 5H, phényl) ; [(7,47; 8,15; 9,1), (3m, 5H, naphthyridine).
**SM :** 638(MH+), 448 (MH-CBZ-tBu)

Synthèse du 2-benzyloxycarbonylamino-3- [2-cyclopropyl-5-méthyl-6-[4-(5,6,7,8-tétrahydro-[1,8]naphthyridin-2-yl-pipéridin-1-yl)-pyrimidin-4-ylamino]-propioniate de tert-butyle

A 1,4 g (2,19 mmoles)de 2-benzyloxycarbonylamino-3- [2-cyclopropyl-5-méthyl-6-(4-[1,8]naphthyridin-2-yl-pipéridin-1-yl)-pyrimidin-4-ylamino]-propionate de tert-butyle mis en solution dans 60 ml d'éthanol, on ajoute 100 mg d'oxyde de platine (IV). Le mélange réactionnel est alors purgé sous vide et surmonté d'un ballon de baudruche contenant de l'hydrogène. On laisse sous agitation et à température ambiante pendant 6 heures. Le mélange réactionnel est ensuite filtré sur clarcel et concentré à sec sous pression réduite (2 kPa). Le résidu est chromatographié sur silice avec un gradient dichlorométhane-méthanol (98-2). On obtient 1,23 g de produit attendu.
**CCM :** Rf=0,10 [silicagel, éluant : dichlorométhane-méthanol 98-2]
**1H-RMN ($CDCl_3$) :** δ 0, 82 et 1, 05 (2m, 4H, cyclopropyl) ; 1, 45 (s, 9H, tBu); 1,68 (m, 1H, cyclopropyl) ; 1,75 à 2,0 (m, 9H, CH3, CH2-CH2-CH2-NH, N-CH2-CH2-CH-CH2-CH2) ; 2,6 (m, 1H, N-CH2-CH2-CH-CH2-CH2) ; 2,72 (t, 2H, CH2-CH2-CH2-NH); 2,9 et 3,65 (2m, 4H, CH2-CH2-N-CH2-CH2) ; 3,43 (m, 2H, CH2-CH2-CH2-NH) ; 3,75 à 4,0 (2m, 2H, NH-CH2-CH-NH) ; 4,36 (m, 1H, NH-CH2-CH-NH) ; [(4,6; 4,75; 6,87), 3m, 3H, NH); 5,1 (s, 2H, O-CH2-Ph) ; 6,42 et 7,12 (2d, 2H, naphthyridine); 7,33 (m, 5H, phényl).
**SM :** 642 (MH+).

Exemple comparatif 17

Acide 2-benzyloxycarbonylamino-3- [2-cyclopropyl-5-méthyl-6-[4-(5,6,7,8-tétrahydro-[1,8]naphthyridin-2-yl-pipéridin-1-yl)-pyrimidin-4-ylamino]-propionique (bistrifluoroacetate)

On agite à température ambiante un mélange de 300 mg (0,47 mmoles) de 2-benzyloxycarbonylamino-3- [2-cyclopropyl-5-méthyl-6-[4-(5,6,7,8-tétrahydro-[1,8]naphthyridin-2-yl-pipéridin-1-yl)-pyrimidin-4-ylamino]-propioniate de tert-butyle mis en solution dans 5 ml de dichlorométhane et de 2 ml d'acide trifluoroacétique pendant 24 heures. On

additionne alors du toluène et évapore à sec le mélange. Le résidu est solubilisé dans un minimum de dichlorométhane puis versé sur un mélange de pentane et d'éther diisopropylique. Le précipité est filtré. On obtient une poudre blanche impure que l'on doit purifier sur silice avec un gradient de dichlorométhane 100% jusqu'à dichlorométhane-méthanol (90-10).

On obtient 320 mg de produit attendu.
**CCM :** Rf=0,16 (silicagel, éluant : dichlorométhane-méthanol-eau-acide acétique 90-10-1-1).
**1H-RMN ($CDCl_3$) :** δ 1,05 (m, 4H, cyclopropyl) ; 1,95 (m, 9H, CH3, CH2-CH2-CH2-NH, N-CH2-CH2-CH-CH2-CH2) ; 2,3 (m, 1H, cyclopropyl); 2,77 (t, 2H, CH2-CH2-CH2-NH); 2,85 à 3,2 et 3,95 (3m, 7H, CH2-CH2-N-CH2-CH2, N-CH2-CH2-CH-CH2-CH2, NH-CH2-CH-NH) ; 3,5 (m, 2H, CH2-CH2-CH2-NH) ; 4,25 (m, 1H, NH-CH2-CH-NH) ; 5,1 (s, 2H, O-CH2-Ph); 6,07 (m, 1H, NH); 6,35 (d, 1H, naphthyridine); 7,2 à 7,4 (m, 6H, naphthyridine et phényl) ; 10,3 (m, 1H, OH acide).
**SM :** 586 (MH+); 584 [MH-).

Exemple comparatif 18

Synthèse de la 2-méthoxy-4,6-dihydroxy-5-méthyl-pyrimidine.

A une solution de 10 g (58 mmoles) de sulfate de méthoxyformamidine dans 100 ml d'éthanol refroidie à 0°C on ajoute 13,8 g (200 mmoles) d'éthylate de sodium et agite le mélange pendant 15 minutes; on additionne alors une solution de 9 ml (52 mmoles) de méthyl malonate de diéthyle dans 50 ml d'éthanol et agite une nuit à température ambiante. On évapore le mélange réactionnel à sec sous pression réduite (2 kPa). Le résidu est repris par 100 ml d'une solution saturée en chlorure de sodium et extrait par 800 et 200 ml de n-butanol. les phases organiques sont séchées sur sulfate de sodium, filtrées et évaporées à sec sous pression réduite (2 kPa). On obtient 5,9 g de produit attendu sous forme d'une solide blanc.
**1H-RMN ($CD_3OD$) :** δ 1, 85 ppm (s, 3H, C-$CH_3$) ; 3,95(s, 3H, C-O$CH_3$).
**SM :** 157 (MH+); 155 (M-H-).

Synthèse de la 4,6-dichloro-2-méthoxy 5-méthyl-pyrimidine.

Un mélange de 0,9 g (5,77 mmoles) de 2-méthoxy-4,6-dihydroxy-5-méthyl-pyrimidine dans 30 ml d'oxychlorure de phosphore est porté au reflux durant 2 heures. Après retour à température ambiante, le mélange réactionnel est versé sur un mélange de glace et d'eau puis on ajoute doucement du bicarbonate de sodium jusqu'à pH basique. On extrait à l'acétate d'éthyle, sèche sur sulfate de magnésium et évapore à sec sous pression réduite (2 kPa). On obtient 0,43 g (Rdt=38%) d'une huile marron.
**CCM :** Rf=0,5 (silicagel, éluant : heptane-acétate d'éthyle 80-20).
**1H-RMN ($CDCl_3$) :** δ 2,68 ppm(s, 3H, C-$CH_3$) ; 3,94(s, 3H, C-O$CH_3$).
**SM :** 193,195 (MH+).

Synthèse du 2-[1-(6-Chloro-2-méthoxy-5-méthyl-pyrimidin-4-yl)-pipéridin-4-yl]-[1,8]naphthyridine :

Dans un monocol contenant 0,48 g (2,2 mmoles) de 4-(1,8-naphthyridin-7-yl)-1-pipéridine on ajoute 30 ml de diméthylacétamide, 0,4 g de 4,6-dichloro-2-méthoxy5-méthyl-pyrimidine (2 mmoles) et 3 ml de diisopropyléthylamine. Ce mélange est chauffé à 120°C pendant 4 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant est évaporé sous pression réduite (2 kPa). Le résidu est chromatographié sur silicagel en éluant avec un gradient d'heptane-acétate d'éthyle de 100-0 à 0-100. On obtient 460 mg de produit attendu sous la forme d'un solide jaune.
**CCM :** Rf=0,28 [silicagel, éluant : acétate d'éthyle (100%)]
**1H-RMN ($CDCl_3$) :** □ 2,17ppm (m, 4H, N-CH2-CH2-CH-CH2) ; 2,25 (s, 3H,C-$CH_3$) ; 3,11 et 4,06 (dt etdl, 4H, CH2-CH2-N-CH2-CH2) ; 3,23 (m, 1H, CH2-CH-CH2,) ; 3,96 (s, 3H, C-OCH3) ; 7,47 et 8,17 (2d, 2H, C-CH=CH-C(CH) =N) ; 7,48 (dd,1H, N-CH=CH-CH=C); 8,19 (dd,1H, N-CH=CH-CH=C) ; 9,12 (dd,1H, N-CH=CH-CH=C).
**SM :** 370,372 (MH+).

Synthèse 2-benzyloxycarbonylamino-3-[2-méthoxy-5-méthyl-6-(4-[1,8]naphthyridin-2-yl-pipéridin-1-yl)-pyrimidin-4-ylamino]-propionate de tert-butyle

On chauffe au reflux un mélange de 460 mg (1,25 mmoles) 2-[1-(6-chloro-2-méthoxy-5-méthyl-pyrimidin-4-yl)-pipéridin-4-yl]-[1,8]naphthyridine, de 405 mg (1,37 mmoles) de 3-amino-2-benzyloxycarbonylamino propionate de tert-butyle, de 250 mg (1,65 mmoles) de fluorure de césium, de 57 mg (0,062 mmole) de tris(dibenzylidèneacétone) dipalladium(0), et de 40 mg (0,062 mmole) de 2,2'-bis(diphényl-phosphino)-1,1'-binaphtyl dans 50 ml de dioxane pendant 2 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient d'heptane - acétate d'éthyle de 100-0 à 0-100. On obtient 300 mg de produit attendu sous forme de solide amorphe jaune.

**CCM :** Rf=0,27 (silicagel, éluant : acétate d'éthyle).

**1H-RMN ($CDCl_3$) :** □ 1,48ppm (s, 1H, tBu) ; 1,91 (s, 3H, C-$CH_3$) ; 2,10 à 2,26 (m, 4H, N-CH2-CH2-CH-CH2); 3,01 et 3,79 (tl et dl, 4H, CH2-CH2-N-CH2-CH2); 3,17 (m, 1H, CH2-CH-CH2,); 3,85 et 3,97 (2m, 2H, NH-$CH_2$-CH-NH); 3,91 (s, 3H, C-O$CH_3$) ; 4,45 (m, 1H, NH-$CH_2$-CH-NH); 5,12 (d, 2H, $CH_2$-Ph);; 7,35 (m, 5H, CH2Ph); 7,48 et 8,16 (2d, 2H, C-CH=CH-C(CH)=N); 7,48 (dd,1H, N-CH=CH-CH=C); 8,19 (dd,1H, N-CH=CH-CH=C); 9,11 (dd,1H, N-CH=CH-CH=C); 4,89 et 6,21 (tl et dl H mobiles).

**SM :** 628 (MH+); 438 (MH- COOCH2Ph+).

Synthèse du 2-benzyloxycarbonylamino-3-{2-méthoxy-5-méthyl-6-[4-(5,6,7,8-tétrahydro-[1,8]naphthyridin-2-yl)-pipéridin-1-yl]-pyrimidin-4-ylamino}-propionate de tert-butyle

On agite un mélange de 300 mg (0,477 mmole) de -2-benzyloxycarbonylamino-3-[2-méthoxy-5-méthyl-6-(4-[1,8]naphthyridin-2-yl-pipéridin-1-yl)-pyrimidin-4-ylamino]-propionate de tert-butyle et de 30 mg d'oxyde de platine dans 30 ml d'éthanol pendant 1 heures sous pression atmosphérique d'hydrogène. Le mélange réactionnel est filtré sur clarcel puis évaporé à sec sous pression réduite (2 kPa). On obtient 170 mg de produit amine sous forme d'une huile orange.

Cette amine est mise en reaction avec 120 mg de benzyloxycarbonyle-succinimide dans 40 ml de diméthoxyéthane pendant 3 heures à température ambiante. Le mélange réactionnel est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane - acétate d'éthyle de 100-0 à 0-100. On obtient 200 mg (Rdt=93%; 67% pour les deux étapes) de produit attendu sous forme d'une huile jaune.

**CCM :** Rf=0,18 (silicagel, éluant : acétate d'éthyle).

**1H-RMN ($CDCl_3$) :** □ 1,46 ppm (s, 1H, tBu) ; 1,77 et 1,97 (m, 6H, $CH_2$-$CH_2$-$CH_2$-NH et $CH_2$-CH-$CH_2$); 1,90 (s, 3H, C-$CH_3$) ; 2,61 (tt, 1H, $CH_2$-CH-$CH_2$); 2,71 (tl, 2H, $CH_2$-$CH_2$-$CH_2$-NH); 2,91 et 3,72 (tl et dl, 4H, CH2-CH2-N-CH2-CH2); 3,41 (m, 2H, $CH_2$-$CH_2$-$CH_2$-NH); 3,84 et 3,95 (2m, 2H, NH-$CH_2$-CH-NH); 3,89 (s, 3H, C-O$CH_3$) ; 4,43 (m, 1H, NH-$CH_2$-CH-NH); 5,11 (d, 2H, $CH_2$-Ph); 6,41 et 7,12 (2d, 2H, C=CH=CH=C) ; 7,36 (m, 5H, CH2Ph) ; 4, 77; 4,85 et 6,21 (m; tl et dl H mobiles).

**SM :** 632 (MH+); 442 (MH- COOCH2Ph+).

Synthèse de l'acide 2-benzyloxycarbonylamino-3-{2-méthoxy-5-méthyl-6-[4-(5,6,7,8-tétrahydro-[1,8]naphthyridin-2-yl)-pipéridin-1-yl]-pyrimidin-4-ylamino}-propionique bis(trifluoroacetate)

On agite 200 mg (0,317 mmole) de 2-benzyloxycarbonylamino-3-{2-méthoxy-5-méthyl-6-[4-(5,6,7,8-tétrahydro-[1,8]naphthyridin-2-yl)-pipéridin-1-yl]-pyrimidin-4-ylamino}-propionate de tert-butyle dans 10 ml de dichlorométhane avec 2 ml d'acide trifluoroacétique à température ambiante jusqu'à disparition du produit de départ en CCM (silicagel, éluant : $CH_2Cl_2$-MeOH-$H_2O$-AcOH 90-10-1-1). On rajoute du toluène et évapore le mélange réactionnel à sec sous pression réduite (2 kPa). Le résidu est solubilisé dans le minimum de dichlorométhane puis versé sur de l'éther diisopropylique. On obtient 170 mg de produit attendu sous forme d'un solide beige.

**CCM :** Rf=0,30 (silicagel, éluant : chlorure de méthylène-méthanol-eau-acide acétique 90-10-1-1).

**1H-RMN ($CDCl_3$) :** □ 1,72 et 2,05 ppm (m, 6H, $CH_2$-$CH_2$-$CH_2$-NH et $CH_2$-CH-$CH_2$) ; 1,93 (s, 3H, C-$CH_3$) ; 2,77 (tl, 2H, $CH_2$-$CH_2$-$CH_2$-NH) ; 2,96 (tl, 1H, $CH_2$-CH-$CH_2$); 3,20 et 3,83 (tl et dl, 4H, CH2-CH2-N-CH2-CH2) ; 3,51 (m, 2H, $CH_2$-$CH_2$-$CH_2$-NH) ; 3,96 (s, 3H, C-O$CH_3$) ; 4,01 (m masqsué, 2H, NH-$CH_2$-CH-NH) ; 4,44 (m, 1H, NH-$CH_2$-CH-NH); 5,09 (d, 2H, $CH_2$-Ph); 6,41 et 7,36 (2d, 2H, C=CH=CH=C); 7,31 (m, 5H, CH2Ph); 6,49; 8,17 et 9,60 (dl; ml et sl H mobiles).

**SM :** 576(MH+); 442(MH-COOCH2Ph+); 574(MH-); 466(574-OCH2Ph-); 1150(2M-).

Exemple comparatif 19

Synthèse de la 2,5-diméthoxy-4,6-dihydroxy-pyrimidine.

A une solution de 9 g (52 mmoles) de sulfate de méthoxyformamidine dans 100 ml d'éthanol refroidie à 0°C on ajoute 9,7 g (180 mmoles) de méthylate de sodium et agite le mélange pendant 15 minutes; on additionne alors une solution de 6,5 ml (46,8 mmoles) de méthoxy malonate de méthyle dans 50 ml d'éthanol et agite une nuit à température ambiante. On évapore le mélange réactionnel à sec sous pression réduite (2 kPa). Le résidu est repris par 100 ml d'une solution saturée en chlorure de sodium et extrait par 800 et 200 ml de n-butanol. les phases organiques sont séchées sur sulfate de sodium, filtrées et évaporées à sec sous pression réduite (2 kPa). On obtient 7 g de produit attendu sous forme d'une solide blanc.
**1H-RMN ($CD_3OD$)** : δ 3,59 ppm (s, 3H, C-O<u>$CH_3$</u>) ; 3,91 (s, 3H, C-O<u>$CH_3$</u>), 4,90 (m H mobiles).
**SM :** 173 (MH+); 171 (M-H-).

Synthèse de la 4,6-dichloro-2,5-diméthoxy-pyrimidine.

Un mélange de 1,7 g (10 mmoles) de 2,5-diméthoxy-4,6-dihydroxy -pyrimidine dans 30 ml d'oxychlorure de phosphore est porté au reflux durant 5 heures. Après retour à température ambiante, le mélange réactionnel est versé sur un mélange de glace et d'eau puis on ajoute doucement du bicarbonate de sodium jusqu'à pH basique. On extrait au butanol, sèche sur sulfate de sodium et évapore à sec sous pression réduite (2 kPa). On obtient 0,50 g d'une huile marron.
**CCM :** Rf=0,5 (silicagel, éluant : heptane-acétate d'éthyle 80-20).
**1H-RMN ($CDCl_3$) :** 3,90 ppm(s, 3H, C-$OCH_3$) ; 4,01 (s, 3H, C-$OCH_3$).

Synthèse du 2-[1-(6-Chloro-2,5-diméthoxy-pyrimidin-4-yl)-pipéridin-4-yl]-[1,8]naphthyridine :

Dans un monocol contenant 1,3 g (7,8 mmoles) de 4-(1,8-naphthyridin-7-yl)-1-pipéridine on ajoute 40 ml de diméthylacétamide, 1,25 g de 4,6-dichloro-2,5-diméthoxy-pyrimidine (5,6 mmoles) et 5 ml de diisopropyléthylamine. Ce mélange est chauffé à 120°C pendant 5 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant est évaporé sous pression réduite (2 kPa). Le résidu est chromatographié sur silicagel en éluant avec un gradient d'heptane-acétate d'éthyle de 100-0 à 0-100. On obtient 1,2 g de produit attendu sous la forme d'un solide jaune.
**CCM :** Rf=0,12 [silicagel, éluant : acétate d'éthyle (100%)]
**1H-RMN ($CDCl_3$) :** δ 2, 10 à 2,20ppm (m, 4H, N-CH2-<u>CH2</u>-CH-<u>CH2</u>) ; 3,13 et 4,87 (dt et dl, 4H, CH2-<u>CH2</u>-N-<u>CH2</u>-CH2); 3,30 (m, 1H, CH2-<u>CH</u>-CH2,); 3,72 et 3,91 (2s, 6H, C-<u>$OCH_3$</u>); 7,47 et 8,19 (2d, 2H, C-<u>CH</u>=<u>CH</u>-C(CH)=N); 7,50 (dd,1H, N-CH=<u>CH</u>-CH=C); 8,22 (dd,1H, N-CH=CH-<u>CH</u>=C); 9,12 (dd,1H, N-<u>CH</u>=CH-CH=C).
**SM :** 386,388 (MH+).

Synthèse du 2-benzyloxycarbonylamino-3-[2,5-diméthoxy-6-(4-[1,8]naphthyridin-2-yl-pipéridin-1-yl)-pyrimidin-4-ylamino]-propionate de tert-butyle

On chauffe au reflux un mélange de 1,2 g (3,1 mmoles) 2-[1-(6-Chloro-2,5-diméthoxy-pyrimidin-4-yl)-pipéridin-4-yl]-[1,8]naphthyridine, de 1,3 g (4,40 mmoles) de 3-amino-2-benzyloxycarbonylamino propionate de tert-butyle, de 750 mg (4,95 moles) de fluorure de césium, de 200 mg (0,217 mmole) de tris(dibenzylidèneacétone)dipalladium(O), et de 120 mg (0,186 mmole) de 2,2'-bis(diphényl-phosphino)-1,1'-binaphtyl dans 150 ml de dioxane pendant 6 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié une première fois sur alumine en éluant avec acétate d'éthyle-éther éthylique 50-50 puis une deuxième fois sur silice avec un gradient d'heptane-acétate d'éthyle de 100-0 à 0-100. On obtient 900 mg de produit attendu sous forme de solide amorphe jaune.
**CCM :** Rf=0,35 (silicagel, éluant : acétate d'éthyle).
**1H-RMN ($CDCl_3$) :** δ 1,48ppm (s, 1H, tBu); 2,12 (m, 4H, N-CH2-<u>CH2</u>-CH-<u>CH2);</u> 3,01 et 4,60 (tl et dl, 4H, CH2-<u>CH2</u>-N-<u>CH2</u>-CH2); 3,21 (m, 1H, CH2-<u>CH</u>-CH2,); 3,57 et 3,88 (2s, 6H, C-O<u>$CH_3$</u>) ; 3,85 (m, 2H, NH-<u>$CH_2$</u>-CH-NH); 4,41 (m, 1H, NH-$CH_2$-<u>CH</u>-NH); 5,12 (s, 2H, <u>$CH_2$</u>-Ph) ; ; 7,35 (m, 5H, CH2<u>Ph</u>); 7,48 et 8,16 (2d, 2H, C-<u>CH</u>=<u>CH</u>-C(CH) =N); 7,48 (dd,1H, N-CH=<u>CH</u>-CH=C); 8,19 (dd,1H, N-CH=CH-<u>CH</u>=C); 9,11 (dd,1H, N-<u>CH</u>=CH-CH=C); 5,37 et 6,29 (tl et dl H mobiles).

**SM :** 644 (MH+); 454 (MH- COOCH2Ph+).

Synthèse du 2-benzyloxycarbonylamino-3-{2,5-diméthoxy-6-[4-(5,6,7,8-tétrahydro-[1,8]naphthyridin-2-yl)-pipéridin-1-yl]-pyrimidin-4-ylamino}-propionate de tert-butyle

On agite un mélange de 85 mg (1,32mmoles) de 2-benzyloxycarbonylamino-3-[2,5-diméthoxy-6-(4-[1,8] naphthyridin-2-yl-pipéridin-1-yl)-pyrimidin-4-ylamino]-propionate de tert-butyle et de 30 mg d'oxyde de platine dans 50 ml d'éthanol pendant 2 heures sous pression atmosphérique d'hydrogène. Le mélange réactionnel est filtré sur clarcel puis évaporé à sec sous pression réduite (2 kPa). Le résidu est chromatographié sur silice avec un gradient d'heptane-acétate d'éthyle-méthanol de 100-0-0 à 0-100-0 puis 0-95-5. On obtient 300 mg de produit attendu sous forme d'un solide blanc.
**CCM :** Rf=0,16 (silicagel, éluant : acétate d'éthyle).
**1H-RMN ($CDCl_3$) :** δ 1,46 ppm (s, 1H, tBu) ; 1,79 et 1,97 (dt et m, 6H, $CH_2$-$\underline{CH_2}$-$CH_2$-NH et $\underline{CH_2}$-CH-$\underline{CH_2}$) ; 2,72 (tl, 3H, $CH_2$-$\underline{CH}$-CH2 et $\underline{CH_2}$-$CH_2$-$CH_2$-NH) ; 2, 92 et 4, 57 (tl et dl, 4H, $\overline{CH}$2-$\underline{CH2}$-N-$\underline{CH2}$-$\overline{C}$H2) ; 3, 43 (m, 2H, $CH_2$-$CH_2$-$\underline{CH_2}$-NH) ; 3, 56 et 3, 87 (2s, 6H, C-O$\underline{CH_3}$) ; 3, 85 (m masqué, 2H, NH-$\underline{CH_2}$-CH-NH); 4,41 (m, 1H, NH-$CH_2$-$\underline{CH}$-NH); 5,12 (s, 2H, $\underline{CH_2}$-Ph); 6,37 et 7,16 (2d, 2H, C=$\underline{CH}$=$\underline{CH}$=C); 7,35 (m, 5H, CH2$\underline{Ph}$); 5,33 et 6,28 (tl et dl H mobiles).
**SM :** 648 (MH+); 458 (MH- COOCH2Ph+).

Exemple comparatif 20

Synthèse de l'acide 2-benzyloxycarbonylamino-3-{2,5-diméthoxy-6-[4-(5,6,7,8-tétrahydro-[1,8]naphthyridin-2-yl)-pipéridin-1-yl]-pyrimidin-4-ylamino}-propionique, bis(trifluoroacetate).

On agite 350 mg (0,54 mmole) de 2-benzyloxycarbonylamino-3-{2,5-diméthoxy-6-[4-(5,6,7,8-tétrahydro-[1,8] naphthyridin-2-yl)-pipéridin-1-yl]-pyrimidin-4-ylamino}-propionate de tert-butyle dans 10 ml de dichlorométhane avec 2 ml d'acide trifluoroacétique à température ambiante jusqu'à disparition du produit de départ en CCM (silicagel, éluant : $CH_2Cl_2$-MeOH-$H_2O$-AcOH 90-10-1-1. On rajoute du toluène et évapore le mélange réactionnel à sec sous pression réduite (2 kPa). Le résidu est purifié par chromatographie sur silice en éluant avec un gradiant d'élution chlorure de méthylène-méthanol de 100-0 à 90-10. On obtient 360 mg de produit attendu sous forme d'un solide blanc.
**CCM :** Rf=0,50 (silicagel, éluant : chlorure de méthylène-méthanol-eau-acide acétique 90-10-1-1).
**1H-RMN ($CD_3OD$) :** δ 1, 79 et 1, 96ppm (q et m, 6H, $CH_2$-$\underline{CH_2}$-$CH_2$-NH et $\underline{CH_2}$-CH-$\underline{CH_2}$) ; 2, 82 (tl, 2H, $\underline{CH_2}$-$CH_2$-$CH_2$-NH) ; 2, 91 (tl, 1H, $CH_2$-$\underline{CH}$-$CH_2$) ; 3,50 et 4,58 (tl et dl, 4H, $CH_2$-$\underline{CH_2}$-N-$\underline{CH_2}$-$CH_2$) ; 3,50 (tl, 2H, $CH_2$-$CH_2$-$\underline{CH_2}$-NH); 3,67 et 3, 87 masqué, 2H NH-$\underline{CH_2}$-CH-NH); 3, 52 et 3,85 (2s, 6H, C-O$\underline{CH_3}$) ; 4,39 (m, 1H, NH-$\underline{CH_2}$-CH-NH); 5,07 (ql, 2H, $\underline{CH_2}$-Ph); 6,62 et 7,58 (2d, 2H, C=$\underline{CH}$=$\underline{CH}$=C); 7,31 (m, 5H, $CH_2\underline{Ph}$).
**SM :** 592(MH+); 458 (MH-COO$CH_2$Ph+); 590(MH-); 482 (590-O$CH_2$Ph-); 1182 (2M-).

Exemple comparatif 21

Synthèse du 4-chloro-6-[4-(5,6,7,8-tétrahydro-[1,8]naphthyridin-2-yl)-pipéridin-1-yl]-pyrimidine-5-carbaldéhyde :

Dans un monocol contenant 3,9 g (17 mmoles) de 7-Pipéridin-4-yl-1,2,3,4-tétrahydro-[1,8]naphthyridine, on ajoute 80 ml diméthylacétamide, 3,9 g (22 mmoles) de 4,6-dichloro-5-formylpyrimidine [préparé selon Liebigs Annalen der Chemie (1972) 766 73-88] et 8 ml de diisopropyléthylamine. Ce mélange est chauffé à 120°C pendant 3 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé sous pression réduite (2 kPa). Le brut obtenu est chromatographié sur silicagel en éluant avec un gradient de chlorure de méthylène et d'acétate d'éthyle (100-0 à 0-100).
On obtient 2,3 g de produit attendu sous la forme d'une poudre jaune.
**CCM :** Rf=0,25 [silicagel, éluant : acétate d'éthyle (100%)]
**1H-RMN ($CDCl_3$) :** δ 1,79 à 2,06 (m, 6H, NH-CH2-$\underline{CH2}$-CH2, N-CH2-$\underline{CH2}$-CH-$\underline{CH2}$); 2,72 (t, 2H, NH-CH2-CH2-$\underline{CH2}$); 2,81 (tl, 1H, CH2-$\underline{CH}$-CH2), 3,26 et 4,21 (tl et dl, 4H, CH2-$\underline{CH2}$-N-$\underline{CH2}$-CH2); 3,43 (sl, 2H, NH-$\underline{CH2}$-CH2-CH2); 6,38 et 7,12 (2d, 2H, $\underline{CH}$=$\underline{CH}$ naphthyridine); 8,35 (s, 1H, N=$\underline{CH}$-N); 10,34 (s, 1H, C-CH=O).
**SM :** 358-360(MH+)

Synthèse du 2-benzyloxycarbonylamino-3-{5-formyl-6-[4-(5,6,7,8-tétrahydro-[1,8]naphthyridin-2-yl)-pipéridin-1-yl]-pyrimidin-4-ylamino}-propionate de tert-butyl.

On chauffe au reflux un mélange de 180 mg (0,5 mmole) de 4-Chloro-6-[4-(5,6,7,8-tétrahydro-[1,8]naphthyridin-2-yl)-pipéridin-1-yl]-pyrimidine-5-carbaldehyde, de 150mg (0,5 mmole) de 3-amino-2-benzyloxycarbonylamino propionate de tert-butyle, de 117 mg (0,77 mmole) de fluorure de césium, de 23 mg (0,025 mmole) de tris(dibenzylidèneacétone) dipalladium(O), et de 32 mg (0,51 mmole) de 2,2'-bis(diphényl-phosphino)-1,1'-binaphtyl dans 30 ml de diméthoxyéthane pendant 2 heures. On refroidit puis ajoute à nouveau 23 mg (0,025 mmole) de tris(dibenzylidèneacétone)dipalladium (O) et chauffe au reflux pendant 2 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un mélange d'acétate d'éthyle-chlorure de méthylène-méthanol 50-45-5. On obtient 120 mg de produit attendu sous forme de solide amorphe blanc.
**CCM :** Rf=0,25 (silicagel, éluant : acétate d'éthyle).
**1H-RMN ($CDCl_3$) :** δ 1,50 (s, 9H, tBu) ; 1,80 à 2,12 (m, 6H, $CH_2$-$\underline{CH_2}$-$CH_2$-NH et $\underline{CH_2}$-CH-$\underline{CH_2}$); 2,72 (t,2H, $\underline{CH_2}$-$CH_2$-$CH_2$-NH); 2,87 (tl, 1H, $CH_2$-$\underline{CH}$-$CH_2$); 3,30 et 4,29 (ql et tl, 4H, $CH_2$-$\underline{CH_2}$-N-$\underline{CH_2}$-$CH_2$); 3,46 (m, 2H, $CH_2$-$CH_2$-$\underline{CH_2}$-NH); 3,92 et 4,03 (2m, 2H, NH-$\underline{CH_2}$-CH-NH) ; 4,47 (m, 1H, NH-$CH_2$-$\underline{CH}$-NH) ; 5,13 (s, 2H, $\underline{CH_2}$-Ph); 6,37 et 7,19 (2d, 2H, $\underline{CH}$=$\underline{CH}$ naphthyridine); 7,36 (m, 5H, Ph)); 8,35 (s, 1H, N=$\underline{CH}$-N); 9,77 (s, 1H, C-CH=O); 6,17 et 9,16 (dl et tl, 2H, NH).
**SM :** 616 (MH+); 560 (MH-tBu+); 426 (MH-COOCH2Ph+).

Synthèse de l'acide 2-benzyloxycarbonylamino-3-{5-formyl-6-[4-(5,6,7,8-tétrahydro-[1,8]naphthyridin-2-yl)-pipéridin-1-yl]-pyrimidin-4-ylamino}-propionique bis(trifluoroacetate).

On agite 120 mg (0,195 mmole) de 2-benzyloxycarbonylamino-3-{5-formyl-6-[4-(5,6,7,8-tétrahydro-[1,8] naphthyridin-2-yl)-pipéridin-1-yl]-pyrimidin-4-ylamino}-propionate de tert-butyle dans 10 ml de dichlorométhane avec 1 ml d'acide trifluoroacétique à température ambiante pendant 24 heures. On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 60 mg de produit attendu sous forme d'un solide blanc.
**CCM :** Rf=0,25 (silicagel, éluant : dichlorométhane-méthanol-eau-acide acétique 90-10-1-1)
**SM :** 560 (MH+); 426 (MH-$COOCH_2Ph$+); 558- (M-H-); 450- (558-$OCH_2Ph$-) ; 1117- (2M-H-).

Exemple comparatif 22

Synthèse du 2-benzyloxycarbonylamino-3-{5-hydroxyméthyl-6-[4-(5,6,7,8-tétrahydro-[1,8]naphthyridin-2-yl)-pipéridin-1-yl]-pyrimidin-4-ylamino}-propionate de tert-butyle

On agite 340 mg (0,55 mmole) de 2-benzyloxycarbonylamino-3-{5-formyl-6-[4-(5,6,7,8-tétrahydro-[1,8] naphthyridin-2-yl)-pipéridin-1-yl]-pyrimidin-4-ylamino}-propionate de tert-butyl dans 50 ml de méthanol avec 350 mg (9,2 mmoles) de borohydrure de sodium à température ambiante pendant 2 heures. On ajoute ensuite 100 ml d'une solution saturée de chlorure d'ammonium et évapore le mélange réactionnel à sec sous pression réduite (2 kPa). Le résidu obtenu est repris par un mélange d'eau et d'acétate d'éthyle. La phase organique est séchée sur sulfate de magnésium puis le solvant évaporé sous pression réduite (2 kPa). Le brut obtenu est chromatographié sur silicagel en éluant avec un gradient de chlorure de méthylène et d'acétate d'éthyle (100-0 à 0-100). On obtient 160 mg de produit attendu sous la forme d'une huile jaune.
**SM :** 618 (MH+); 616- (M-H-); 508- (616-OCH2Ph-)
**1H-RMN ($CDCl_3$) :** δ 1,48 (s, 9H, tBu); 1,80 à 2,05(m, 6H, $CH_2$-$\underline{CH_2}$-$CH_2$-NH et $\underline{CH_2}$-CH-$\underline{CH_2}$); 2,72 (t,2H, $\underline{CH_2}$-$CH_2$-$CH_2$-NH); 2,87 (tl, 1H, $CH_2$-$\underline{CH}$-$CH_2$); 2,99 et 3,81 (tl et dl, 4H, $CH_2$-$\underline{CH_2}$-N-$\underline{CH_2}$-$CH_2$); 3,42 (m, 2H, $CH_2$-$CH_2$-$\underline{CH_2}$-NH); 3,90 (m, 2H, NH-$\underline{CH_2}$-CH-NH); 4,44 (m, 1H, NH-$CH_2$-$\underline{CH}$-NH) ; 5,09 (s, 2H, $\underline{CH_2}$-Ph); 6,40 et 7,14 (2d, 2H, $\underline{CH}$=$\underline{CH}$ naphthyridine); 7,32 (m, 5H, Ph)); 8,27 (s, 1H, N=$\underline{CH}$-N); 5,98 et 6,18 (m et dl, 2H, NH).

Synthèse de l'acide 2-benzyloxycarbonylamino-3-{5-hydroxyméthyl-6-[4-(5,6,7,8-tétrahydro-[1,8]naphthyridin-2-yl)-pipéridin-1-yl]-pyrimidin-4-ylamino}-propionique, bis(trifluoroacetate).

On agite 150 mg (0,24 mmole) de 2-benzyloxycarbonylamino-3-{5-hydroxyméthyl-6-[4-(5,6,7,8-tétrahydro-[1,8] naphthyridin-2-yl)-pipéridin-1-yl]-pyrimidin-4-ylamino}-propionate de tert-butyle dans 15 ml de dichlorométhane avec 0,8 ml d'acide trifluoroacétique à température ambiantependant 24 heures. On rajoute du toluène et évapore le mélange

réactionnel à sec sous pression réduite (2 kPa). Le résidu est solubilisé dans le minimum de dichlorométhane puis versé sur de l'éther diisopropylique. Le précipité est filtré. On obtient 100 mg de produit attendu sous forme d'un solide blanc.
**CCM :** Rf=0,40 (silicagel, éluant : dichlorométhane-méthanol-eau-acide acétique 85-15-2-2)
**SM :** 562 (MH+); 560- (M-H-); 452- (560-OCH2Ph-); 1121- (2M-H-).

Exemple comparatif 23

En opérant comme décrit aux exemples précédents, par l'intermédiaire de la 5-amino-4,6-dichloro-pyrimidine et de 2,5-diméthoxytétrahydrofuranne, on prépare le 2-benzyloxycarbonylamino-3-{5-pyrrol-1-yl-6-[4-(5,6,7,8-tétrahydro-[1,8]naphthyridin-2-yl)-pipéridin-1-yl]-pyrimidin-4-ylamino}-propionate de tert-butyle
**CCM :** Rf=0,7 (silicagel, éluant : CH2Cl2/ MeOH 90:10)
**SM :** 653 (MH+); 463 (MH-(tBu et CO-O-CH2-C6H5)+ puis l'acide 2-benzyloxycarbonylamino-3-{5-pyrrol-1-yl-6-[4-(5,6,7,8-tétrahydro-[1,8]naphthyridin-2-yl)-pipéridin-1-yl]-pyrimidin-4-ylamino}-propionique
**CCM :** Rf=0,5 (silicagel, éluant : CH2Cl2/ MeOH 90:10)
**SM :** 597 (MH+); 463 (MH-(CO-O-CH2-C6H5)+
**1H-RMN (MeOD) :** $\delta$ de 1,60 à 2.00 (3m, 6H, H2, H7 et H7'); de 2,70 à 2,85(m, 3H, H6 et H3); 3,5(m, 2H, H1); 3,62 et 3,92(2m, 2H, H12) ; 4,35(m, 1H, H13); 5,10(m, 2H, -O-C$H_2$-$C_6H_5$) ; 6,32(m, 2H, H11) ; 6,55(d, 1H, H5); 6,67(m, 2H, H10) ; de 7,25 à 7,40(m, 5H, - O-CH2-C6H5); 7,55(d, 1H, H4); 8,12(m, 1H, H9)

Exemples comparatifs 24 à 37

Mode opératoire général de préparation des amines

**Etape a)**

On laisse agiter 10 minutes à TA 75 mg (0,35 mmoles) de triacétoxyborohydrure de sodium dans 6 ml de tétrahydrofurane. Puis on ajoute un mélange de 100 mg (0,208 mmoles) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle et 0,228 mmoles d'aldéhyde en solution dans 3 ml de tétrahydrofurane. Pour certains aldéhydes la réaction étant plus lente, soit le mélange réactionnel est agité à température ambiante pendant 4 heures, soit il est chauffé au reflux pendant 4 heures. Le mélange réactionnel est ensuite extrait à l'acétate d'éthyle après lavage par une solution de bicarbonate de sodium saturée. La phase organique obtenue est séchée sur du sulfate de magnésium avant d'être concentrée à sec sous pression réduite (2 kPa). Le résidu est ensuite chromatographié sur silicagel avec l'éluant suivant : Acétate d'éthyle-dichlorométhane/méthanol(90/10) 50-50. On obtient une masse $m_y$ de produit attendu.

**CCM :** Rf (éluant : dichlorométhane-méthanol(90/10)- acétate d'éthyle 50-50.

**Etape b)**

On agite une masse $m_y$ de 3-{5-éthyl-6-[4-(5,6,7,8-tétrahydro-[1,8]naphthyridin-2-yl)-pipéridin-1-yl]-pyrimidin-4-ylamino}-2-alkylamino-propionate de tert-butyle dans 5 ml de dichlorométhane avec 850 $\mu$l d'acide trifluoroacétique à température ambiante jusqu'à disparition du produit de départ en CCM (silicagel, éluant : $CH_2Cl_2$-MeOH-$H_2O$-AcOH 90-10-1-1). On rajoute du toluène et on évapore le mélange réactionnel à sec sous pression réduite (2 kPa). Le résidu est solubilisé dans un minimum de dichlorométhane puis versé sur de l'éther diisopropylique. Le précipité est filtré. On obtient une masse $m_z$ de l'acide attendu.
**CCM :** Rf (éluant : dichlorométhane-méthanol-eau-acide acétique 90-10-1-1).

Exemple comparatif 24

**Etape a)**

Synthèse du 2-(2-Ethyl-butylamino)-3-{5-éthyl-6-[4-(5,6,7,8-tétrahydro-[1,8]naphthyridin-2-yl)-pipéridin-1-yl]-pyrimidin-4-ylamino}-propionate de tert-butyle.

On laisse agiter 10 minutes à TA 75 mg (0,35 mmoles) de triacétoxyborohydrure de sodium dans 6 ml de tétrahydrofurane. Puis on ajoute un mélange de 100 mg (0,208 mmoles) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle et 0,228 mmoles de 2-éthylbutylaldéhyde en solution dans 3 ml de tétrahydrofurane. Le mélange réactionnel est agité à température ambiante

pendant 4 heures 30. Puis il est extrait à l'acétate d'éthyle après lavage par une solution de bicarbonate de sodium saturée. La phase organique obtenue est séchée sur du sulfate de magnésium avant d'être concentrée à sec sous pression réduite (2 kPa). Le résidu est ensuite chromatographié sur silicagel avec l'éluant suivant : Acétate d'éthyle-dichlorométhane/méthanol(80/20) 50-50. On obtient une masse $m_y$ de produit attendu.
**CCM :** Rf=0,27 (éluant : dichlorométhane-méthanol(80/20)- acétate d'éthyle 50-50).
**1H-RMN ($CDCl_3$)** : δ 0,9 (t, 6H, CH3-CH2-CH-CH2-CH3); 1,2 (t, 3H, CH2-CH3) ; 1,47 (s, 9H, tBu); 1,7 (m, 1H, $(CH2CH3)_2$-CH-CH2), 1,85 et 2,05 (2m, 6H, N-CH2-CH2-CH-CH2-CH2, CH2-CH2-CH2-NH); 2,4 et 2,53 (m, 3H, N-CH2-CH2-CH-CH2-CH2, $(CH2CH3)_2$-CH-CH2-NH); 2,6 et 2,75 (m, 4H, CH2-CH2-CH2-NH, CH2-CH3); 2,96 et 3,56 (2m, 4H, N-CH2-CH2-CH-CH2-CH2); 3,32 (m, 1H, NH-CH2-CH-NH); 3,45 (m, 2H, CH2-CH2-CH2-NH); 3,7 et 3,9 (m, 2H, NH-CH2-CH-NH); 5,42 (t, 1H, NH mobile); 6,43 et 7,17 (2d, 2H, CH=CH naphthyridine); 8,32 (s, 1H, N=CH-N).
**SM :** 566(MH+), 510(MH-tBu).

**Etape b)**

Synthèse de l'Acide 2-(2-Ethyl-butylamino)-3-{5-éthyl-6-[4-(5,6,7,8-tétrahydro-[1,8]naphthyridin-2-yl)-pipéridin-1-yl]-pyrimidin-4-ylamino}-propionique, Bis(trifluoroacetate)

On agite 80 mg (0,141 mmoles) de 3-[[5-éthyl-6-[4-(1,2,3,4-tétrahydro-1,8-naphthyridin-7-yl)-1-pipéridinyl]-4-pyrimidinyl]amino]-N-(4-méthoxy-benzoyle)alaninate de (1,1-diméthyléthyle) dans 5 ml de dichlorométhane avec 400 μl d'acide trifluoroacétique à température ambiante pendant 9 heures.
On ajoute alors du toluène et on évapore le mélange réactionnel à sec sous pression réduite (2 kPa). Le résidu est solubilisé dans le minimum de dichlorométhane puis versé sur de l'éther diisopropylique. Le précipité est filtré. On obtient 90 mg de produit attendu.
**CCM :** Rf=0,50 (silicagel, éluant : dichlorométhane-méthanol-eau-acide acétique 70-30-6-3).
**1H-RMN ($CDCl_3$)** : δ 0,9 (t, 6H, CH3-CH2-CH-CH2-CH3); 1,2 (t, 3H, CH2-CH3); 1,8 (m, 1H, $((CH2CH3)_2$-CH-CH2), 1,85 et 2,1 (2m, 6H, N-CH2-CH2-CH-CH2-CH2, CH2-CH2-CH2-NH); 2,56 (q, 2H, CH2-CH3); 2,78 (t, 2H, CH2-CH2-CH2-NH); 2,9 et 3,1 (m, 3H, $(CH2CH3)_2$-CH-CH2-NH, N-CH2-CH2-CH-CH2-CH2); 3,28 et 3,9 (2m, 4H, N-CH2-CH2-CH-CH2-CH2); 3,53 (m, 2H, CH2-CH2-CH2-NH); 4,25 (m, 2H, NH-CH2-CH-NH); 6,4 et 7,38 (2d, 2H, CH=CH naphthyridine); 8,33 (s, 1H, N=CH-N) ; 10,05 (m, 1H, COOH mobile).
**+** 510(MH+).
$M_x$ : masse d'aldéhyde introduit
$M_y$ : masse d'ester obtenu.
$M_z$ : masse d'acide obtenu.

| Exemple comparatif | Acide obtenu | FW (Base libre) | FW (Base libre + 2 TFA) | Mz (mg) |
|---|---|---|---|---|
| 25 | 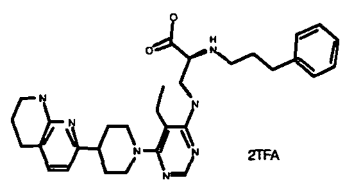 | 543.72 | 771.72 | 90 |
| 26 | 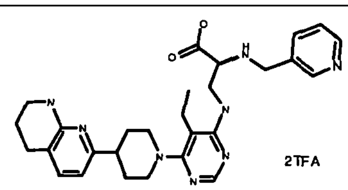 | 516.65 | 744.65 | 25 |
| 27 | 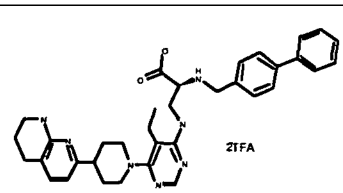 | 591.76 | 819.76 | 30 |

(suite)

| Exemple comparatif | Acide obtenu | FW (Base libre) | FW (Base libre + 2 TFA) | Mz (mg) |
|---|---|---|---|---|
| 28 | 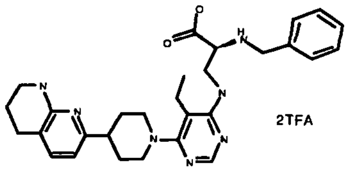 | 571.77 | 799.77 | 35 |
| 29 | 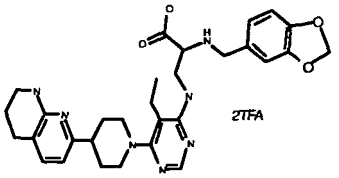 | 559.67 | 787.67 | 46 |
| 30 | 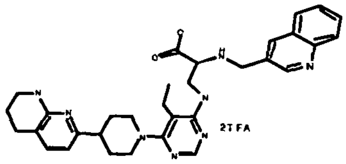 | 566.71 | 794.71 | 34 |
| 31 | 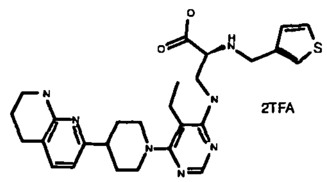 | 521.69 | 749.69 | 14 |
| 32 | 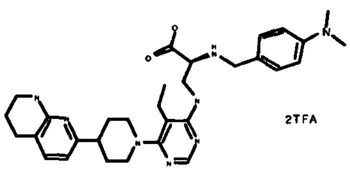 | 558.73 | 786.73 | 38 |
| 33 | 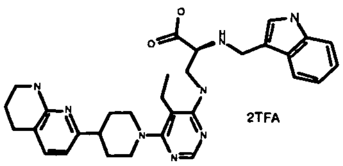 | 554.70 | 782.70 | 13 |
| 34 | 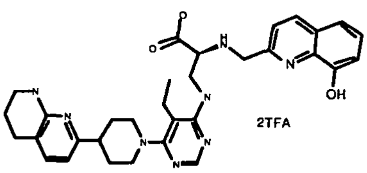 | 582.71 | 810.71 | 14 |
| 35 | 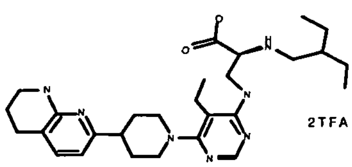 | 509.70 | 737.70 | 90 |

(suite)

| Exemple comparatif | Acide obtenu | FW (Base libre) | FW (Base libre + 2 TFA) | Mz (mg) |
|---|---|---|---|---|
| 36 | 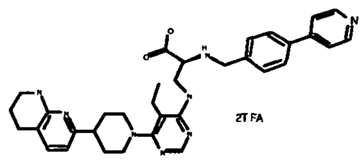 | 592.75 | 820.75 | 10 |
| 37 | 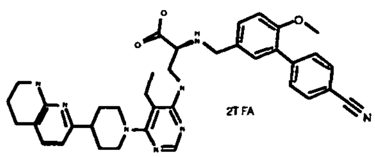 | 646.80 | 876.80 | 40 |

Les produits obtenus ci-dessus sont préparés à partir des matières premières suivantes :

| Exemple comparatif | Aldéhyde | FW | Mx (mg) | produit attendu | FW | $M_y$ (mg) | Rdt |
|---|---|---|---|---|---|---|---|
| 25 | 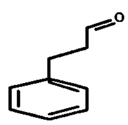 | 138.18 | 28 | 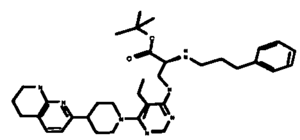 | 599.83 | 100 | 94 |
| 26 | 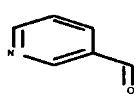 | 107.11 | 25 | 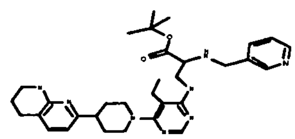 | 572.76 | 37 | 31 |
| 27 | 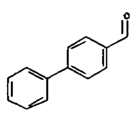 | 182.22 | 42 | 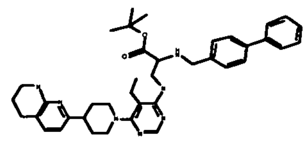 | 647.87 | 20 | 15 |
| 28 | 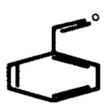 | 106.12 | 24 | 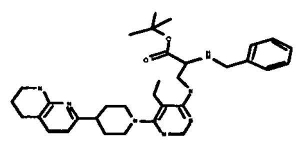 | 571.77 | 36 | 30 |
| 29 | 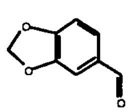 | 150.13 | 34 | 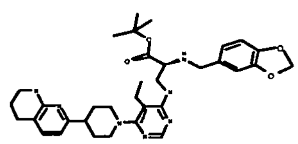 | 615.78 | 40 | 31 |
| 30 | 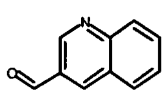 | 157.17 | 36 | 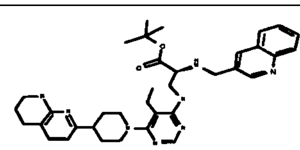 | 622.82 | 79 | 61 |
| 31 | 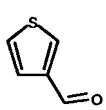 | 112.15 | 26 | 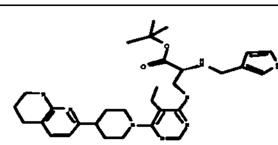 | 577.8 | 26 | 21 |

(suite)

| Exemple comparatif | Aldéhyde | FW | Mx (mg) | produit attendu | FW | $M_y$ (mg) | Rdt |
|---|---|---|---|---|---|---|---|
| 34 | 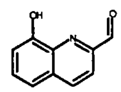 | 173,17 | 40 | 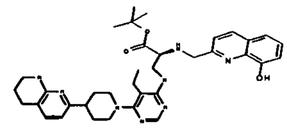 | 638,82 | 30 | 23 |
| 35 | 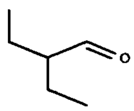 | 100,16 | 22 | 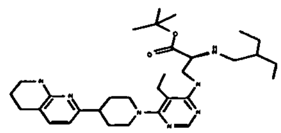 | 565,81 | 89 | 38 |
| 36 | 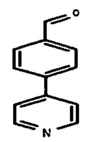 | 183,21 | 42 | 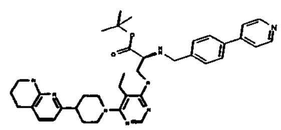 | 648,86 | 32 | 24 |
| 37 | 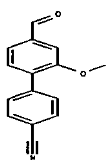 | 237,26 | 54 | 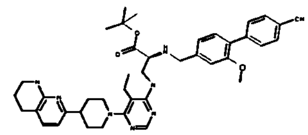 | 702,91 | 42 | 29 |

Exemple comparatif 38

Synthèse du 3-[5-méthyl-6-[4-(5,6,7,8-tétrahydro-(1,8)naphtyridin-2-yl)-pipéridin-1-yl]-pyrimidin-4-ylamino]-2-(4-méthoxy benzoyl)-propionate de tert-butyle

A un mélange de 109 mg (0,233 mmoles) de 2-amino-3-{5-méthyl-6-[4-(5,6,7,8-tétrahydro-[1,8]naphthyridin-2-yl)-pipéridin-1-yl]-pyrimidin-4-ylamino}-propionate de tert-butyle en solution dans 6 ml de dichlorométhane et 650 µl de pyridine, on additionne 40 mg (0,233 mmoles)de chlorure de 4-méthoxy-benzoyle en solution dans 3 ml de dichlorométhane. Le mélange réactionnel est agité à température ambiante pendant 1 heure. Puis, le solvant est évaporé sous pression réduite (2 kPa) et le résidu est chromatographié sur silicagel avec l'éluant suivant : acétate d'éthyle-dichlorométhane 50-50, acétate d'éthyle-dichlorométhane/méthanol (95/5) 50-50 puis en terminant par acétate d'éthyle-dichlorométhane/méthanol (9/1) 50-50. On obtient 98 mg de produit attendu.

**CCM :** Rf=0,62 (silicagel, éluant : dichlorométhane-méthanol-eau-acide acétique 85-15-2-2)

**1H-RMN ($CDCl_3$)** : δ 1,54ppm (s, 9H, tBu) ; 1, 20 (t, 3H, $CCH_3$) ; 1, 78 à 2,05 (m, 6H, $CH_2$-CH-$CH_2$ et $CH_2$-$CH_2$-$CH_2$-NH) ; 2, 66 (tt, 1H, $CH_2$-CH-$CH_2$) ; 2,72 (t, 2H, $CH_2$-$CH_2$-$CH_2$-NH) ; 2,92 et 3,68 (ql et m, 4H, $CH_2$-$CH_2$-N-$CH_2$-$CH_2$); 3,42 (m, 2H, $CH_2$-$CH_2$-$CH_2$-NH); 3,86 (s, 3H, $COCH_3$); 3,97 (t, 2H, NH-$CH_2$-CH-NH); 4,78 (q, 1H, NH-$CH_2$-CH-NH) ; 5,17 (t, 1H, 1H, NH-$CH_2$-CH-NH); 6,41 et 7,13 (2d, 2H, H naphthyridine); 6,92 et 7,79 (2d, 4H, H benzoyl); 8,32ppm (s, 1H, N=CH-N); 8,23 (H mobile).

**SM :** 602 (MH+); 412 (MH-COOCH2Ph+).

Synthèse de l'acide 3-[5-méthyl-6-[4-(5,6,7,8-tétrahydro-(1,8)naphtyridin-2-yl)-pipéridin-1-yl]-pyrimidin-4-ylamino]-2-(4-méthoxy benzoyl)-propionique, bis(trifluoroacétate)

On agite 20 mg (0,033 mmole) de 3-[5-méthyl-6-[4-(5,6,7,8-tétrahydro-(1,8)naphtyridin-2-yl)-pipéridin-1-yl]-pyrimidin-4-ylamino]-2-(4-méthoxy benzoyl)-propinate de tert-butyle dans 2 ml de dichlorométhane avec 0,3 ml d'acide trifluoroacétique à température ambiante pendant 3 heures. On rajoute du toluène et évapore le mélange réactionnel à sec sous pression réduite (2 kPa). Le résidu est solubilisé dans le minimum de dichlorométhane puis versé sur de l'éther diisopropylique. Le précipité est filtré. On obtient 20 mg de produit attendu sous forme d'un solide beige.

**1H-RMN ($CDCl_3$) :** δ 1,77 à 2,15 (m, 6H, $CH_2$-CH-$CH_2$ et $CH_2$-$CH_2$-$CH_2$-NH); 2,37 (s, 3H, $CCH_3$); 2,77 (tl, 2H, $CH_2$-$CH_2$-$CH_2$-NH); 2,99 (tl, 1H, $CH_2$-CH-$CH_2$); 3,22 et 3,97 (tl et dl, 4H, $CH_2$-$CH_2$-N-$CH_2$-$CH_2$); 3,52 (m, 2H, $CH_2$-$CH_2$-$CH_2$-NH); 3,91 et 4,14 (2m, 2H, NH-$CH_2$-CH-NH); 4,82 (m, 1H, NH-$CH_2$-CH-NH); 6,89 et 7,82 (2d, 2H, H naphthyridine); 7,19 et 7,38 (2d, 4H, Hbenzoyl); 8,25ppm (s, 1H, N=CH-N); 6,39; 7,72; 8,09 et 9,61 H mobiles.

**SM :** 546 (MH+)

Exemples comparatifs 39 à 51

Mode opératoire général de préparation des amides

**Etape a)**

A 112,5 mg (0,23 mmoles) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle en solution dans 6 ml de dichlorométhane on ajoute 650 µl de pyridine, après agitation pendant 15 minutes à TA, on additionne 0,23 mmoles (masse $m_x$) de chlorure d'acide en solution dans 3 ml de dichlorométhane. Le mélange réactionnel est agité à température ambiante pendant 1 à 3 heures selon le chlorure d'acide utilisé. Puis, le solvant est évaporé sous pression réduite (2 kPa) et le résidu est chromatographié sur silicagel avec l'éluant suivant : acétate d'éthyle-dichlorométhane 50-50, acétate d'éthyle-dichlorométhane/méthanol (95/5) 50-50 et terminant par acétate d'éthyle- dichlorométhane/méthanol (90-10) 50-50. On obtient une masse $m_y$ de produit attendu.
**CCM :** Rf (éluant : dichlorométhane-méthanol(90/10)- acétate d'éthyle 50-50).

**Etape b)**

On agite une masse $m_y$ de de tert-butyle ester de l'etape a) dans 5 ml de dichlorométhane avec 850 µl d'acide trifluoroacétique à température ambiante jusqu'à disparition du produit de départ en CCM (silicagel, éluant : $CH_2Cl_2$-MeOH-$H_2O$-AcOH 90-10-1-1). On rajoute du toluène et on évapore le mélange réactionnel à sec sous pression réduite (2 kPa). Le résidu est solubilisé dans le minimum de dichlorométhane puis versé sur de l'éther diisopropylique. Le précipité est filtré. On obtient une masse $m_z$ de bis trifluoro acétate de l'acide attendu.
**CCM :** Rf (éluant : dichlorométhane-méthanol-eau-acide acétique 90-10-1-1

Exemple comparatif 39

**Etape a)**

Synthèse du 3-[5-éthyl-6-[4-(5,6,7,8-tétrahydro-(1,8)naphtyridin-2-yl)-pipéridin-1-yl]-pyrimidin-4-ylamino]-2-(4-méthoxy benzoyl)-propionate de tert-butyle

A un mélange de 112,5 mg (0,233 mmoles) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle en solution dans 6 ml de dichlorométhane et 650 µl de pyridine, on additionne 40 mg (0,233 mmoles)de chlorure de 4-méthoxy-benzoyle en solution dans 3 ml de dichlorométhane. Le mélange réactionnel est agité à température ambiante pendant 1 heure. Puis, le solvant est évaporé sous pression réduite (2 kPa) et le résidu est chromatographié sur silicagel avec l'éluant suivant : acétate d'éthyle-dichlorométhane (50-50), acétate d'éthyle-dichlorométhane/méthanol (95/5) 50-50 puis en terminant par acétate d'éthyle-dichlorométhane/méthanol (9/1) 50-50. On obtient 98 mg de produit attendu.
**CCM :** Rf=0,62 (silicagel, éluant : dichlorométhane-méthanol-eau-acide acétique 85-15-2-2)
**$^1$H-RMN ($CDCl_3$)** : δ 1,13 (t, 3H, $CH_2$-CH3) ; 1,42 (s, 9H, tBu) ; 1,73 et 1,93 (2m, 6H, N-CH2-CH2-CH-CH2-CH2, CH2-CH2-CH2-NH); 2,41 (q, 2H, CH2-CH3); 2,62 (m, 3H, CH2-CH2-CH2-NH, N-CH2-CH2-CH-CH2-CH2); 3,90 et 3,53 (2m, 4H, N-CH2-CH2-CH-CH2-CH2); 3,35 (m, 2H, CH2-CH2-CH2-NH); 3,78 (s, 3H, O-CH3); 3,85 (m, 2H, NH-CH2-CH-); 4,71 (m, 1H, NH-CH2-CH-NH) ; 5,20 (t, 1H, NH mobile) 7,07 et 8,12 (2d, 2H, CH=CH naphthyridine); 6,85 et 7,72(2d, 4H, CH=CH phényl); 8,25 (s, 1H, N=CH-N).
**SM :** 616 (MH+), 560 (MH-tbu).

**Etape b)**

Synthèse du 3-[5-éthyl-6-[4-(5,6,7,8-tétrahydro-(1,8)naphtyridin-2-yl)-pipéridin-1-yl]-pyrimidin-4-ylamino]-2-(4-méthoxy benzoyl)-propionique, bis(trifluoroacétate)

On agite 90 mg (0,146 mmoles) de 3-[5-éthyl-6-[4-(5,6,7,8-tétrahydro-(1,8)naphtyridin-2-yl)-pipéridin-1-yl]-pyrimidin-4-ylamino]-2-(4-méthoxy benzoyl)-propionate de tert-butyle dans 5 ml de dichlorométhane avec 850 µl d'acide trifluoroacétique à température ambiante pendant 7 heures.
On ajoute ensuite du toluène et on évapore le mélange réactionnel à sec sous pression réduite (2 kPa). Le résidu est solubilisé dans le minimum de dichlorométhane puis versé sur de l'éther diisopropylique. Le précipité est filtré. On obtient 85 mg de produit attendu.

**CCM :** Rf=0,27 (silicagel, éluant : dichlorométhane-méthanol-eau-acide acétique 90-10-1-1).
**1H-RMN ($CDCl_3$)** : δ 1,17 (t, 3H, CH2-**CH3**); 1,82 et 2,05 (2m, 6H, N-CH2-CH2-CH-CH2-CH2, CH2-CH2-CH2-NH); 2,53 (q, 2H, CH2-CH3); 2,77 (t, 2H, CH2-CH2-CH2-NH); 2,98 (m, 1H, N-CH2-CH2-CH-CH2-CH2); 3,25 et 3,90 (2m, 4H, N-CH2-CH2-CH-CH2-CH2); 3,52 (m, 2H, CH2-CH2-CH2-NH); 3,82 (s, 3H, O-CH3); 4,20 (m, 2H, NH-CH2-CH-) 4,85 (m, 1H, NH-CH2-CH-NH); 6,40 et 7,75 (2m, 1H, NH mobile) 7,37 et 8,17 (2d, 2H, CH=CH naphtiridine); 6,90 et 8,85(2d, 4H, CH=CH phényl); 8,32 (s, 1H, N=CH-N).
**SM :** 560(MH+).
$M_x$ : masse de chlorure d'acide introduit.
$M_y$ : masse d'ester obtenu.
$M_z$ : masse d'acide obtenu.

| Exemple comparatif | Acide obtenu | FW (Base libre) | FW (Base libre + 2TFA) | Mz (mg) | SM (MH+) |
|---|---|---|---|---|---|
| 39 | 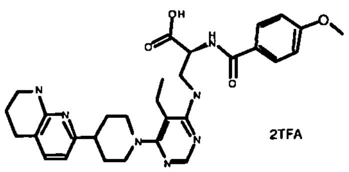 2TFA | 559.67 | 787.67 | 85 | 560 |
| 40 | 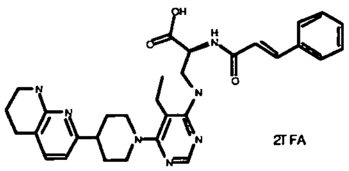 2TFA | 555.69 | 783.69 | 50 | 556 |
| 41 | 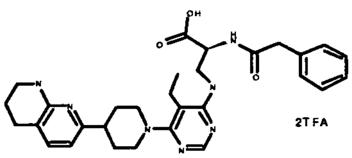 2TFA | 543.67 | 771.67 | 62 | 544 |
| 42 | 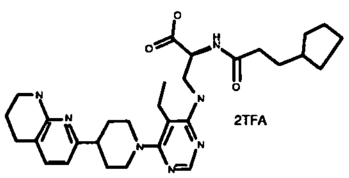 2TFA | 549.72 | 777.72 | 70 | 550 |
| 43 | 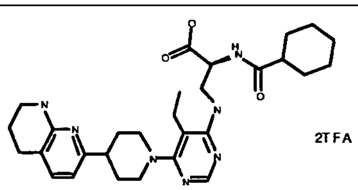 2TFA | 535.70 | 763.7 | 66 | 536 |
| 44 | 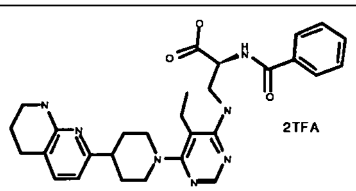 2TFA | 529.65 | 757.65 | 84 | 530 |
| 45 | 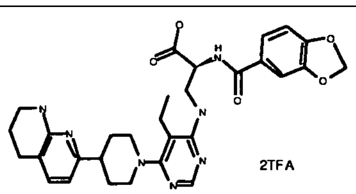 2TFA | 573.66 | 801.66 | 89 | 574 |

(suite)

| Exemple comparatif | Acide obtenu | FW (Base libre) | FW (Base libre + 2TFA) | Mz (mg) | SM (MH+) |
|---|---|---|---|---|---|
| 46 | 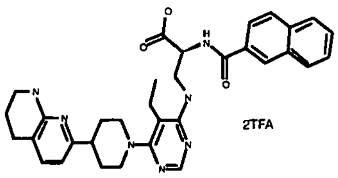 | 579.71 | 807.71 | 95 | 580 |
| 47 | 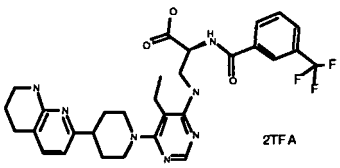 | 597.64 | 825.64 | 64 | 598 |
| 48 | 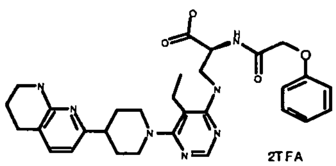 | 559.67 | 787.67 | 80 | 560 |
| 49 | 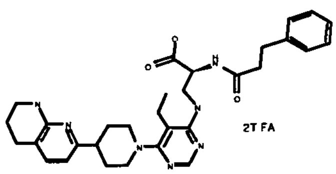 | 557.70 | 785.7 | 75 | 558 |
| 50 | 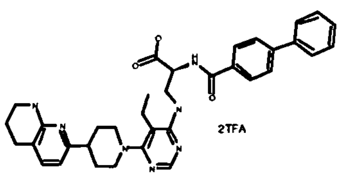 | 605.75 | 833.75 | 123 | 605 |
| 51 | 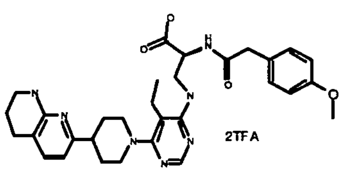 | 573.70 | 801.7 | 155 | 574 |

Les produits obtenus ci-dessus sont préparés à partir des matières premières suivantes :

| Exemple comparatif | Chlorure d'acide | mx (mg) | ester formé | FW | $M_y$ (mg) | Rdt | SM (MH+) | Rf |
|---|---|---|---|---|---|---|---|---|
| 39 | 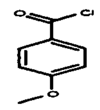 | 40 | 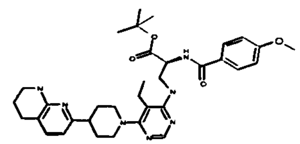 | 615.78 | 98 | 68 | 616 | 0.53 |
| 40 | 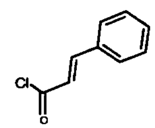 | 30 | 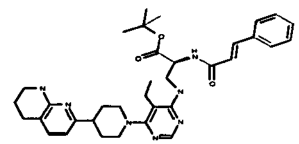 | 611.79 | 50 | 46 | 612 | |
| 41 | 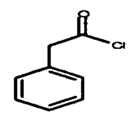 | 36.2 | 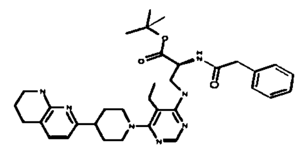 | 599.78 | 81 | 58 | 600 | 0.50 |
| 42 | 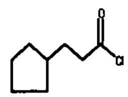 | 37.4 | 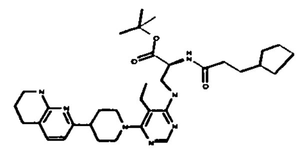 | 605.83 | 81 | 57 | 606 | 0.53 |
| 43 | 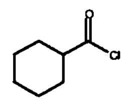 | 34.2 | 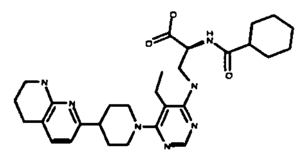 | 591.8 | 74 | 53.5 | 592 | 0.53 |
| 44 | 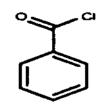 | 33 | 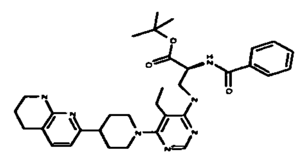 | 585.76 | 86 | 63 | 586 | 0.47 |
| 45 | 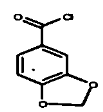 | 43 | 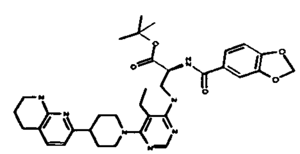 | 629.77 | 92 | 62.5 | 630 | 0.50 |

(suite)

| Exemple comparatif | Chlorure d'acide | mx (mg) | ester formé | FW | $M_y$ (mg) | Rdt | SM (MH+) | Rf |
|---|---|---|---|---|---|---|---|---|
| 46 | 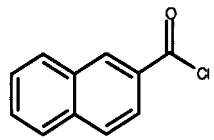 | 44.5 | 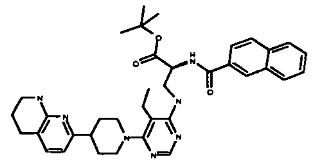 | 635.82 | 94 | 63 | 636 | 0.53 |
| 47 | 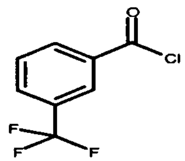 | 48.7 | 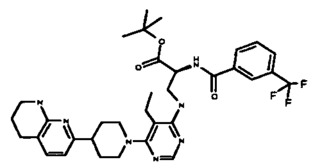 | 653.75 | 94 | 61.5 | 654 | 0.53 |
| 48 | 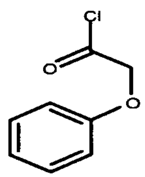 | 40 | 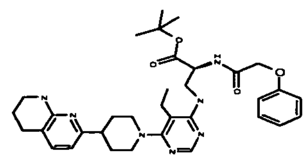 | 615.78 | 92 | 64 | 616 | 0.58 |
| 49 | 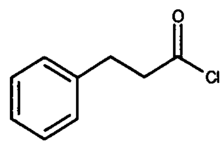 | 39.4 | 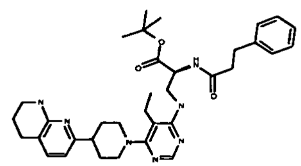 | 613.81 | 85 | 59 | 614 | 0.58 |
| 50 | 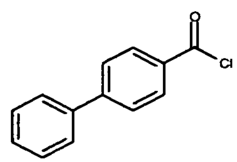 | 46 | 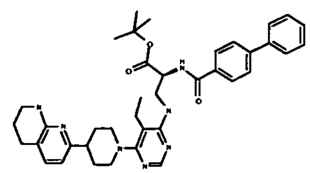 | 661.85 | 63 | 38 | 662 | 0.20 |
| 51 | 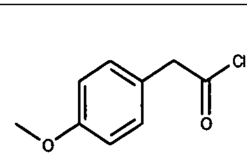 | 36 | 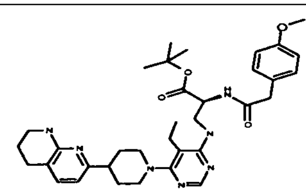 | 629.81 | 90 | 57 | 630 | 0.16 |

Exemple comparatif 52

Synthèse du 3-[5-éthyl-6-[4-(5,6,7,8-tétrahydro-(1,8)naphtyridin-2-yl)-pipéridin-1-yl]-pyrimidin-4-ylamino]-2-[3-(2-nitrophényl)-uréido]-propionate de tert-butyle

Un mélange de 240 mg (0,50 mmole) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle et de 82 mg (0,50 mmole) de 2-nitrophénylisocynate dans 15 ml de tétrahydrofurane est agité durant 3 heures à température ambiante. Le mélange réactionnel est évaporé à sec sous pression réduite (2 kPa) et le résidu est repris par de l'acétate d'éthyle et de l'eau. La phase organique est séparée, séchée sur sulfate de magnésium et le solvant évaporé sous pression réduite (2 kPa). Le résidu est chromatographié sur alumine en éluant avec un gradiant d'acétate d'éthyle-chlorure de méthylène-méthanol de 50-50-0 à 50-50-10. On obtient 260 mg de produit attendu sous forme d'un solide jaune.
**CCM :** Rf=0,12 (silicagel, éluant : acétate d'éthyle-chlorure de méthylène-méthanol 50-47-3).
**1H-RMN ($CDCl_3$)** : δ 1,20 (t, 3H, $CH_2\underline{CH_3}$) ; 1,45 (s, 9H, tBu); 1,80 à 2,02(m, 6H, $\underline{CH_2}$-CH-$\underline{CH_2}$ et $CH_2$-$\underline{CH_2}$-$CH_2$-NH) ; 2,50 (q, 2H, $\underline{CH_2}$-$CH_3$); 2,66 (tl, 1H, $CH_2$-$\underline{CH}$-$CH_2$); 2,73 (t, 2H, $\underline{CH_2}$-$CH_2$-$CH_2$-NH); 2,97 et 3,62 (tl et m, 4H, $CH_2$-$\underline{CH_2}$-N-$\underline{CH_2}$-$CH_2$); 3,43 (m, 2H, $CH_2$-$CH_2$-$\underline{CH_2}$-NH); 3,95 (m, 2H, NH-$\underline{CH_2}$-CH-NH); 4,57 (m, 1H, NH-$CH_2$-$\underline{CH}$-NH); 5,03 (t, 1H, 1H, $\underline{NH}$-$CH_2$-CH-NH); 6,42 et 7,15 (2d, 2H, H naphthyridine); 7,03 et 7,58 (2t, 2H, C=CH-$\underline{CH}$=$\underline{CH}$-CH=C); 7,43 et 9,88 (d et s, H mobiles); 8,19 et 8,66 (2d, 2H, C=$\underline{CH}$-CH=CH-$\underline{CH}$=C); 8,36 ppm (s, 1H, N=$\underline{CH}$-N).
**SM :** 646 (MH+); 590 (MH-tBu+); 644- (M-H-).

Synthèse de l'acide 3-[5-éthyl-6-[4-(5,6,7,8-tétrahydro-(1,8)naphtyridin-2-yl)-pipéridin-1-yl]-pyrimidin-4-ylamino]-2-[3-(4-méthoxy-2-aminophényl)-uréido]-propionique, bis(trifluoroacetate)

On agite 110 mg (0,17 mmole) de 3-[5-éthyl-6-[4-(5,6,7,8-tétrahydro-(1,8)naphtyridin-2-yl)-pipéridin-1-yl]-pyrimidin-4-ylamino]-2-[3-(4-méthoxy-2-nitro-phényl)-réido]-propionate de tert-butyle et 70 mg de zinc dans 5 ml d'acide acétique à température ambiante jusqu'à disparition du produit de départ en CCM (silicagel, éluant : $CH_2Cl_2$-MeOH-$H_2O$-AcOH 90-10-1-1). On filter sur clarcel, rajoute du cyclohexane et évapore le mélange réactionnel à sec sous pression réduite (2 kPa). Le résidu est repris par de l'acétate d'éthyle et de l'eau. La phase organique est séparée, séchée sur sulfate de magnésium et le solvant évaporé sous pression réduite (2 kPa). Le brut est chromatographié sur alumine en éluant avec un gradiant de chlorure de méthylène-méthanol de 100-0 à 90-10.
Le produit obtenu est agité dans 4 ml de dichlorométhane avec 0,5 ml d'acide trifluoroacétique à température ambiante jusqu'à disparition du produit de départ en CCM (silicagel, éluant : $CH_2Cl_2$-MeOH-$H_2O$-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 40 mg de produit attendu sous forme d'un solide beige.
**CCM :** Rf=0,27 (silicagel, éluant : dichlorométhane-méthanol-eau-acide acétique 85-15-2-2)
**SM :** 662 (MH+); 606 (MH-tBu+); 660- (M-H-);
**1H-RMN (DMSO d6) :** δ 1,07 (m, 3H, $CH_2\underline{CH_3}$) ; 1,65 à 2,05 (m, 6H, $\underline{CH_2}$-CH-$\underline{CH_2}$ et $\underline{CH_2}$-$\underline{CH_2}$-$CH_2$-NH) ; 2,50 (masqué, 2H, $\underline{CH_2}$-$CH_3$); 2,77 (t, 2H, $\underline{CH_2}$-$CH_2$-$CH_2$-NH); 2,87 (m, 1H, $CH_2$-$\underline{CH}$-$CH_2$); 3,01 et 3,55 (tl et dl, 4H, $CH_2$-$\underline{CH_2}$-N-$\underline{CH_2}$-$CH_2$); 3,45 (m, 2H, $CH_2$-$CH_2$-$\underline{CH_2}$-NH); 3,72 et 3,84 (2m, 2H, NH-$\underline{CH_2}$-CH-NH); 4,53 (m, 1H, NH-$CH_2$-$\underline{CH}$-NH) ; 6,68 et 7,56 (2d, 2H, H naphthyridine); 7,65 à 7,00 (m, 2H, C=CH-$\underline{CH}$=$\underline{CH}$-CH=C); 8,12 et 8,50 (m, 2H, C=$\underline{CH}$-CH=CH-$\underline{CH}$=C); 8,28ppm (s, 1H, N=$\underline{CH}$-N).
**SM** : 560 (MH+); 558- (M-H-)

Exemples comparatifs 54 à 62

Mode opératoire général de préparation des urées :

**Etape a)**

A 120 mg (0,207 mmoles) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle en solution dans 5 ml de tétrahydrofurane on ajoute une masse $m_x$ (0,207 mmoles) d'isocyanate en solution dans 3 ml de tétrahydrofurane. Le mélange réactionnel est agité à température ambiante pendant 3 heures et 30 minutes. Puis, le solvant est évaporé sous pression réduite (2 kPa) et le résidu est chromatographié sur silicagel avec l'éluant suivant : Acétate d'éthyle 100%, acétate d'éthyle-dichlorométhane /méthanol(95/5) 50-50, acétate d'éthyle-dichlorométhane/méthanol(90-10) 50-50 et terminant par acétate d'éthyle/méthanol (95-5).
On obtient une masse $m_y$ de produit attendu.
**CCM :** Rf (éluant : acétate d'éthyle -méthanol(90/10).

**Etape b)**

On agite une masse my de tert-butyl ester de l'étape a) dans 5 ml de dichlorométhane avec 500 µl d'acide trifluoroacétique à température ambiante jusqu'à disparition du produit de départ en CCM (silicagel, éluant : $CH_2Cl_2$-MeOH-$H_2O$-AcOH 90-10-1-1). On rajoute du toluène et on évapore le mélange réactionnel à sec sous pression réduite (2 kPa). Le résidu est solubilisé dans le minimum de dichlorométhane puis versé sur de l'éther éthylique. Le précipité est filtré. Il s'agit d'un mélange du produit attendu et du sous produit de cyclisation. Ce mélange est purifié sur silice pour donner une masse $m_z$ de produit attendu.
**CCM :** Rf (éluant : dichlorométhane-méthanol-eau-acide acétique (90-10-1-1)

Exemple comparatif 53

Synthèse de l'acide 3-[5-éthyl-6-[4-(5,6,7,8-tétrahydro-(1,8)naphtyridin-2-yl)-pipéridin-1-yl]-pyrimidin-4-ylamino]-2-[3-(2-nitrophényl)-uréido]-propionique, bis(trifluoroacetate)

On agite 30 mg (0,046 mmole) de 3-[5-éthyl-6-[4-(5,6,7,8-tétrahydro-(1,8)naphtyridin-2-yl)-pipéridin-1-yl]-pyrimidin-4-ylamino]-2-[3-(2-nitrophényl)-uréido]-propinate de tert-butyle dans 3 ml de dichlorométhane avec 0,5 ml d'acide trifluoroacétique à température ambiante jusqu'à disparition du produit de départ en CCM (silicagel, éluant : $CH_2Cl_2$-MeOH-$H_2O$-AcOH 90-10-1-1). On rajoute du toluène et évapore le mélange réactionnel à sec sous pression réduite (2 kPa). Le résidu est solubilisé dans le minimum de dichlorométhane puis coulé sur de l'éther diisopropylique. Le précipité est filtré. On obtient 35 mg de produit attendu sous forme d'un solide jaune.
**CCM :** Rf=0,50 (silicagel, éluant : dichlorométhane-méthanol-eau-acide acétique 85-15-2-2)
**SM :** 590 (MH+); 588- (M-H-);
**1H-RMN ($CDCl_3$) :** δ 1,19 (t, 3H, $CH_2\underline{CH_3}$) ; 1,82 à 2,05 (m, 6H, $\underline{CH_2}$-CH-$\underline{CH_2}$ et $CH_2$-$\underline{CH_2}$-$CH_2$-NH); 2,50 (t, 2H, $\underline{CH_2}$-$CH_3$); 2,76 (t, 2H, $\underline{CH_2}$-$CH_2$-$CH_2$-NH); 2,98 (tl, 1H, $CH_2$-$\underline{CH}$-$CH_2$); 3,25 et 3,84 (tl et dl, 4H, $CH_2$-$\underline{CH_2}$-N-$\underline{CH_2}$-$CH_2$); 3,49 (m, 2H, $CH_2$-$CH_2$-$\underline{CH_2}$-NH); 3,87 et 4,06 (2m, 2H, NH-$\underline{CH_2}$-CH-NH); 4,63 (m, 1H, NH-$CH_2$-$\underline{CH}$-NH) ; 6,41 et 7,37 (2d, 2H, H naphthyridine);); 7,06 et 7,54 (2t, 2H, C=$\underline{CH}$-CH=$\underline{CH}$-CH=C); 8,11 et 8,32 (2d, 2H, C=$\underline{CH}$-CH=CH-$\underline{CH}$=C); 8,27 ppm (s, 1H, N=$\underline{CH}$-N).

Exemple comparatif 54

**Etape a)**

Synthèse du 2-(3-benzo[1,3]dioxol-5-yl-uréido)-3-{5-éthyl-6-[4-(5,6,7,8-tétrahydro-[1,8]naphthyridin-2-yl)-pipéridin-1-yl]-pyrimidin-4-ylamino}-propionate de tert-butyle

A 120 mg (0,207 mmoles) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle en solution dans 5 ml de tétrahydrofurane, on additionne 34 mg (0,207 mmoles)de 5-isocyanato-benzo[1,3]dioxole en solution dans 3 ml de tétrahydrofurane. Le mélange réactionnel est agité à température ambiante pendant 3 heures et 30 minutes. Puis, le solvant est évaporé sous pression réduite (2 kPa) et le résidu est chromatographié sur silicagel avec l'éluant suivant : Acétate d'éthyle 100%, acétate d'éthyle-dichlorométhane/méthanol(95/5) 50-50, acétate d'éthyle-dichlorométhane/méthanol(90-10) 50-50 et terminant par acétate d'éthyle/méthanol (95-5). On obtient 60 mg de produit attendu.
**CCM :** Rf=0,29 (silicagel, éluant : acétate d'éthyle -méthanol 90/10)
**1H-RMN ($CDCl_3$) :** δ 1,18 (t, 3H, $CH_2$-$\underline{CH_3}$) ; 1,47 (s, 9H, tBu) ; 1,8 et 2,05 (2m, 6H, N-CH2-$\underline{CH2}$-CH-$\underline{CH2}$-CH2, CH2-$\underline{CH2}$-CH2-NH); 2,48 (q, 2H, $\underline{CH2}$-CH3); 2,67 (m, 3H, N-CH2-CH2-$\underline{CH}$-CH2-CH2, $\underline{CH2}$-CH2-CH2-NH); 2,95 et 3,63 (2m, 4H, N-$\underline{CH2}$-CH2-CH-CH2-$\underline{CH2}$); 3,45 (m, 2H, CH2-CH2-$\underline{CH2}$-NH); 3,85 (m, 2H, NH-$\underline{CH2}$-CH-NH); 4,6 (m, 1H, NH-CH2-$\underline{CH}$-NH); 5,32 (t, 1H, $\underline{NH}$ mobile); 5,97 (s, 2H, O-CH2-O); 6,15 (dl, 1H, NH mobile); 6,42 et 7,2 (2d, 2H, CH=CH naphthyridine); 6,65 et 6,9 (3d, 3H, phényl); 8,12 (s, 1H, N=$\underline{CH}$-N).
**SM :** 645(MH+), 588(MH-tBu).

**Etape b)**

Synthèse de l'acide 2-(3-benzo[1,3]dioxol-5-yl-uréido)-3-{5-éthyl-6-[4-(5,6,7,8-tétrahydro-[1,8]naphthyridin-2-yl)-pipéridin-1-yl]-pyrimidin-4-ylamino}-propionique, bis(trifluoroacetate)

On agite 55 mg (0,085 mmoles) de 2-(3-benzo[1,3]dioxol-5-yl-ureido)-3-{5-éthyl-6-[4-(5,6,7,8-tétrahydro-[1,8]naphthyridin-2-yl)-pipéridin-1-yl]-pyrimidin-4-ylamino}-propionate de tert-butyle dans 5 ml de dichlorométhane avec 500

µl d'acide trifluoroacétique à température ambiante pendant 13 heures.

On ajoute alors du toluène et on évapore le mélange réactionnel à sec sous pression réduite (2 kPa). Le résidu est solubilisé dans le minimum de dichlorométhane puis versé sur de l'éther éthylique. Le précipité est filtré, séché, puis purifié sur silice.

On obtient 39 mg de produit attendu.

**CCM :** Rf=0,25 (silicagel, éluant : dichlorométhane-méthanol-eau-acide acétique 90-10-1-1.

**1H-RMN (MeOD) :** δ 1,17 (t, 3H, CH2-CH3); 1,85 et 2 (m, 6H, N-CH2-CH2-CH-CH2-CH2, CH2-CH2-CH2-NH); 2,55 (q, 2H, CH2-CH3); 2,8 (m, 2H, N-CH2-CH2-CH-CH2-CH2, CH2-CH2-CH2-NH); 2,95 et 3,5 (2m, 4H, N-CH2-CH2-CH-CH2-CH2); 3,33 (m, 2H, CH2-CH2-CH2-NH); 3,65 et 3,95 (m, 2H, NH-CH2-CH-); 4,47 (m, 1H, NH-CH2-CH-NH); 5,32 (t, 1H, NH mobile); 5,5 (s, 1H, NH mobile); 5,9 (s, 2H, O-CH2-O); 7,5 (d, 1H, CH=CH naphthyridine); 6,6 et 7,02 (m, 3H, phényl,1H, NH mobile); 8,05 (s, 1H, N=CH-N).

**SM :** 589(MH+).

$M_x$ : masse d'isocyanate introduit

$M_y$ : masse d'ester obtenu.

$M_z$ : masse d'acide obtenu

| Exemple comparatif | Acide obtenu | FW (Base libre) | FW (Base libre + 2 TFA) | Mz (mg) | SM (MH+) |
|---|---|---|---|---|---|
| 55 | 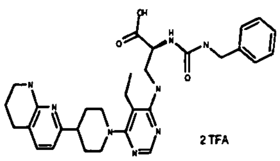 2 TFA | 558.69 | 786.69 | 48 | 559 |
| 56 | 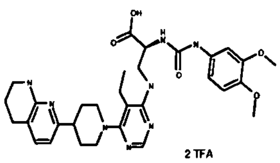 2 TFA | 604.71 | 832.71 | 51 | 605 |
| 57 | 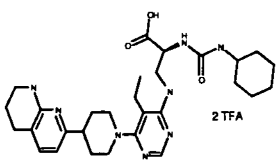 2 TFA | 550.71 | 778.71 | 51 | 551 |
| 58 | 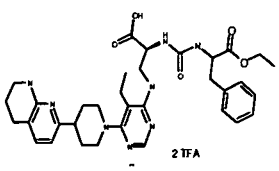 2 TFA | 644.78 | 872.78 | 49 | 645 |
| 59 | 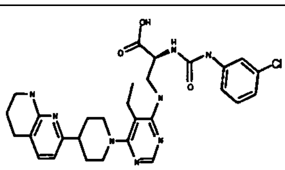 | 579.11 | 807.11 | 48 | 579 |
| 60 | 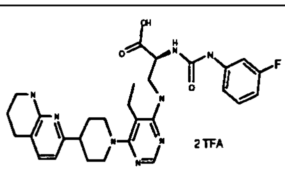 2 TFA | 562.65 | 790.65 | 50 | 563 |
| 61 | 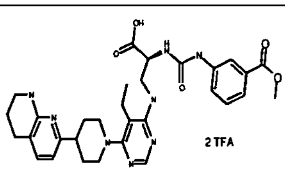 2 TFA | 602.70 | 830.70 | 43 | 603 |

(suite)

| Exemple comparatif | Acide obtenu | FW (Base libre) | FW (Base libre + 2 TFA) | Mz (mg) | SM (MH+) |
|---|---|---|---|---|---|
| 62 | 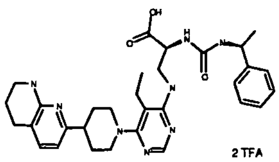 2 TFA | 572.71 | 800.71 | 29 | 573 |

Les produits obtenus ci-dessus peuvent être préparés à partir des matières premières suivantes :

| Exemple comparatif | Isocyanate | Mx (mg) | produit attendu | $M_y$ (mg) | Rdt | SM (MH+) |
|---|---|---|---|---|---|---|
| 55 | 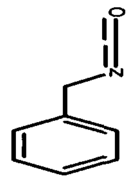 | 28.0 | 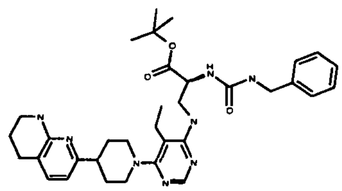 | 56.5 | 44 | 615 |
| 56 | 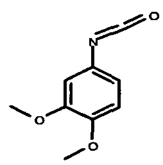 | 37.0 | 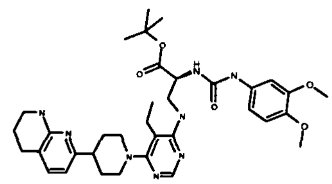 | 51.7 | 38 | 661 |
| 57 | 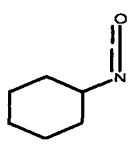 | 26.0 | 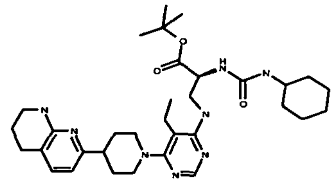 | 80 | 63 | 607 |
| 58 | 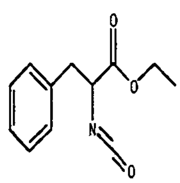 | 45.5 | 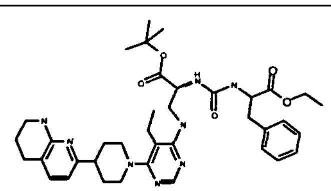 | 56 | 38 | 701 |
| 59 | 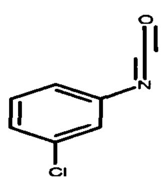 | 31.7 | 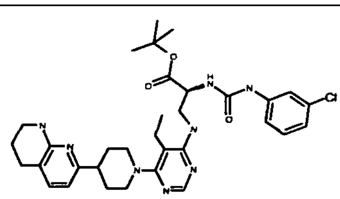 | 54.4 | 41 | 635 |
| 60 | 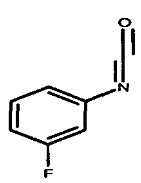 | 29.0 | 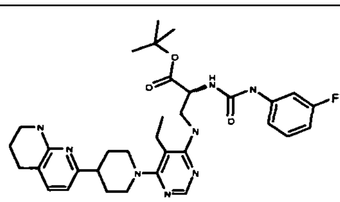 | 57.6 | 45 | 619 |

Exemple comparatif 63

Synthèse du 2-{N-[(diméthylamino)sulfonylamino]}-3-{5-éthyl-6-[4-(5,6,7,8-tétrahydro-[1,8]naphthyridin-2-yl)-pipéridin-1-yl]-pyrimidin-4-ylamino}-propionate de tert-butyle

A une solution de 2 g (4,27 mmoles) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle de dans 200 ml de tétrahydrofurane anhydre est additionné 16 ml de triéthylamine puis une solution de 700 µl (6,4 mmoles) de diméthylaminosulfonyl chloride dans 40 ml de tétrahydrofurane anhydre. L'addition se fait au goutte à goutte, à température ambiante, sous flux d'azote. Le mélange réactionnel est maintenu sous agitation pendant 48 heures. Puis, le solvant est évaporé sous pression réduite (2 kPa) et le résidu est repris avec un mélange d'eau, d'une solution saturée de bicarbonate de sodium et de l'acétate d'éthyle. La phase organique est décantée, séchée sur sulfate de magnésium et le solvant éliminé par évaporation sous pression réduite (2 kPa). Le résidu est chromatographié sur silicagel en éluant avec l'acétate d'éthyle 100%. On obtient 1,1 g (de produit attendu.
**CCM :** Rf=0,1 (silicagel, éluant : acétate d'éthyle 100%)
**1H-RMN ($CDCl_3$) :** δ 1, 48 (s, 9H, tBu) ; 1, 93 et 2, 02 (2m, 6H, N-CH2-CH2-CH-CH2-CH2, CH2-CH2-CH2-NH); 2,01 (s, 3H, CH3); 2,73 (m, 3H, N-CH2-CH2-CH-CH2-CH2, CH2-CH2-CH2-NH); 2,81 (s, 6H, N($CH3)_2$); 2,95 et 3,70 (2m, 4H, N-CH2-CH2-CH-CH2-CH2); 3,44 (m, 2H, CH2-CH2-CH2-NH); 3,81 et 3,91 et 4,11 (3m, 3H, NH-CH2-CH-NH); 4,87 (t, 1H, NH mobile); 5,86 (dl, 1H, NH mobile); 6,41 et 7,17 (2d, 2H, CH=CH naphtiridine); 8,28 (s, 1H, N=CH-N).
**SM :** 575(MH+).

Exemple comparatif 64

Synthèse de l'acide 2-{N-[(diméthylamino)sulfonylamino]}-3-{5-éthyl-6-[4-(5,6,7,8-tétrahydro-[1,8]naphthyridin-2-yl)-pipéridin-1-yl]-pyrimidin-4-ylamino}-propionique,bis (trifluoroacetate).

On agite 290 mg (0,5 mmoles) du 2-{N-[(diméthylamino)sulfonylamino]}-3-{5-éthyl-6-[4-(5,6,7,8-tétrahydro-[1,8]naphthyridin-2-yl)-pipéridin-1-yl]-pyrimidin-4-ylamino}-propionate de tert-butyle dans 30 ml de dichlorométhane avec 3 ml d'acide trifluoroacétique à température ambiante jusqu'à disparition du produit de départ en CCM (silicagel, éluant : $CH_2Cl_2$-MeOH-$H_2O$-AcOH 90-10-1-1). On rajoute du toluène et on évapore le mélange réactionnel à sec sous pression réduite (2 kPa). Le résidu est solubilisé dans le minimum de dichlorométhane avec un peu de méthanol puis versé sur de l'éther éthylique. Le précipité est filtré. On obtient 197,3 mg de produit attendu.
C**CM :** Rf=0,2 (silicagel, éluant : dichlorométhane-méthanol-eau-acide acétique 90-10-1-1).
**1H-RMN ($CDCl_3$) :** δ1,94 et 2,04 (2m, 6H, N-CH2-CH2-CH-CH2-CH2, CH2-CH2-CH2-NH); 2,11 (s, 3H, CH3); 2,74 (s, 6H, N($CH3)_2$); 2,76 (m, 2H, CH2-CH2-CH2-NH); 3,01 (m, 1H, N-CH2-CH2-CH-CH2-CH2); 3,29 et 3,96 (2m, 4H, N-CH2-CH2-CH-CH2-CH2); 3,52 (m, 2H, CH2-CH2-CH2-NH); 3,80 et 4,04 et 4,21 (3m, 3H, NH-CH2-CH-NH); 6,42 et 7,39 (2d, 2H, CH=CH naphtiridine); 7,12 (m, 1H, NH mobile); 8,33 (s, 1H, N=CH-N).
**SM :** 519(MH+).

Exemple comparatif 65

Synthèse du 3-[5-méthyl-6-[4-(5,6,7,8-tétrahydro-(1,8)naphtyridin-2-yl)-pipéridin-1-yl]-pyrimidin-4-ylamino]-N-[(1,1-diméthylthyloxycarbonyl)aminosulfonyl]-propionate de tert-butyle.

A 0,13 ml (1,5 mmoles) de chlorosulfonyl isocyanate en solution dans 30 ml de dichlorométhane on ajoute goutte à goutte 111 mg (1,5 mmoles) de terbutanol en solution dans 30 ml de dichlorométhane et on agite à température ambiante pendant 45 minutes. Cette solution est ensuite introduite goutte à goutte sur un mélange de 700 mg (1,5 mmoles) de 2-amino-3-{5-éthyl-6-[4-(5,6,7,8-tétrahydro-[1,8]naphthyridin-2-yl)-pipéridin-1-yl]-pyrimidin-4-ylamino}-propionate de tert-butyle et 0,35 ml (2 mmoles) de diisopropyléthylamine dans 400 ml de dichlorométhane. Le mélange réactionnel est agité à température ambiante pendant 2 heure. Puis, le solvant est évaporé sous pression réduite (2 kPa) Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant est évaporé sous pression réduite (2 kPa). On obtient 830 mg de produit attendu sous la forme d'une huile jaune.
**CCM :** Rf=0,12 (silicagel, éluant : dichlorométhane-méthanol-eau-acide acétique 90-10-1-1)
**1H-RMN ($CDCl_3$)** : δ 1,48 et 1,51ppm (2s, 18H, tBu); 1,85 à 2,15 (m, 6H, $CH_2$-CH-$CH_2$ et_ $CH_2$-$CH_2$-$CH_2$-NH) ; 1, 98 (s, 3H, $CCH_3$) ; 2, 85 à 3,05 (m, 3H, $CH_2$-$CH_2$-$CH_2$-NH et $CH_2$-CH-$CH_2$); 2,78 et 3,74 (tl et dl, 4H, $CH_2$-$CH_2$-N-$CH_2$-$CH_2$) ; 3,52 (m, 2H, $CH_2$-$CH_2$-$CH_2$-NH); 3,89 et 3,99 (2dt, 2H, NH-$CH_2$-CH-NH); 4,32 (dt, 1H, NH-$CH_2$-CH-NH); 4,88 (tl, 1H,

1H, NH-$CH_2$-CH-NH); 6,42 et 7,36 (2d, 2H, H naphthyridine); 8,28ppm (s, 1H, N=CH-N).
**SM** : 647 (MH+); 645 (MH-).

Synthèse du 3-[5-méthyl-6-[4-(5,6,7,8-tétrahydro-(1,8)naphtyridin-2-yl)-pipéridin-1-yl]-pyrimidin-4-ylamino]-N-[N, N-(1,1-diméthyléthyloxycarbonyl)(phénylméthyl) aminosulfonyl]-propionate de tert-butyle

A un mélange de 130 mg (0,2 mmole) de 3-[5-méthyl-6-[4-(5,6,7,8-tétrahydro-(1,8)naphtyridin-2-yl)-pipéridin-1-yl]-pyrimidin-4-ylamino]-N-[(1,1-diméthyléthyloxycarbonyl)amino-sulfonyl]-propionate de tert-butyle et de 60 mg (0,3 mmole) de triphénylphosphine en solution dans 10 ml de dichlorométhane on ajoute 0,047 ml de diéthyl azodicarboxylate puis goutte à goutte 0,041 ml (0,4 mmole) d'alcool benzyique en solution dans 5 ml de dichlorométhane et on agite à température ambiante pendant 1 heure. On ajoute alors à nouveau 0,047 ml de diéthyl azodicarboxylate puis goutte à goutte 0,041 ml (0,4 mmole) d'alcool benzyique en solution dans 5 ml de dichlorométhane et on agite à température ambiante pendant 1 heure. Puis, le solvant est évaporé sous pression réduite (2 kPa) Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant est évaporé sous pression réduite (2 kPa). Le brut est purifié par chromatographie sur silice en éluant avec un gradiant d'élution chlorure de méthylène-méthanol de 100-0 à 90-10. On obtient 80 mg de produit attendu sous la forme d'un solide beige.
**CCM :** Rf=0,12 (silicagel, éluant : dichlorométhane-méthanol-eau-acide acétique 90-10-1-1)
**1H-RMN ($CDCl_3$) :** δ 1,38 et 1,53ppm (2s, 18H, tBu); 1,85 à 2,15 (m, 6H, $CH_2$-CH-$CH_2$ et_$CH_2$-$CH_2$-$CH_2$-NH); 1,98 (s, 3H, $CCH_3$) ; 2,75 et 3,71 (td et m, 4H, $CH_2$-$CH_2$-N-$CH_2$-$CH_2$) ; 2,82 (td, 1H, $CH_2$-CH-$CH_2$); 3,05 (m, 3H, $CH_2$-$CH_2$-$CH_2$-NH); 2, 96 (dt, 2H, $CH_2$-$CH_2$-$CH_2$-NH) ; 3, 59 et 3,81 (2m, 2H, NH-$CH_2$-CH-NH) ; 4,78 (t, 1H, NH-$CH_2$-CH-NH); 4,72 et 4,96 (2d, 2H, $CH_2$-Ph) ; 6,42 et 7,33 (2d, 2H, H naphthyridine); 7,32 (m, 5H, $CH_2$-Ph) 8,26 (s, 1H, N=CH-N).
**SM :** 737 (MH+); 735 (MH-).

Synthèse de l'acide 3-[5-méthyl-6-[4-(5,6,7,8-tétrahydro-(1,8)naphtyridin-2-yl)-pipéridin-1-yl]-pyrimidin-4-ylamino]-N-[N,N-(1,1-diméthyléthyloxycarbonyl)(phénylméthyl) aminosulfonyl]-propionique, bis(trifluoroacetate).

On agite 73 mg (0,1 mmole) de 3-[5-méthyl-6-[4-(5,6,7,8-tétrahydro-(1,8)naphtyridin-2-yl)-pipéridin-1-yl]-pyrimidin-4-ylamino]-N-[N,N-(1,1diméthyléthyloxycarbonyl)(phénylméthyl) aminosulfonyl]-propionate de tert-butyle dans 5 ml de dichlorométhane avec 1 ml d'acide trifluoroacétique à température ambiante pendant 3 heures. On rajoute du toluène et évapore le mélange réactionnel à sec sous pression réduite (2 kPa). Le résidu est solubilisé dans le minimum de dichlorométhane puis versé sur de l'éther diisopropylique. Le précipité est filtré. On obtient 35 mg de produit attendu sous forme d'un solide blanc.
**SM** : 579 (MH-).

Exemple comparatif 66 Synthèse du chlorhydrate de l'acide 2-(4-méthoxy-benzènesulfonylamino)-3-{2-methoxy-5-méthyl-6-[4-(5,6,7,8-tétrahydro-[1,8]naphthyridin-2-yl)-piperidin-1-yl]-pyrimidin-4-ylamino}-propionique :

On agite 2 g (2,99 moles)de l'ester tert-butylique de l'acide 2-(4-méthoxy-benzènesulfonylamino)-3-{2-méthoxy-5-methyl-6-[4-(5,6,7,8-tétrahydro-[1,8]naphthyridin-2-yl)-piperidin-1-yl]-pyrimidin-4-ylamino}-propionique dans 20 ml d'une solution d'acide chlorhydrique 6N dans l'eau, le mélange réactionnel reste hétérogène, prise en masse rapide du chlorhydrate de l'ester formé, on ajoute alors 20 ml d'eau distillée pour améliorer la solubilité.

On laisse agiter à température ambiante pendant 20 heures avant d'ajouter 2 fois 5 ml d'acide chlorhydrique 6N dans l'eau.

Le mélange réactionnel est maintenu sous agitation pendant au total 60 heures. Puis il est concentré à sec sous pression réduite (2 kPa) en présence successivement de toluène et d'isopropanol.

Le résidu obtenu est solubilisé dans le minimum de dichlorométhane et de méthanol puis versé sur de l'éther éthylique par 2 fois. Le précipité formé est filtré, lavé à l'éther puis au pentane et séché sous vide. On obtient 1.62 g d'une poudre blanche.
**CCM** : Rf=0,41 (silicagel, éluant :dichlorométhane-méthanol 90-10)
**1H-RMN ($CDCl_3$)** : δ 0,95 (m, 7H, -CH3, CH2-CH2-CH2-CH2-CH2) ; 1,05 (m, 2H, CH2-CH2-CH2-CH2-CH2); 1,8 (m, 4H, CH2-CH2-CH-CH2-CH2); 1,92 (m, 1H, CH2-CH2-CH-CH2-CH2); 2,17 et 2,67(2m, 4H, N-CH2-CH2-CH-CH2-CH2), 2,5 (t, 2H, CH2-CH2-CH2-NH); 2,57 et 2,9 (2m, 2H, NH-CH2-CH-NH); 2,82 (s, 3H, -OCH3); 3,05 (s, 3H, -OCH3); 3,23 (m, 1H, NH-CH2-CH-NH); 5,67 et 6,6 (2d, 2H, CH=CH naphthyridine); 5,95 et 6,7 (2d, 4H, CH=CH benzène)
**SM** : 612(MH)+; 306(MH)2+; 610(MH)

Synthèse de l'ester tert-butylique de l'acide 2-(4-méthoxy-benzènesulfonylamino)-3-{2-methoxy-5-methyl-6-[4-(5,6,7,8-tetrahydro-[1,8]naphthyridin-2-yl)-piperidin-1-yl]-pyrimidin-4-ylamino}-propionique :

Etape 1

Synthèse de l'ester tert-butylique de l'acide 2-amino-3-{2-méthyl,5-méthoxy-6-[4-(5,6,7,8-tétrahydro-[1,8]naphthyridin-2-yl)-pipéridin-1-yl]-pyrimidin-4-ylamino}-propionique :

Dans un mono col contenant 5,4 g (8,5 mmoles)de 2-benzyloxycarbonylamino-3-{2-méthyl,5-methoxy-6-[4-(5,6,7,8-tetrahydro-[1,8]naphthyridin-2-yl)-piperidin-1-yl]-pyrimidin-4-ylamino}-propionique acid ter-butyl ester, on charge 500 ml d'acide acétique 100% et 500 mg d'oxyde de platine(5-10%). Ce mélange est purgé sous vide et laissé sous agitation à TA et sous pression atmosphérique d'hydrogène pendant 20 heures.

Le milieu hétérogène obtenu est filtré sur clarcel. Le filtrat est concentré à sec en présence de cyclohexane. Puis repris par un mélange d'acétate d'éthyle et d'une solution de bicarbonate de sodium saturée. La phase organique est extraite et séchée sur sulfate de magnésium puis le solvant est évaporé sous vide. Le résidu obtenu est chromatographié sur silicagel avec l'éluant suivant : dichlorométhane-heptane 50-50 jusqu'à (dichlorométhane-méthanol 90-10 / Acétate d'éthyle) (50-50). On obtient 2,75 g de produit attendu sous la forme de cristaux jaune pâle (rendement = 65%).
**CCM** : Rf=0.32, éluant : (dichlorométhane-méthanol 90-10 / Acétate d'éthyle) (50-50) sur silice
**1H-RMN (CDCl3)** : δ 1,47 (s, 9H, tBu); 1,93 (m, 9H, N-CH2-<u>CH2</u>-CH-<u>CH2</u>-CH2, CH2-<u>CH2</u>-CH2-NH, <u>CH3-</u>); 2,7 (m, 3H, N-CH2-CH2-<u>CH</u>-CH2-CH2, <u>CH2</u>-CH2-CH2-NH) ; 2,92 et 3,72 (2m, 4H, CH2-<u>CH2</u>-N-<u>CH2</u>-CH2-CH); 3,42 (m, 2H, CH2-CH2-<u>CH2</u>-NH); 3,5 (m, 2H, NH-<u>CH2</u>-CH-NH); 3,65 (m, 1H, NH-CH2-<u>CH</u>-NH); 3,9 (s, 3H, -N=N-O<u>CH3</u>);5,0 et 5,65 (m, 2H, NH mobile); 6,4 et 7,13 (2d, 2H, <u>CH</u>=<u>CH</u> naphthyridine)
**SM** : 498 (MH) +

Etape 2

A un mélange de 2,2 g (4,43 mmoles) de 2-amino-3-[2-méthoxy-5-methyl-6-[4-(5,6,7,8-tetrahydro-[1,8] naphthyridin-2-yl)piperidin-1-yl)-pyrimidin-4-ylamino]-propionic acid tert-butyl ester en solution dans 800 ml de tétrahydrofurane et 950 μl de triéthylamine, on additionne goutte à goutte 915 mg (4,43 mmoles)de chlorure de 4-méthoxybenzènesulfonyle en solution dans 200 ml de tétrahydrofurane. Le milieu réactionnel est agité à température ambiante pendant une nuit. Puis, le solvant est évaporé sous pression réduite (2 kPa) et le résidu est chromatographié sur alumine avec l'éluant suivant : acétate d'éthyle-dichlorométhane 50-50 à acétate d'éthyle-(dichlorométhane / méthanol)(90/10) 50-50.

On obtient 2,02 g (Rdt=68%) de produit attendu.
**CCM :** Rf=0,19 (silicagel, éluant : Acétate d'éthyle 100%)
**1H-RMN ($CDCl_3$) :** δ 1,3 (s, 9H, tBu); 1,95 (m, 9H, N-CH2-<u>CH2</u>-CH-<u>CH2-CH2,</u> CH2-<u>CH2</u>-CH2-NH, -<u>CH3</u>); 2,75 (m, 3H, N-CH2-CH2-<u>CH</u>-CH2-CH2, <u>CH2</u>-CH2-CH2-NH); 2,95 et 3,75 (2m, 4H, N-<u>CH2</u>-CH2-CH-CH2-<u>CH2)</u>; 3,47 (m, 2H, CH2-CH2-<u>CH2</u>-NH); 3,6 (m, 1H, NH-CH2-<u>CH</u>-NH); 3,85 (s, 3H, N=C(O<u>CH3</u>)-N); 3,9 (s, 3H, Ph-<u>OCH3</u>); 3,97 (m, 2H, NH-<u>CH2</u>-CH-NH); 4,85 (m, H, <u>NH</u> mobile); 5,8 (dl, 1H, NH mobile); 6.42 et 7,22 (2d, 2H, CH=CH naphthyridine); 6,92 et 7,75 (2d, 4H, CH=CH benzène)
SM : 668(MH)+; 334(MH)2+

Exemple 67

Synthèse du chlorhydrate de l'acide 2-(4-méthoxybenzènesulfonylamino)-3-{2-methyl-5-methyl-6-[4-(5,6,7,8-tétrahydro-[1,8]naphthyridin-2-yl)-pipéridin-1-yl]-pyrimidin-4-ylamino}-propionique :

On agite 3 g (4,6 moles) de l'ester tert-butylique de l'acide 2-(4-méthoxy-benzènesulfonylamino)-3-{(2,5)-diméthyl-6-[4-(5,6,7,8-tétrahydro-[1,8]naphthyridin-2-yl)-pipéridin-1-yl]-pyrimidin-4-ylamino}-propionique dans 30 ml d'une solution d'acide chlorhydrique 6N dans l'eau, le mélange réactionnel reste hétérogène, prise en masse rapide du chlorhydrate de l'ester formé, on ajoute alors 20 ml d'acide chlorhydrique 6N d'eau, le mélange redevient limpide.

On laisse agiter à température ambiante pendant 4 heures, le mélange redevient laiteux.

Puis celui-ci est concentré à sec sous pression réduite (2 kPa) en présence successivement de toluène et d'isopropanol.

Le résidu obtenu est solubilisé dans le minimum de dichlorométhane et de méthanol puis versé sur de l'éther éthylique. Le précipité formé est filtré, lavé à l'éther puis au pentane et séché sous vide. On obtient 2,86 g d'une poudre blanche.
**CCM :** Rf=0,23 (silicagel, éluant :dichlorométhane-méthanol- eau-acide acétique 85-15-2-2)

**1H-RMN (MeOD) :** δ 1,05 (m, 9H, -CH3, N-CH2-CH2-CH-CH2-CH2, CH2-CH2-CH2-NH); 1,57 (s, 3H, -CH3); 1,85 (t, 2H, CH2-CH2-CH2-NH); 2,02 (m, 1H, N-CH2-CH2-CH-CH2-CH2); 2,32 et 2,8 (2m, 4H, N-CH2-CH2-CH-CH2-CH2); 2,55 (m, 2H, CH2-CH2-CH2-NH); 2,52 et 2,95 (2m, 2H, NH-CH2-CH-NH); 2,87 (s, 3H, -OCH3); 3,23 (m, 1H, NH-CH2-CH-NH); 5,72 et 6,65 (2d, 2H, CH=CH naphthyridine); 6,02 et 6,75 (2d, 4H, CH=CH benzène)
**SM** : 596(MH)+; 594(MH)-

Synthèse de l'ester tert-butylique de l'acide 2-(4-methoxybenzenesulfonylamino)-3-{(2,5)-diméthyl-6-[4-(5,6,7,8-tetrahydro-[1,8]naphthyridin-2-yl)-piperidin-1-yl]-pyrimidin-4-ylamino}-propionique :

Etape 1

Synthèse de l'ester tert-butylique de l'acide 2-amino-3-{2,5-diméthyl-6-[4-(5,6,7,8-tétrahydro-[1,8]naphthyridin-2-yl)-pipéridin-1-yl]-pyrimidin-4-ylamino}-propionique

Dans un mono col contenant 13 g (21,1 mmoles) de 2-benzyloxycarbonylamino-3-{2,5-diméthyl-6-[4-(5,6,7,8-tétrahydro-[1,8]naphthyridin-2-yl)-pipéridin-1-yl]-pyrimidin-4-ylamino}-propionique acid ter-butyl ester, préparé selon la demande internationale (WO 2004 048375(A1))on charge 1 litre d'acide acétique 100% et 1 g d'oxyde de platine (5-10%). Ce mélange est purgé sous vide et laissé sous agitation à température ambiante et sous pression atmosphérique d'hydrogène pendant 20 heures.

Le milieu hétérogène obtenu est filtré sur clarcel. Le filtrat est concentré à sec en présence de cyclohexane. Puis repris par un mélange d'acétate d'éthyle et d'une solution de bicarbonate de sodium saturée. La phase organique est extraite et séchée sur sulfate de magnésium puis le solvant est évaporé sous vide. Le résidu obtenu est chromatographié sur silicagel avec l'éluant suivant : dichlorométhane 100% jusqu'à dichlorométhane-méthanol 95-5. On obtient 7,6 g de produit attendu sous la forme de cristaux jaune pâle (rendement=74%).
**CCM** : Rf=0.23 (éluant : dichlorométhane-méthanol 95-5 sur silice)
**1H-RMN ($CDCl_3$) :** δ 1,47 (s, 9H, tBu) ; 1,92 (m, 6H, CH2-CH2-CH2-NH, CH2-CH2-CH-CH2-CH2); 2,05 (s, 3H, -CH3); 2,42 (s, 3H, N=N-CH3); 2,72 (t, 2H, CH2-CH2-CH2-NH); 2,85 (m, 1H, N-CH2-CH2-CH-CH2-CH2); 2,97 et 3,65 (2m, 4H, CH2-CH2-N-CH2-CH2-CH); 3,47 (m, 2H, CH2-CH2-CH2-NH); 3,5 et 3,7 (2m, 2H, NH-CH2-CH-NH); 3,9 (m, 1H, NH-CH2-CH-NH) ; 4,97 et 5,55 (m, 2H, NH mobile); 6,37 et 7,25 (2d, 2H, CH=CH naphthyridine)
**SM** : 482 (MH)+; 426 $[MH - tBu]^+$

Etape 2

A un mélange de 750 mg (1,56 mmoles) de 2-Amino-3-[(2,5)-dimethyl-6-[4-(5,6,7,8-tetrahydro-[1,8]naphthyridin-2-yl)-pyrimidin-4-ylamino]-propionic acid tert-butyl ester en solution dans 350 ml de tétrahydrofurane et 7 ml de triéthylamine, on additionne goutte à goutte 322 mg (1,56 mmoles)de chlorure de 4-méthoxy-benzènesulfonyle en solution dans 35 ml de tétrahydrofurane. Le milieu réactionnel est agité à température ambiante pendant une nuit. Puis, le solvant est évaporé sous pression réduite (2 kPa) et le résidu est chromatographié sur alumine avec l'éluant suivant : acétate d'éthyle-dichlorométhane 50-50 à acétate d'éthyle-méthanol)(98/2).

On obtient 2,02 g (Rdt=68%) de produit attendu.
**CCM :** Rf=0,25 (silicagel, éluant : Acétate d'éthyle-méthanol (90/10)
**1H-RMN ($CDCl_3$) :** δ 1,32 (s, 9H, tBu) ; 1,9 (m, 6H, N-CH2-CH2-CH-CH2-CH2, CH2-CH2-CH2-NH); 2,05 (s,3H, -CH3); 2,45(s, 3H, -CH3); 2,75 (m, 3H, N-CH2-CH2-CH-CH2-CH2, CH2-CH2-CH2-NH); 2,95 et 3,7 (2m, 4H, N-CH2-CH2-CH-CH2-CH2); 3,47 (m, 2H, CH2-CH2-CH2-NH); 3,8 (m, 2H, NH-CH2-CH-NH); 3,85(s, 3H, Ph-OCH3); 3,95 (m, 1H, NH-CH2-CH-NH); 4,65 et 7,1 (2m, 2H, NH mobile); 6.42 et 7,25 (2d, 2H, CH=CH naphthyridine); 6,92 et 7,75 (2d, 4H, CH=CH benzène)
**SM** : 652(MH)+; 650(MH)-

Exemples comparatifs 68 à 80

Etape a : activation de l'alcool

1,5 équivalents de di(N-succinimidyl)carbonate est mis en solution dans 4 ml de chlorure de méthylène. On ajoute à température ambiante un équivalent d'alcool mis en solution dans du chlorure de méthylène, ainsi que 2 équivalents de triéthylamine. Le mélange réactionnel est laissé sous agitation pendant 6 heures.

Puis, si l'alcool activé formé est stable, il peut être isolé par extraction à l'acétate d'éthyle et lavage au bicarbonate de sodium avant addition sur l'amine.

Etape b : addition de l'amine

On ajoute à l'alcool activé 0,8 équivalent de ter-butyl ester de l'acide 2-amino-3-{2,5-diméthyl-6-[4-(5,6,7,8-tétrahydro-[1,8]naphthyridin-2-yl)-pipéridin-1-yl]-pyrimidin-4-ylamino}-propionique, ainsi qu'un équivalent de triéthylamine.

Le mélange est laissé sous agitation pendant une nuit à température ambiante.

Le mélange réactionnel est ensuite extrait à l'acétate d'éthyle après lavage par une solution de bicarbonate de sodium saturée. La phase organique obtenue est séchée sur du sulfate de magnésium avant d'être concentrée à sec sous pression réduite (2 kPa). Le résidu est ensuite chromatographié sur alumine avec l'éluant suivant : Acétate d'éthyle/éther isopropylique jusqu'à acétate d'éthyle 100%. On obtient l'ester attendu avec un rendement de 38 à 90%.

Etape c : hydrolyse de l'ester

On agite une masse $m_c$ de 3-[[2,5-diméthyl-6-[4-(1,2,3,4-tétrahydro-1,8-naphthyridin-7-yl)-1-pipéridinyl]-4-pyrimidinyl]amino]-N-(alkyl)alaninate de (1,1-diméthyléthyle) dans 5 ml de dichlorométhane et 500 µl d'acide trifluoroacétique à température ambiante jusqu'à disparition du produit de départ en CCM (silicagel, éluant : $CH_2Cl_2$-MeOH-$H_2O$-AcOH 90-10-1-1). Puis on ajoute du toluène au mélange réactionnel pour évaporation à sec sous pression réduite (2 kPa). Le résidu obtenu est solubilisé dans un minimum de dichlorométhane puis versé sur de l'éther diisopropylique. Le précipité est filtré, lavé au pentane puis séché. On obtient une masse $m_D$ d'acide attendu sous forme de poudre.

| Exemple comparatif | alcool utilisé | Produit attendu | FW | Rendement | SM [MZ+H] |
|---|---|---|---|---|---|
| 68 | 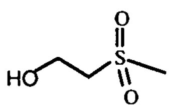 | 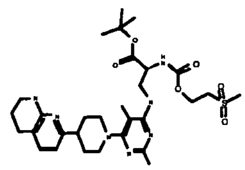 | 631.8 | 38 | 632 |
| 69 | 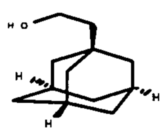 | 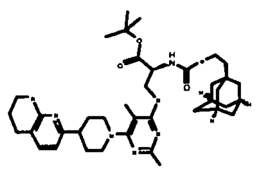 | 673.91 | 51 | 674 |
| 70 | 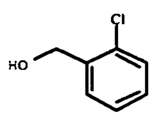 | 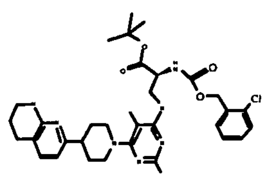 | 650.23 | 49 | 650 |
| 71 | 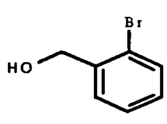 | 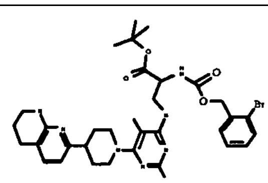 | 694.68 | 59 | 694 |
| 72 | 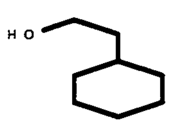 | 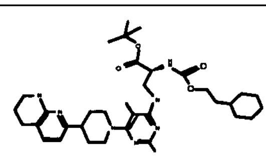 | 635.86 | 46 | 636 |
| 73 | 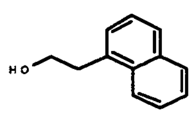 | 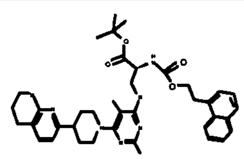 | 679.87 | 59 | 680 |

(suite)

| Exemples comparatif | Alcool utilisé | Produit attendu | FW | Rendement | SM [M/Z+H] |
|---|---|---|---|---|---|
| 74 | 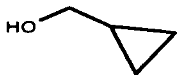 | 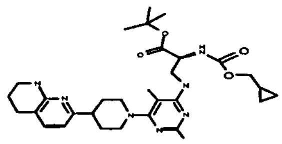 | 579.75 | 64 | 580 |
| 75 | 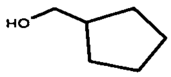 | 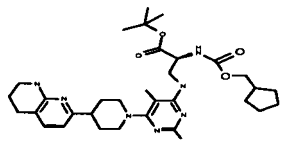 | 607.8 | 66 | 608 |
| 76 | 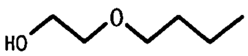 | 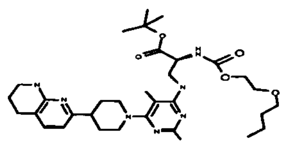 | 625.82 | 54 | 626 |
| 77 | 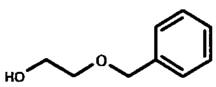 | 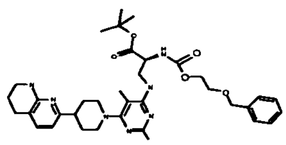 | 659.84 | 48 | 660 |
| 78 | 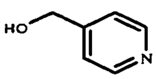 | 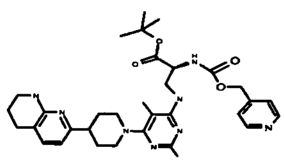 | 616.77 | 90 | 617 |
| 79 | 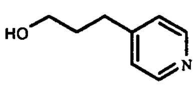 | 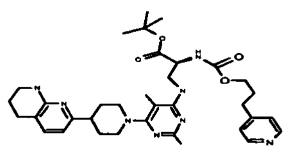 | 644.82 | 46 | 645 |
| 80 | 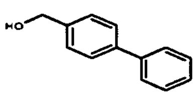 | 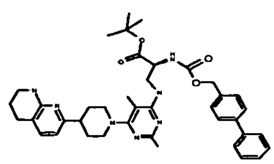 | 691.88 | 50 | 692 |

| Exemple comparatif | Ester de départ | $m_C$ (mg) | Acide attendu | FW (base libre) | $m_D$ (mg) | SM $[M/Z+H]^+$ |
|---|---|---|---|---|---|---|
| 68 | 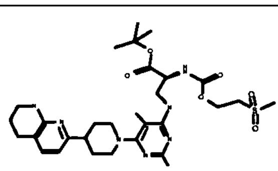 | 250 | $nCF_3COOH$ 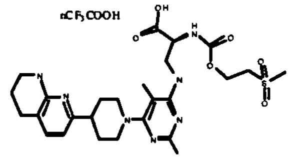 | 575.69 | 210 | 576 |

(suite)

| Exemple comparatif | Ester de départ | $m_c$ (mg) | Acide attendu | FW (base libre) | $m_D$ (mg) | SM $[M/Z+H]^+$ |
|---|---|---|---|---|---|---|
| 69 | 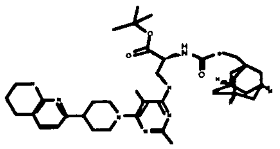 | 320 | $nCF_3COOH$ 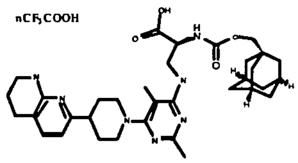 | 617.84 | 320 | 618 |
| 70 | 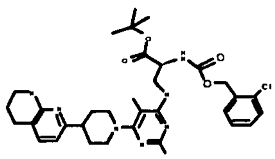 | 320 | $nCF_3COOH$ 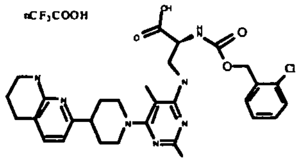 | 594.12 | 420 | 594, 596 |
| 71 | 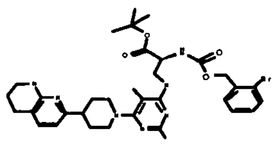 | 430 | $nCF_3COOH$ 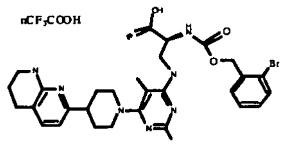 | 638.57 | 480 | 639 |
| 72 | 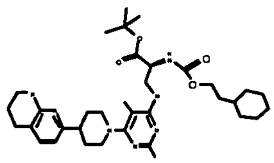 | 330 | $nCF_3COOH$ 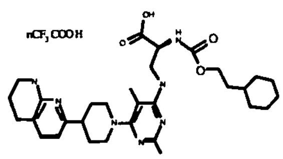 | 579.75 | 450 | 580 |
| 73 | 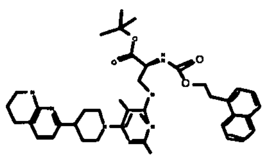 | 410 | $nCF_3COOH$ 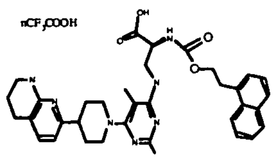 | 623.76 | 510 | 624 |
| 74 | 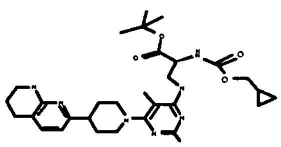 | 390 | $nCF_3COOH$ 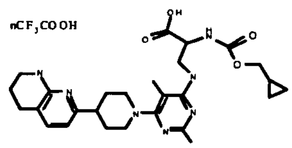 | 523.64 | 610 | 524 |
| 75 | 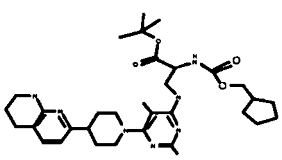 | 500 | $nCF_3COOH$ 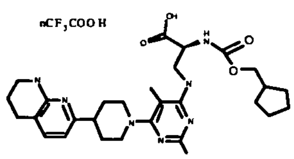 | 551.69 | 720 | 552 |
| 76 | 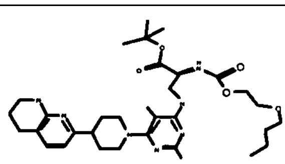 | 400 | $nCF_3COOH$ 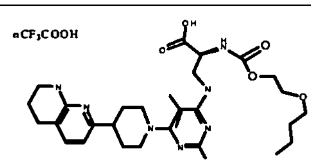 | 569.71 | 610 | 570 |
| 77 | 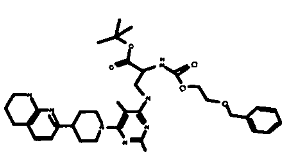 | 310 | $nCF_3COOH$ 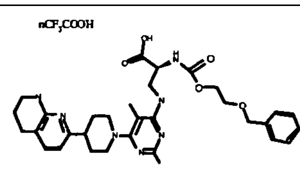 | 603.73 | 490 | 604 |

(suite)

| Exemple comparatif | Ester de départ | $m_c$ (mg) | Acide attendu | FW (base libre) | $m_D$ (mg) | SM $[M/Z+H]^+$ |
|---|---|---|---|---|---|---|
| 78 | 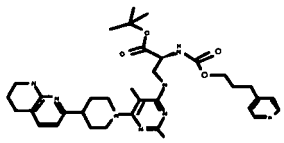 | 300 | $nCF_3COOH$ 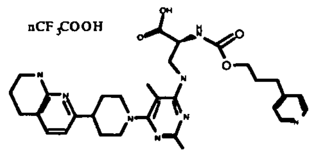 | 588.72 | 243 | 294 (Z=2) |
| 79 | 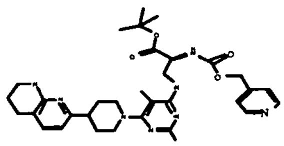 | 400 | $nCF_3COOH$ 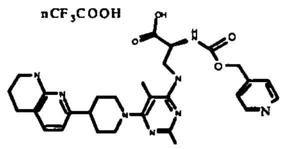 | 560.66 | 400 | 561 |
| 80 | 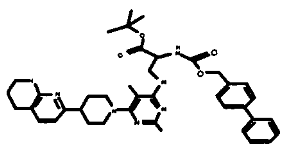 | 260 | $nCF_3COOH$ 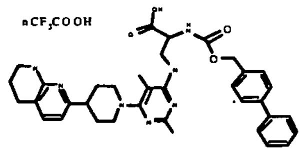 | 635.77 | 220 | 636 |

Exemple comparatif 81

Synthèse du tert-butyl ester de l'acide 2-cyclohexylméthoxycarbonylamino-3-{2,5-diméthyl-6-[4-(5,6,7,8-tétrahydro-[1,8]naphthyridin-2-yl)-pipéridin-1-yl]-pyrimidin-4-ylamino}-propionique

Etape a :

Synthèse de l'alcool activé N-succinimidyl carbonic acid cyclohexylmethyl ester

5.38 g (10.5 mmoles)de di(N-succinimidyl)carbonate est mis en solution dans 60 ml de chlorure de méthylène. On ajoute à température ambiante 2,16 ml (17,55 mmoles) de cyclohexylméthanol mis en solution dans 10 ml de chlorure de méthylène, et 4,92 ml (35,1 mmoles) de triéthylamine. Le mélange réactionnel est laissé sous agitation pendant 1 nuit.

Puis, le mélange réactionnel est concentré à sec avant extraction à l'acétate d'éthyle et lavage par une solution de bicarbonate de sodium saturée. La phase organique obtenue est séchée sur du sulfate de magnésium avant d'être concentrée à sec sous pression réduite (2 kPa). On obtient 4,4 g d'alcool activé qui est utilisé tel quel dans l'étape suivante (rendement quantitatif).

**CCM :** Rf=0,84 (silicagel, éluant : Acétate d'éthyle - triéthylamine (90-10).

**1H-RMN (CDCl3) :** δ 1,02 (m, 2H, CH2-CH2-CH2-CH2-CH2); 1,25 (m, 4H, CH2-CH2-CH2-CH2-CH2); 1,75 (m, 5H, CH2-CH2-CH-CH2-CH2 et CH2-CH2-CH-CH2-CH2);
2,85 (s, 4H, CO-CH2-CH2-CO); 4,15 (d, 2H, OCOO-CH2-cyclohexyl)

Etape b :

On ajoute à 4,35 g (17 mmoles) d'alcool activé en solution dans 250 ml de chlorure de méthylène, 8,8 g (17 mmoles) de 2-amino-3-{2,5-diméthyl-6-[4-(5,6,7,8-tétrahydro-[1,8]naphthyridin-2-yl)-pipéridin-1-yl]-pyrimidin-4-ylamino}-propionique acid ter-butyl ester, ainsi que 2,47 ml (17 mmoles) de triéthylamine.

Le mélange est laissé sous agitation pendant une nuit à température ambiante.

Le mélange réactionnel est ensuite extrait à l'acétate d'éthyle après lavage par une solution de bicarbonate de sodium saturée. La phase organique obtenue est séchée sur du sulfate de magnésium avant d'être concentrée à sec sous pression réduite (2 kPa). Le résidu obtenu est ensuite chromatographié sur silicagel avec l'éluant suivant : acétate d'éthyle/heptane jusqu'à acétate d'éthyle 100%. On obtient 6,9 g de produit attendu (rendement = 65%).

**CCM :** (éluant : acétate d'éthyle- dichlorométhane 50-50 sur alumine, et acétate d'éthyle-heptane 80-20 sur silice).

**CCM :** Rf=0.23 (éluant : acétate d'éthyle-heptane (60/40)

**1H-RMN (MeOD) :** δ 0,23 (m, 2H, CH2-CH2-CH2-CH2-CH2) ; 0,5 (m, 4H, CH2-CH2-CH2-CH2-CH2); 0,65 (s, 9H, tBu); 0,82 (m, 1H, CH2-CH2-CH-CH2-CH2); 0,95 (m, 4H, CH2-CH2-CH-CH2-CH2); 1,1 (m, 6H, N-CH2-CH2-CH-CH2-CH2, CH2-CH2-CH2-NH); 1,2 (s, 3H, CH3-); 1,62 (s, 3H, N=C(CH3)-N); 1,82 (m, 1H, N-CH2-CH2-CH-CH2-CH2); 1,95 (t,

2H, CH2-CH2-CH2-NH) ; 2,65 (m, 2H, CH2-CH2-CH2-NH) ; 2,12 et 2,85 (2m, 4H, N-CH2-CH2-CH-CH2-CH2); 3,05 (m, 2H, NH-CH2-CH-NH, 2H, NH-CO-CH2-cyclohexyl); 3,5 (m, 1H, NH-CH2-CH-NH); 5,65 et 6,4 (2d, 2H, CH=CH naphthyridine)
**SM** : 622 (MH)+, 283 (MH-tBu)2+, 426 (MH-tBu-COOcyclohexyl)+.

Exemple comparatif 82

Synthèse du chlorhydrate de l'acide 2-Cyclohexylmethoxycarbonylamino-3-{2,5-dimethyl-6-[4-(5,6,7,8-tetrahydro-[1,8] naphthyridin-2-yl)-piperidin-1-yl]-pyrimidin-4-ylamino}-propionique :

On agite 28 g (0,045 moles) de tert-butyl ester de l'acide 2-cyclohexylméthoxycarbonylamino-3-{2,5-diméthyl-6-[4-(5,6,7,8-tétrahydro-[1,8]naphthyridin-2-yl)-pipéridin-1-yl]-pyrimidin-4-ylamino}-propionique dans 76 ml d'une solution d'acide chlorhydrique 6N dans l'eau, à température ambiante pendant 20 heures.
Le mélange réactionnel est concentré à sec sous pression réduite (2 kPa) en présence successivement de toluène et d'isopropanol.
Le résidu obtenu est solubilisé dans le minimum de dichlorométhane puis versé sur de 1 éther éthylique. Le précipité formé est filtré, lavé à l'éther puis au pentane et séché sous vide. On obtient 29 g d'une poudre blanche.
**CCM :** Rf=0,24 (silicagel, éluant : dichlorométhane-méthanol-eau-acide acétique 90-10-1-1).
**1H-RMN (CDCl3)** : δ 0,93 (m, 2H, CH2-CH2-CH2-CH2-CH2); 1,25 (m, 4H, CH2-CH2-CH2-CH2-CH2); 1,6 (m, 1H, CH2-CH2-CH-CH2-CH2); 1,7 (m, 4H, CH2-CH2-CH-CH2-CH2); 1,1 (m, 9H, N-CH2-CH2-CH-CH2-CH2, CH2-CH2-CH2-NH, CH3-); 2,55 (s, 3H, N=C(CH3)-N); 2,77 (m, 1H, N-CH2-CH2-CH-CH2-CH2); 2,97 (t, 2H, CH2-CH2-CH2-NH) ; 2, 52 et 3,97 (2m, 4H, N-CH2-CH2-CH-CH2-CH2); 3,5 (m, 2H, CH2-CH2-CH2-NH); 3,7 et 3,97 (2m, 2H, NH-CH2-CH-NH) ; 3,87 (m, 2H, NH-CO-CH2-cyclohexyl); 4,35 (m, 1H, NH-CH2-CH-NH); 6,3 (m, 1H, NH mobile); 6,4 et 7,37 (2d, 2H, CH=CH naphthyridine); 10,15 (m, 1H, COOH mobile).
**SM** : 566(MH)+; 283(MH)2+; 564(MH)-; 426(MH-COOcyclohexyl)+.

Exemple comparatif 83

Synthèse du chlorhydrate de l'acide 2-cyclohexylméthoxycarbonylamino-3-{2,5-dimethyl-6-[4-(5,6,7,8-tetrahydro-[1,8] naphthyridin-2-yl)-piperidin-1-yl]-pyrimidin-4-ylamino}-propionique tert-butyl ester

On agite 27,2 g (43,8 mmoles) de 2-cyclohexylmethoxycarbonylamino-3-{2,5-dimethyl-6-[4-(5,6,7,8-tetrahydro-[1,8]naphthyridin-2-yl)-piperidin-1-yl]-pyrimidin-4-ylamino}-propionic acid tert-butyl ester dans 240 ml d'un mélange 50/50 de dichlorométhane et d'éther éthylique, puis on ajoute lentement un mélange composé de 21.9 ml d'acide chlorhydrique 2N dans l'éther et de 20 ml d'éther éthylique.
Le mélange réactionnel doit resté limpide lors de l'addition.
Le solvant est ensuite partiellement évaporé sous pression réduite (2 kPa), la solution contenant un minimum de solvant, est versé sur 1 litre d'éther isopropylique puis sur 500 ml de pentane, le précipité formé est filtré puis séché sous vide.
On obtient 28,5 g d'une poudre blanche.
**1H-RMN ($CDCl_3$)** : δ 0,95 (m, 2H, CH2-CH2-CH2-CH2-CH2); 1,23 (m, 4H, CH2-CH2-CH2-CH2-CH2); 1,47 (s, 9H, tBu); 1,67 (m, 5H, CH2-CH2-CH-CH2-CH2, CH2-CH2-CH-CH2-CH2); 2,02 (m, 9H, N-CH2-CH2-CH-CH2-CH2, CH2-CH2-CH2-NH, CH3-); 2,65 et 3,75 (2m, 4H, N-CH2-CH2-CH-CH2-CH2) ; 2,77 (t, 2H, CH2-CH2-CH2-NH); 2,95 (m, 1H, N-CH2-CH2-CH-CH2-CH2); 3,52 (m, 2H, CH2-CH2-CH2-NH); 3,8 (m, 2H, NH-CH2-CH-NH, 2H, NH-CO-CH2-cyclohexyl); 4,4 (m, 1H, NH-CH2-CH-NH); 6,47 et 7,37 (2d, 2H, CH=CH naphthyridine); 8,8 (m, 1H, NH mobile)
**SM** : 622(MH)+, 283(MH-tBu)2+.

**Test pharmacologique : Test ELISA Kistrine/Récepteur Vitronectine ( v 3)**

Protocole :

Des plaques 96 puits MaxiSorp sont coatées une nuit à 40°C avec 100 μl de Kistrine à 1 μg/ml (dilution en tampon de coating : carbonate 0,05 M/NaOH pH 9,6). Le lendemain, les puits sont vidés et les ligands (kistrine) sont ensuite fixés (tampons de fixation : PBS contenant 0,5% de BSA (pH = 7,4)) pendant 1 heure à température ambiante avec une agitation douce de 125 rpm. Les puits sont lavés six fois (tampon de lavage : PBS contenant 0,05% de Tween 20 (pH 7,7) puis on ajoute par puits et dans cet ordre :

- 40 μl de tampon d'incubation

- 10 µl de la dilution du produit à tester (les produits sont dilués dans un mélange 50:50 DMSO/Eau)
- 50 µl de récepteur $\alpha_v\square_3$ humain (cf Pytella et al. Methods Enzymol. (1987) 144 (Dilution en tampon d'incubation, à adapter suivant le lot de récepteur et selon le ligand). Le ligand, le récepteur $\alpha_v\square_3$ et les produits à étudier sont co-incubés pendant 3 heures à température ambiante avec une agitation douce de 125 rpm.

Les puits sont à nouveau lavés six fois, puis incubés pendant 2 heures à température ambiante avec une agitation douce de 125 rpm, en présence de 100 µl d'anticorps anti-récepteur couplé à une peroxydase (L'anticorps 4B12-HRP est dilué en tampon d'incubation (50 mM TRIS pH 7,4; 0,5% BSA; 0,05% Tween 20; 1 mM $MnCl_2$; 50 µM $CaCl_2$; 50 µM $MgCl_2$; 100 mM NaCl). La dilution est à adapter suivant le lot de récepteur.
Les puits sont ensuite lavés six fois avant la mesure de la liaison ligand-récepteur faite par l'intermédiaire d'un kit révélateur de peroxydase (TBM Microwell Peroxidase Substrate System Kirkegaard; Ref cat 50-76-00).
Ce kit contient un flacon A de substrat (3,3',5,5'-tétraméthylebenzidine à 0,4 g/l) et un flacon B ($H_2O_2$ à 0,02% en tampon Citrate/Acide citrique). Extemporanément, un volume de A est mélangé à un volume de B, puis le mélange réactionnel est distribué à raison de 100 µl/puits.
La réaction enzymatique se développe entre 6 à 10 minutes pour Kistrine/$\alpha_v\square_3$ puis son évolution est stoppée par l'addition de 100 µl d'acide phosphorique 1M. La densité ) optique est déterminée à 450 nm.

Expression des résultats

On trace la courbe suivante : le pourcentage de liaison en fonction du logarithme de chaque concentration du produit testé.
Pour chaque produit, on détermine l'IC50 suivant la formule suivante : IC50=(B0+ Bmin)/2
B0=Maximum de liaison en l'absence de tout produit
Bmin=Minimum de liaison en présence de la concentration la plus élevée de produit.

**EXEMPLE K/VnR $IC_{50}$ (nM)**

**COMPARATIF**

1 à 65 2 à 10000

Activité in vivo

**Hypercalcémie induite par l'hormone parathyroïdienne (PTH) dans un modèle de rat thyroparathyroïdectomisés (TPXT)**

La stimulation de la résorption osseuse est induite chez des rats TPXT par perfusion de PTH et les variations de la résorption osseuse sont suivies par la concentration en calcium dans le sérum.
Des rats mâles Sprague Dawley pesant 150-200 g sont thyroparathyroïdectomisés. Les rats sont soumis à un régime standard contenant 7 g Ca/kg (UAR) et de l'eau de Volvic. L'efficacité de la thyroparathyroïdectomie est testée en ) mesurant les concentrations en Ca dans le sérum 8 jours après l'opération chez des animaux à jeun depuis la veille. Les rats sont considérés comme thyroparathyroïdectomisés lorsque les taux de Ca dans le sérum sont inférieurs à 80 mg/l. La PTH (1-34) de rat (Bachem) est dissoute dans 0,15M de NaCl Cys.HCl 2% et délivré par des minipompes osmotiques (ALZET 2001D) à la dose de 200 pmol/kg/h. Les minipompes sont introduites dans les cavités intrapéritonéales sous anesthésie (kétamine - 75 mg/kg et acépromazine - 2,5 mg/kg) chez des rats TPXT à jeun depuis la veille. Les rats TPXT contrôles reçoivent les minipompes remplies avec le véhicule de la PTH.
Soit le produit à tester soit le véhicule (contrôles et rats traités par la PTH) sont administrés 2 fois par voie sous-cutanée (2 ml/kg de poids corporel) aux temps 0 et 3 h après le début de l'infusion de PTH. Le test est poursuivi pendant les 6 heures suivantes. A la fin du traitement, la totalité du sang est collectée après décapitation. Les échantillons de sang sont centrifugés à 3000 tpm pendant 15 mn (CR422 Jouan) pour obtenir le sérum.
Les concentrations totales de Ca dans le sérum sont mesurées par colorimétrie (Ciba-Corning) en utilisant un système de lecture de microplaques IEMS Labsystems, à 540 nm.
La différence entre les valeurs moyennes de calcémie des rats traités et des groupes contrôles est analysée en variance et par le test de Dunnett.
L'activité d'un produit est calculée par la formule suivante

$$\text{Effet \%} = \frac{\text{Calcémie (produit) - calcémie (PTH)}}{\text{Calcémie (PTH) - calcémie (contrôle)}} \times 100$$

Les produits comparatifs et le produit selon l'invention testés dans cette méthode décrite se sont montrés actifs chez le rat à des doses allant de 2 fois 1 mg/kg à 2 fois 10 mg/kg par voie sous cutanée, et à des doses allant de 2 fois 3 mg/kg à 2 fois 30 mg/kg par voie orale.

## Revendications

1. Le dérivé de pyrimidine de formule (I') :

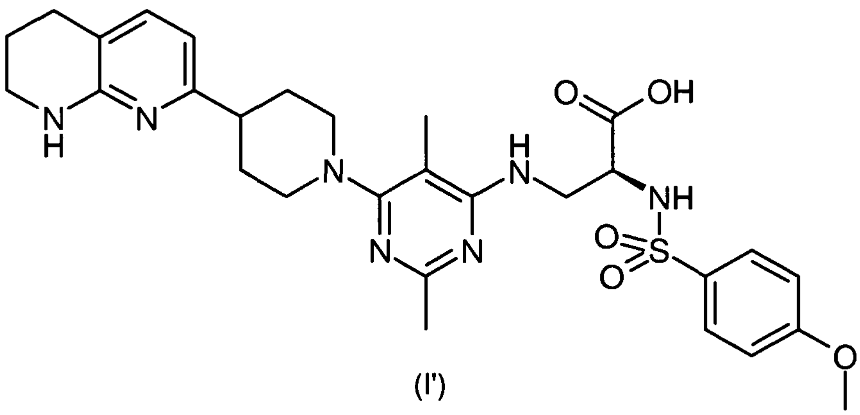

(I')

ainsi que les sels d'addition physiologiquement acceptables et les solvates de ce composé.

2. A titre de médicament, le composé de formule (I') et/ou ses sels physiologiquement acceptables.

3. Une composition pharmaceutique comprenant un médicament tel que défini à la revendication 2 à l'état pur ou en présence d'un ou plusieurs excipients.

4. A titre de médicament, selon la revendication 2, ayant une activité antagoniste du récepteur de la vitronectine, le composé de formule (I') et/ou ses sels physiologiquement acceptables.

5. A titre de médicament, selon la revendication 2, ayant une activité inhibitrice de la résorption osseuse ou pour le traitement ou la prévention de l'ostéoporose, le composé de formule (I') et/ou ses sels physiologiquement acceptables.

6. A titre de médicament, selon la revendication 2, ayant une activité inhibitrice de la croissance tumorale ou des métastases cancéreuses, le composé de formule (I') et/ou ses sels physiologiquement acceptables.

7. A titre de médicament, selon la revendication 2, ayant une activité anti-inflammatoire ou pour le traitement ou la prévention des désordres cardiovasculaires, la resténose, l'artériosclérose, les néphropathies ou rétinopathies, le composé de formule (I') et/ou ses sels physiologiquement acceptables.

8. Utilisation du composé de formule (I') et/ou ses sels physiologiquement acceptables pour la préparation de médicaments destinés à la prévention ou au traitement de l'ostéoporose.

9. Utilisation du composé de formule (I') et/ou ses sels physiologiquement acceptables pour la préparation de médicaments destinés à inhiber la croissance tumorale ou les métastases cancéreuses.

10. Utilisation du composé de formule (I') et/ou ses sels physiologiquement acceptables pour la préparation de médicaments destinés à la prévention ou au traitement des désordres cardiovasculaires, de la resténose, de l'artériosclérose, des néphropathies ou des rétinopathies.

## Claims

1. Pyrimidine derivative of formula (I'):

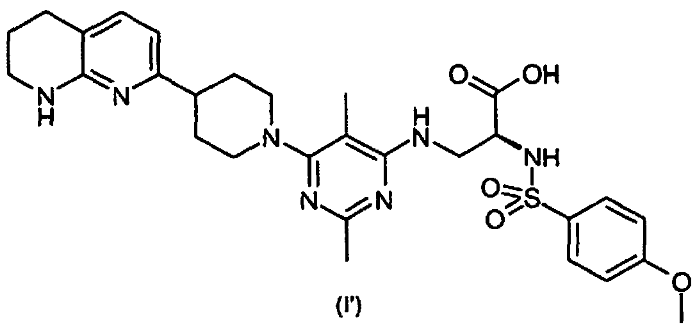

(I')

and the physiologically acceptable addition salts and the solvates of this compound.

2. As a medicament, the compound of formula (I') and/or its physiologically acceptable salts.

3. Pharmaceutical composition comprising a medicament as defined in Claim 2, in a pure state or in the presence of one or more excipients.

4. As a medicament, according to Claim 2, having a vitronectin receptor antagonist activity, the compound of formula (I') and/or its physiologically acceptable salts.

5. As a medicament, according to Claim 2, having a bone resorption inhibiting activity or for the treatment or prevention of osteoporosis, the compound of formula (I') and/or its physiologically acceptable salts.

6. As a medicament, according to Claim 2, having a tumour growth or cancer metastasis inhibiting activity, the compound of formula (I') and/or its physiologically acceptable salts.

7. As a medicament, according to Claim 2, having an anti-inflammatory activity or for the treatment or prevention of cardiovascular disorders, restenosis, arteriosclerosis, nephropathies or retinopathies, the compound of formula (I') and/or its physiologically acceptable salts.

8. Use of the compound of formula (I') and/or its physiologically acceptable salts, for the preparation of medicaments intended for the prevention or treatment of osteoporosis.

9. Use of the compound of formula (I') and/or its physiologically acceptable salts, for the preparation of medicaments intended for inhibiting tumour growth or cancer metastasis.

10. Use of the compound of formula (I') and/or its physiologically acceptable salts, for the preparation of medicaments intended for the prevention or treatment of cardiovascular disorders, restenosis, arteriosclerosis, nephropathies or retinopathies.

## Patentansprüche

1. Pyrimidinderivat der Formel (I') :

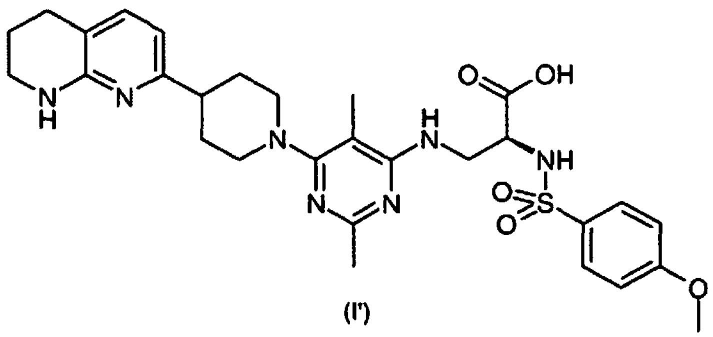

(I')

sowie die physiologisch unbedenklichen Additionssalze und die Solvate dieser Verbindung.

**2.** Verbindung der Formel (I') und/oder ihre physiologisch unbedenklichen Salze als Arzneimittel.

**3.** Pharmazeutische Zusammensetzung, umfassend ein wie in Anspruch 2 definiertes Arzneimittel im reinen Zustand oder in Gegenwart von einem oder mehreren Grundstoffen.

**4.** Verbindung der Formel (I') und/oder ihre physiologisch unbedenklichen Salze als Arzneimittel nach Anspruch 2 mit Aktivität als Vitronektinrezeptorantagonist.

**5.** Verbindung der Formel (I') und/oder ihre physiologisch unbedenklichen Salze als Arzneimittel nach Anspruch 2 mit Hemmwirkung auf die Knochenresorption oder für die Behandlung oder Vorbeugung von Osteoporose.

**6.** Verbindung der Formel (I') und/oder ihre physiologisch unbedenklichen Salze als Arzneimittel nach Anspruch 2 mit Hemmwirkung auf das Tumorwachstum oder auf Krebsmetastasen.

**7.** Verbindung der Formel (I') und/oder ihre physiologisch unbedenklichen Salze als Arzneimittel nach Anspruch 2 mit entzündungshemmender Wirkung oder für die Behandlung oder Vorbeugung von Herz-Kreislauf-Störungen, Restenose, Arteriosklerose, Nephropathien oder Retinopathien.

**8.** Verwendung einer Verbindung der Formel (I') und/oder ihrer physiologisch unbedenklichen Salze für die Herstellung von Arzneimitteln zur Vorbeugung oder Behandlung von Osteoporose.

**9.** Verwendung einer Verbindung der Formel (I') und/oder ihrer physiologisch unbedenklichen Salze für die Herstellung von Arzneimitteln für die Hemmung des Tumorwachstums oder von Krebsmetastasen.

**10.** Verwendung einer Verbindung der Formel (I') und/oder ihrer physiologisch unbedenklichen Salze für die Herstellung von Arzneimitteln zur Vorbeugung oder Behandlung von Herz-Kreislauf-Störungen, Restenose, Arteriosklerose, Nephropathien oder Retinopathien.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 05771271 A
- WO 9412181 A
- WO 9408577 A
- EP 528586 A
- EP 528587 A
- WO 9532710 A
- WO 9600574 A
- WO 9600730 A
- WO 9800395 A
- WO 9932457 A
- WO 9937621 A
- EP 0820991 A
- WO 2004048375 A
- WO 2004048375 A1


### Littérature non-brevet citée dans la description


- **Brown et al.** *cardiovascular Res.,* 1994, vol. 28, 1815
- **Brook et al.** *Cell,* 1994, vol. 79, 1157
- **Cheresh et al.** *Science,* 1995, vol. 270, 1500
- **Fleicher et al.** *Advanced Drug Delivery Review,* 1996, vol. 19, 115-130
- Design of prodrugs. Elsevier, 1985
- **H. Bungaard.** *Drugs of the Future,* 1991, vol. 16, 443
- **Saulnier et al.** *Bioorg. Med. Chem. Lett.,* 1994, vol. 4, 1985
- **Safadi et al.** *Pharmaceutical Res.,* 1993, vol. 10, 1350
- *Dermot Cox DN§P,* Mai 1995, vol. 8 (4), 197-205
- Drug targeting, voir Targeted Drug Delivery. **R. C. Juliano et al.** Handbook of Expérimental Pharmacology. Springer Verlag, vol. 100
- *J. Med. Chem.,* 2001, vol. 44 (8), 1158-1176
- *J. Org. Chem.,* 2004, vol. 69, 1959-1966
- Liebigs Annalen der Chemie. 1972, vol. 766, 73-88
- **Pytella et al.** Dilution en tampon d'incubation, à adapter suivant le lot de récepteur et selon le ligand. *Methods Enzymol.,* 1987, 144